(19) Europäisches Patentamt / European Patent Office / Office européen des brevets

(11) **EP 2 327 712 B1**

(12) **EUROPEAN PATENT SPECIFICATION**

(45) Date of publication and mention of the grant of the patent:
**15.10.2014 Bulletin 2014/42**

(51) Int Cl.:
**C07K 5/00** *(2006.01)*    **C07K 7/00** *(2006.01)*
**A61K 38/00** *(2006.01)*    **C07K 16/00** *(2006.01)*
**G01N 33/53** *(2006.01)*

(21) Application number: **10180952.3**

(22) Date of filing: **23.07.1999**

(54) **Treatment of Crohn's disease with copolymer 1 and polypeptides**

Behandlung von Morbus Crohn mit Copolymer 1 und ähnlichen Polypeptiden

Traitement de la maladie de Crohn à l'aide du copolymère 1 et des polypeptides apparentés

(84) Designated Contracting States:
**AT BE CH CY DE DK ES FI FR GB GR IE IT LI LU MC NL PT SE**

(30) Priority: **23.07.1998 US 93859 P**
**25.09.1998 US 101825 P**
**02.10.1998 US 102960 P**
**12.11.1998 US 108184 P**
**30.10.1998 US 106350 P**

(43) Date of publication of application:
**01.06.2011 Bulletin 2011/22**

(62) Document number(s) of the earlier application(s) in accordance with Art. 76 EPC:
**99937423.4 / 1 098 902**

(73) Proprietors:
• **YEDA RESEARCH AND DEVELOPMENT CO., LTD.**
**76100 Rehovot (IL)**
• **President and Fellows of Harvard College**
**Cambridge, MA 02138 (US)**

(72) Inventors:
• **Aharoni, Rina**
**76346, Rehovot (IL)**
• **Teitelbaum, Dvora**
**76209, Rehovot (IL)**
• **Arnon, Ruth**
**76100, Rehovot (IL)**
• **Sela, Michael**
**76100, Rehovot (IL)**
• **Fridkis-Hareli, Masha**
**Cambridge, MA 02138 (US)**
• **Strominger, Jack, L.**
**Cambridge,MA 02138-5779 (US)**

(74) Representative: **Vossius & Partner**
**Siebertstrasse 4**
**81675 München (DE)**

(56) References cited:
**WO-A1-98/30227    US-A- 5 679 640**

• **BORNSTEIN M B ET AL: "A PILOT TRIAL OF COP 1 IN EXACERBATING-REMITTING MULTIPLE SCLEROSIS", NEW ENGLAND JOURNAL OF MEDICINE, MASSACHUSETTS MEDICAL SOCIETY, BOSTON, MA, US, vol. 317, no. 7, 13 August 1987 (1987-08-13), pages 408-414, XP009042359, ISSN: 1533-4406**
• **FRIDKIS-HARELI M. ET AL.: "Direct binding of myelin basic protein and synthetic copolymer 1 to class II major histocompatibility comples molecules on living antigen-presenting cells - specificity and promiscuity", PROCEEDINGS OF THE NATIONAL ACADEMY OF SCIENCES OF USA, vol. 91, 1994, pages 4872-4876, XP000891508, WASHINGTON US**
• **MASHA FRIDKIS-HARELI AND JACK L STROMINGER: "Promiscuous Binding of Synthetic Copolymer 1 to PurifiedHLA-DR Molecules", JOURNAL OF IMMUNOLOGY, AMERICAN ASSOCIATION OF IMMUNOLOGISTS, US, vol. 160, 1 January 1998 (1998-01-01), pages 4386-4397, XP007917947, ISSN: 0022-1767**

**Description**

INTRODUCTION

The present invention provides compositions and methods for treating Crohn's disease using therapeutically effective amounts of a peptide or polypeptide related to Copolymer 1. Copolymer 1 is a heterogeneous mixture of synthetic random linear copolymers of tyrosine, alanine, glutamic acid and lysine and, in appropriate therapeutic amounts and average molecular sizes, is used to treat multiple sclerosis. When such mixtures of synthetic random linear copolymers consist essentially of the three of the four amino acids found in Copolymer 1, they are referred to as Terpolymers. The present invention relates in part to Terpolymers. Preferably, the Terpolymers are composed of tyrosine, alanine and lysine, or of glutamic acid, tyrosine and lysine, or of glutamic acid, alanine and lysine. Surprisingly, the Terpolymers have efficacy for treating a variety of auto-immune diseases and bind to Class II major histocompatibility complex (MHC) molecules as well as to antigen presenting cells.

The present specification also includes certain peptides, comprising the four amino acids of Copolymer 1, which have also been found to have efficacy for treating a variety of autoimmune diseases and bind to Class II major histocompatibility complex (MHC) molecules as well as to antigen presenting cells. Such peptides are referred to below as "Copeptides," to stress their relationship to Copolymer 1.

BACKGROUND OF THE INVENTION

Autoimmune diseases occur when an organism's immune system fails to recognize some of the organism's own tissues as "self" and attacks them as "foreign." Normally, self-tolerance is developed early by developmental events within the immune system that prevent the organism's own T cells and B cells from reacting with the organism's own tissues. MHC cell surface proteins help regulate these early immune responses by binding to and presenting processed peptides to T cells.

This self-tolerance process breaks down when autoimmune diseases develop. Now the organism's own tissues and proteins are recognized as "autoantigens" and are attacked by the organism's immune system. For example, multiple sclerosis is believed to be an autoimmune disease occurring when the immune system attacks the myelin sheath, whose function is to insulate and protect nerves. It is a progressive disease characterized by demyelination, followed by neuronal and motor function loss. Rheumatoid arthritis ("RA") is also believed to be an autoimmune disease which involves chronic inflammation of the synovial joints and infiltration by activated T cells, macrophages and plasma cells, leading to a progressive destruction of the articular cartilage. It is the most severe form of joint disease. The nature of the autoantigen(s) attacked in rheumatoid arthritis is poorly understood, although collagen type II is a candidate.

A tendency to develop multiple sclerosis and rheumatoid arthritis is inherited -- these diseases occur more frequently in individuals carrying one or more characteristic MHC class II alleles. For example, inherited susceptibility for rheumatoid arthritis is strongly associated with the MHC class II DRB1 *0401, DRB 1 *0404, or DRB 1*0405 or the DRB1*0101 alleles. The histocompatibility locus antigens (HLA) are found on the surface of cells and help determine the individuality of tissues from different persons. Genes for histocompatibility locus antigens are located in the same region of chromosome 6 as the major histocompatibility complex (MHC). The MHC region expresses a number of distinctive classes of molecules in various cells of the body, the genes being, in order of sequence along the chromosome, the Class I, II and III MHC genes. The Class I genes consist of HLA genes, which are further subdivided into A, B and C subregions. The Class II genes are subdivided into the DR, DQ and DP subregions. The MHC-DR molecules are the best known; these occur on the surfaces of antigen presenting cells such as macrophages, dendritic cells of lymphoid tissue and epidermal cells. The Class III MHC products are expressed in various components of the complement system, as well as in some non-immune related cells.

A number of therapeutic agents have been developed to treat autoimmune diseases, including steroidal and non-steroidal anti-inflammatory drugs, for example, methotrexate; various interferons; and certain inhibitors of prostaglandin synthesis. However, these agents can be toxic when used for more than short periods of time or cause undesirable side effects. Other therapeutic agents bind to and/or inhibit the inflammatory activity of tumor necrosis factor (TNF), for example, anti-TNF specific antibodies or antibody fragments, or a soluble form of the TNF receptor. These agents target a protein on the surface of a T cell and generally prevent interaction with an antigen presenting cell (APC). However, therapeutic compositions containing natural folded proteins are often difficult to produce, formulate, store, and deliver. Moreover, the innate heterogeneity of the immune system can limit the effectiveness of drugs and complicate long-term treatment of autoimmune diseases.

Thus in order to effectively treat autoimmune diseases and other immune conditions, new drugs are needed that do not have the side effects of the present therapeutic agents and which adequately address to the innate heterogeneity of the immune system.

REFERENCES

Aharoni, et al., 58 immunology Letters 79 (1997).
Allison, in IMMUNOSUPPRESSION AND ANTI-INFLAMMATORY DRUGS, ANNALS OF THE NEW YORK ACADEMY OF SCIENCE 696:xi (1993).
Ben-Nun A et al., 243 J NEUROL (Suppl 1) S14-S22 (1996).
Dorling et al., 6 CUR. OPINIONS IMMUNOL. 765 (1994).
Ferrara et al., 324 NEW ENGLAND J. OF MEDICINE 667 (1991).
Fridkis-Hareli, et al., 63 J. NEUROCHEM. 63 (Suppl. 1) S61 (1994).
Fridkis-Hareli, et al., 163 CELL. IMMUNOL. 229. (1995).
Fridkis-Hareli, et al., 160 J. IMMUNOL. 4386 (1998).
Johnson, 1 NEUROLOGY 65-70 (1995).
Kay et al., 22 TRANSPLANTATION PROCEEDINGS 96 (1990).
Kelemen, et al., 102 INT ARCH ALLERGY IMMUNOL. 309 (1993).
Mengle-Gaw, The Major Histocompatibility Complex (MHC), in the ENCYCLOPEDIA OF MOLECULAR BIOLOGY 602-06 (Oxford: Blackwell Science Ltd., 1994).
Rothbard, J. B., et al,. 9 ANNU. REV. IMMUNOL. 527 (1991).
Schlegel, et al., 84 BLOOD 2802 (1994).
Sela M et al., 88 BULL INST PASTEUR 303-14 (1990).
Stazl, 22 TRANSPLANTATION PROCEEDINGS 5 (1990).
Sykes, 10 THE FASEB JOURNAL 721 (1996).
Teitelbaum et al., 1 EUR. J. IMMUNOL. 242-48 (1971).
Teitelbaum et al., 3 EUR. J. IMMUNOL. 273-79 (1973).
Teitelbaum et al., 64 J. NEUROIMMUNOL. 209-17 (1996).
Thomson, 10 IMMUNOLOGY TODAY 6 (1988).
Van Den Bogaerde, et al., 52 TRANSPLANTATION 15 (1991).
Webb et al. 13 IMMUNOCHEM. 333 (1976).

WO983022 disclose the use of a copolymer consisting of Y,E,A,K and having MW within 2,000 and 40,000 daltons for treating an autoimmune disease, multiple sclerosis.

US5,679,640 discloses methods for treating autoimmune diseases involving peptides that bind to MHC and inhibit T cells. Peptides binding to HLA-DR1 are proposed to treat rheumatoid arthritis.

Fridkis-Hareli, M. et al. (1994, Proc. Natl. Acad. Sci. USA, vol. 91, p. 4872-4876 and 1998, Journal of Immunology, vol. 160, p. 4386-4397) disclose that Cop1 binds to several DRs.

## SUMMARY OF THE INVENTION

The invention in its broadest form is defined in independent claims 1 and 8:

1. Use of a mixture of polypeptides having an average molecular weight of 2,000 to 40,000 daltons, wherein each polypeptide consists of glutamic acid, tyrosine, alanine and lysine, in an amount effective to treat a human subject afflicted with Crohn's disease for the preparation of a pharmaceutical composition to be administered to a subject afflicted with Crohn's disease for the treatment of Crohn's disease.

8. Copolymer 1 for use in treating Crohn's disease.

Preferred embodiments are defined in dependent claims 2-7. The disclosure of treatment of diseases other than Crohn's disease in the present specification is for reference only.

Surprisingly, Copolymer 1 and the Terpolymers and Copeptides of the present invention can be used to treat a variety of autoimmune diseases in a heterogeneous patient population. These Terpolymers and Copeptides can inhibit some of the physiological responses of T cells that attack self-antigens as these diseases progress. Moreover, Copolymer 1 and the Terpolymers and Copeptides of the present invention bind with high affinity to antigen presenting cells from different genetic backgrounds and to several class II MHC molecules to block immune cell recognition and attack. In addition, the Terpolymers and Copeptides can stimulate the growth and functioning of Copolymer 1-specific T cells to further treat and prevent various autoimmune diseases.

Accordingly, the present disclosure provides a composition having a polypeptide comprising three different amino acids selected from the group of amino acids comprising Copolymer 1, that is, glutamic acid, alanine, lysine, and tyrosine, in the approximate relative molar ratios found in Copolymer 1.

The present disclosure is also directed to peptides comprising three different amino acids selected from the group of amino acids comprising Copolymer 1,

The present disclosure is also directed to pharmaceutical compositions which include a therapeutically effective amount of a Terpolymer consisting essentially of amino acids tyrosine, alanine and lysine, in the molar ratio of from about 0.005 to about 0.25 tyrosine, from about 0.3 to about 0.6 alanine, and from about 0.1 to about 0.5 lysine. and a pharmaceutically acceptable carrier. This Terpolymer is preferably substantially free of glutamic acid.

The present disclosure further provides a pharmaceutical composition which includes a therapeutically effective amount of a Terpolymer consisting essentially of glutamic acid, tyrosine and lysine, in the molar ratio of from about 0.005 to about 0.300 glutamic acid, from about 0.005 to about 0.250 tyrosine; and from about 0.3 to about 0.7 lysine, and a pharmaceutically acceptable carrier. The Terpolymer is preferably substantially free of alanine.

The present disclosure is also directed to pharmaceutical compositions which include a therapeutically effective amount of a Terpolymer consisting essentially of amino acids tyrosine, glutamic acid and alanine in the molar ratio of from about 0.005 to about 0.25 tyrosine, from about 0.005 to about 0.3 glutamic acid, and from about 0.005 to about 0.8 lysine and a pharmaceutically acceptable carrier. This Terpolymer is preferably substantially free of lysine.

The present disclosure also provides a pharmaceutical composition which includes a therapeutically effective amount of a Terpolymer consisting essentially of glutamic acid, alanine and lysine, in the molar ratio of from about 0.005 to about 0.3 glutamic acid, from about 0.005 to about 0.6 alanine; and from about 0.2 to about 0.7 lysine and a pharmaceutically acceptable carrier. This Terpolymer is preferably substantially free of tyrosine.

The present disclosure also provides a pharmaceutical composition to treat autoimmune diseases which includes a therapeutically effective amount of Copolymer 1 or a polypeptide consisting essentially of glutamic acid, alanine, tyrosine and lysine, and a pharmaceutically acceptable carrier.

The present disclosure further provides methods for treating and preventing autoimmune diseases in a mammal which include administering a therapeutically effective amount of a composition comprising Copolymer 1, a Terpolymer or a Copeptide. In another embodiment, the method for treating autoimmune diseases in a mammal further involves inhibiting proliferation of T cells involved in the immune attack. In another embodiment, the method for treating autoimmune diseases in a mammal involves binding the Terpolymer or Copeptide to an antigen presenting cell. In yet another embodiment, the method for treating autoimmune disease in a mammal involves binding the Terpolymer or Copeptide to a major histocompatibility complex class II protein which is associated with autoimmune diseases.

Autoimmune diseases contemplated by the present disclosure include arthritic conditions, demyelinating diseases and inflammatory diseases. For example, autoimmune diseases which can be treated by the present compositions include multiple sclerosis, autoimmune hemolytic anemia, autoimmune oophoritis, autoimmune thyroiditis, autoimmune uveoretinitis, Crohn's disease, chronic immune thrombocytopenic purpura, colitis, contact sensitivity disease, diabetes mellitus, Graves disease, Guillain-Barre's syndrome, Hashimoto's disease, idiopathic myxedema, myasthenia gravis, psoriasis, pemphigus vulgaris, rheumatoid arthritis, or systemic lupus erythematosus. The present compositions can be used to treat one or more of these diseases.

## BRIEF DESCRIPTION OF THE DRAWINGS

As used below

"GAL" is a random terpolymer of glutamic acid, alanine and lysine;

"TGA" or "YEA" is a random terpolymer of tyrosine, glutamic acid, and alanine;

"TAL" or "YAK" is a random terpolymer of tyrosine, alanine, and lysine;

"GTL" or "YEK" is a random terpolymer of glutamic acid, tyrosine, and lysine; and

"YEAK" is Copolymer 1.

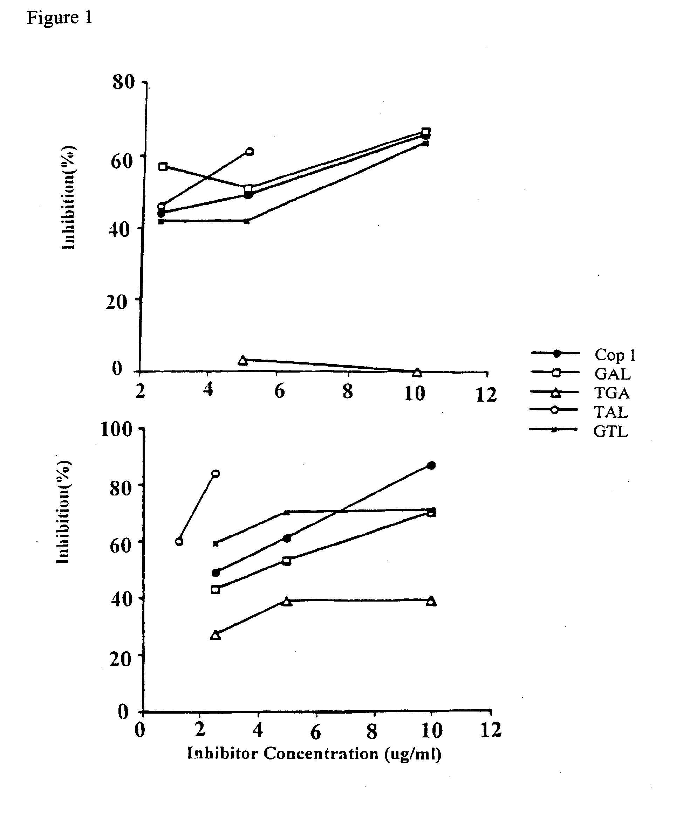

Figure 1 illustrates the effect of Copolymer 1 and Terpolymers on proliferation of T cells which are specific for certain myelin basic protein (MBP) peptides. Several of the present Terpolymers inhibit proliferation of T cell lines which are specific for myelin basic protein antigen, MBP 84-102 (SEQ ID NO: 1). The following symbols were used: Copolymer 1 (●); GAL (□); TGA (△); TAL (○); GTL (X). Figure 1A illustrates inhibition of the proliferation of mouse T cell clone MBP-sp-1 specific to MBP 84-102 peptide (0.5 g/well). As a control, proliferation of mouse T cell clone MBP-sp-1 stimulated by MBP 84-102 (SEQ ID NO: 1) without inhibitor was 21,145 cpm. Figure 1B illustrates inhibition of the response of human T cell clone GP-25 to MBP 84-102 peptide (0.125 g/well). Proliferation of human T cell clone GP-25 stimulated by MBP 84-102 (SEQ ID NO: 1) peptide without inhibitor was 11,442 cpm.

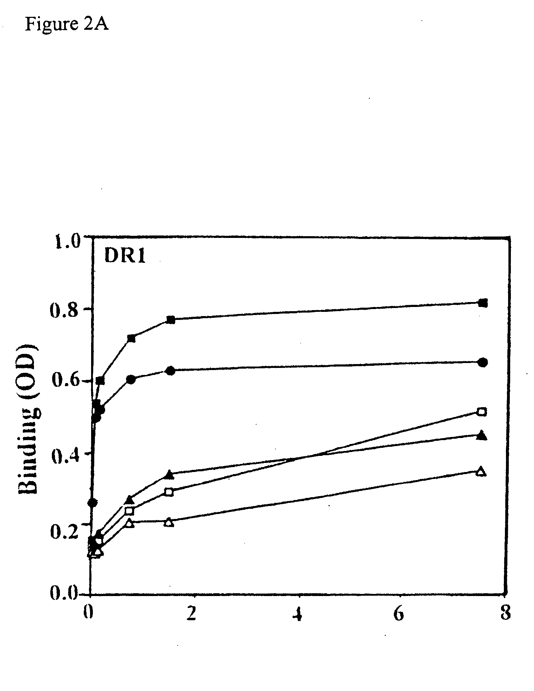

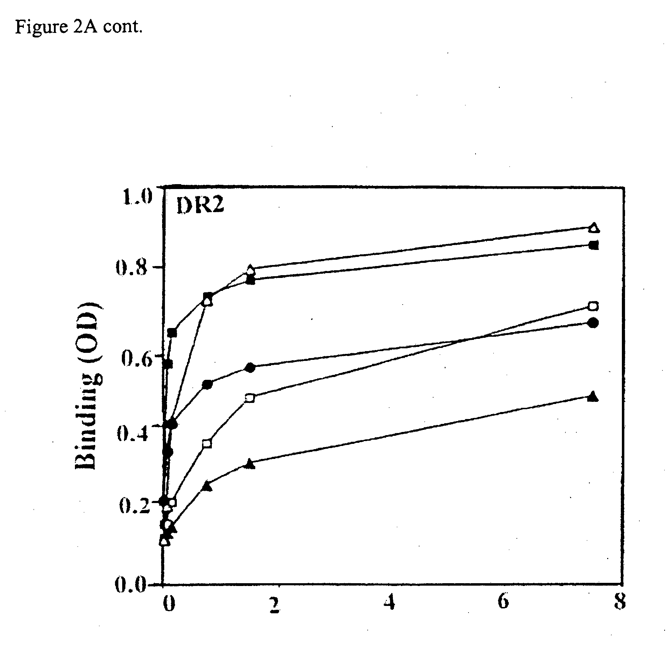

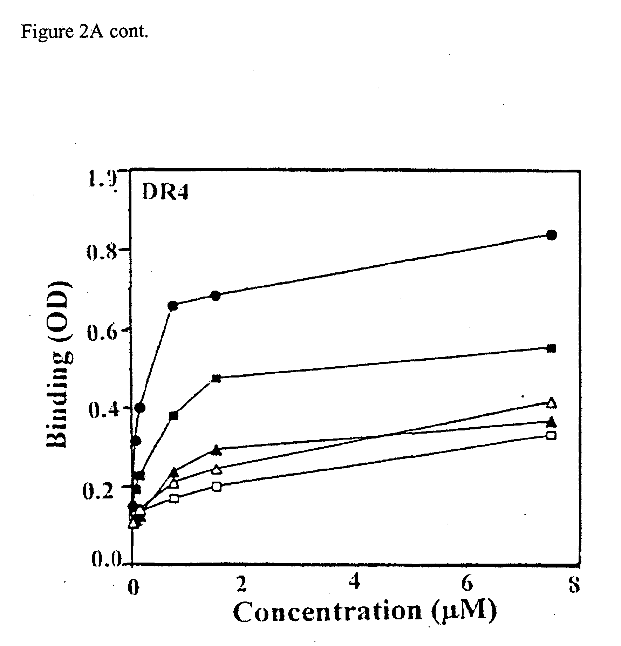

Figure 2A compares binding of the present polypeptides with the binding of Copolymer 1, to Class II major histocompatibility molecules DR1 (top), DR2 (middle) and DR4 (bottom). Binding by Copolymer 1 (■), was compared to binding by the following biotinylated Terpolymers: GAL (□); TGA (▲); GTL (△); and TAL (♦). The amount of Class II major histocompatibility molecule was held constant at 0.5 $\mu$g/sample and the concentration of polypeptide was varied between 0-8 $\mu$M as indicated on the y-axis. Binding was at pH 5.0 for 40 hr at 37°C. Binding was detected by capturing the polypeptide-class II complexes with an LB3.1 antibody and detecting the amount of biotinylated polypeptide bound by monitoring the absorbency at 410 nm after reaction with streptavidin-conjugated alkaline phosphatase.

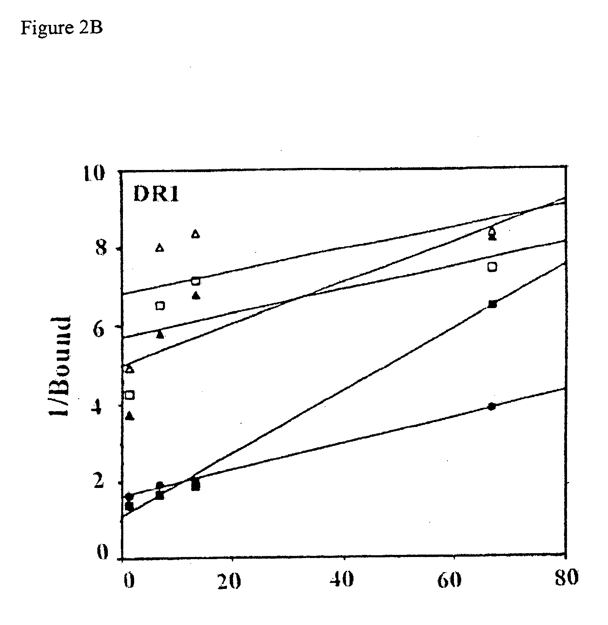

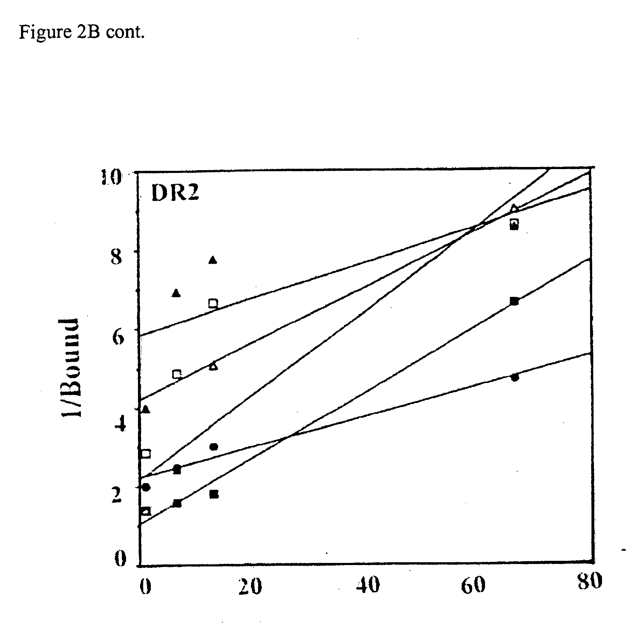

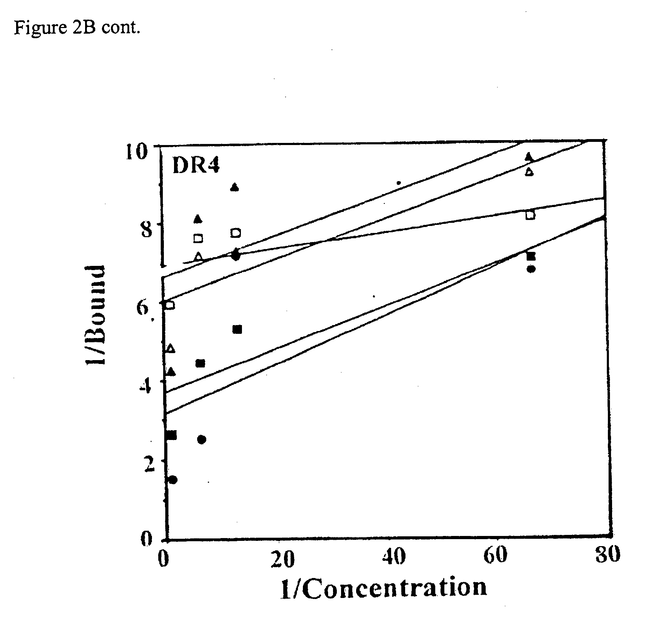

Figure 2B provides Lineweaver-Burke plots of the binding data provided in Figure 2A.

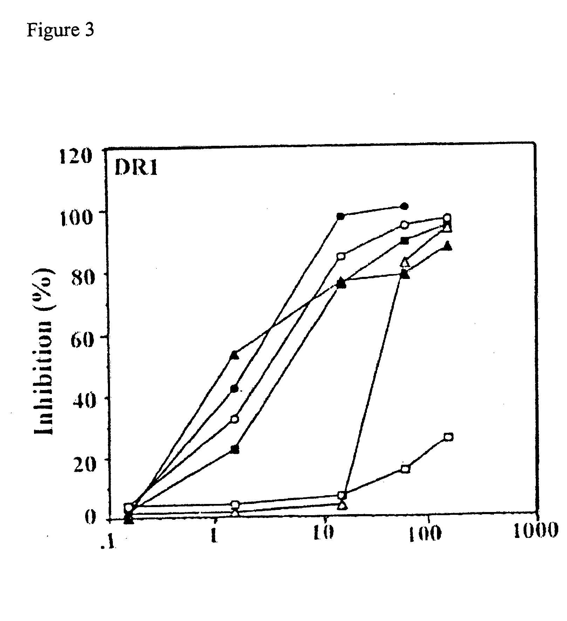

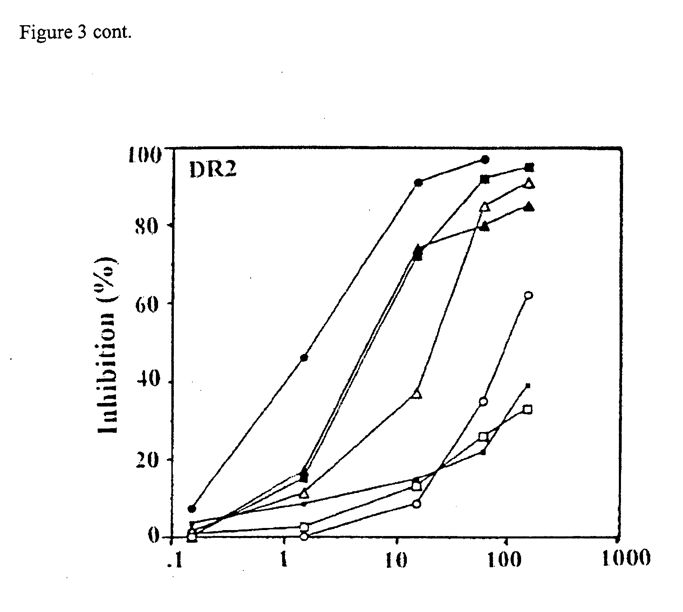

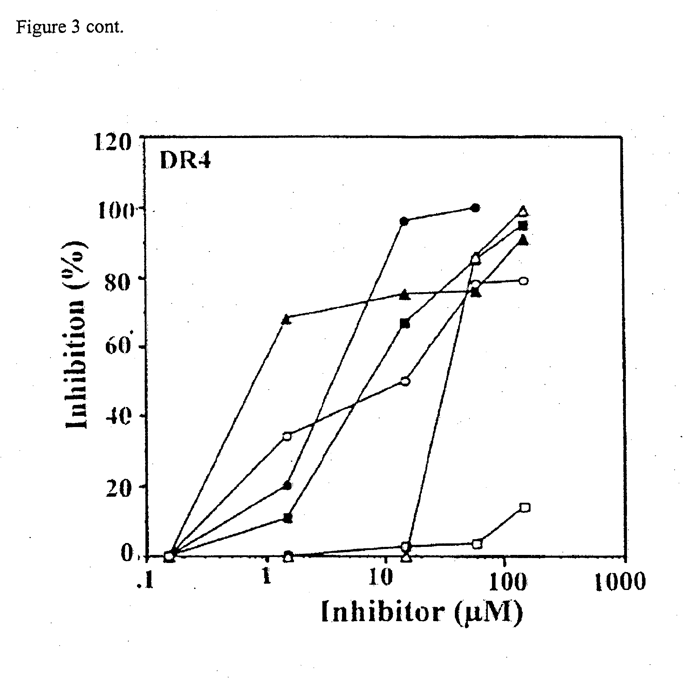

Figure 3 illustrates the competitive inhibition of Copolymer 1 binding to Class II major histocompatibility molecules by the present polypeptides. Purified HLA-DR1 (top), HLA-DR2 (middle) and HLA-DR4 (bottom) molecules were incubated with a constant amount (1.5 $\mu$M) of biotinylated Copolymer 1, either alone or in the presence of one of the following unlabeled polypeptides: Copolymer 1 (■); GAL (□); TGA (▲); GTL (△); and TAL (♦). Inhibition by these polypeptides was compared to inhibition by the myelin basic protein antigen HA 306-318 (○) (SEQ ID NO: 2). Binding was at pH 5.0 for 40 hr at 37°C. The amount of unlabeled polypeptide was varied between 0.1 -1000 $\mu$M, as indicated on the y-axis. Specific binding is expressed as the percentage of inhibition using equation 1:

$$\text{percent inhibition} = 100 - \frac{(\text{signal with competitor} - \text{background})}{(\text{signal without competitor} - \text{background})} \times 100$$

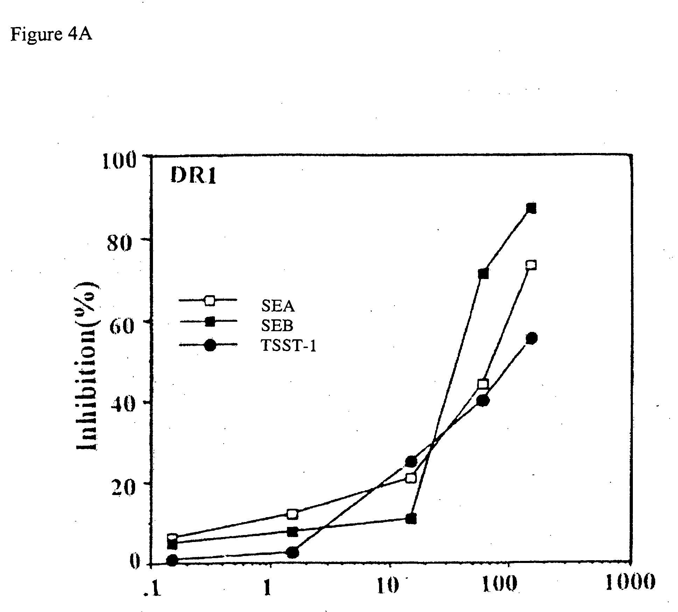

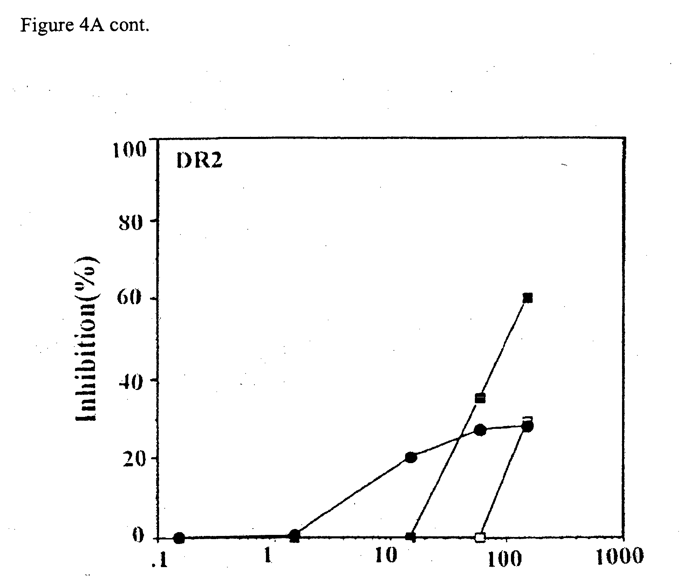

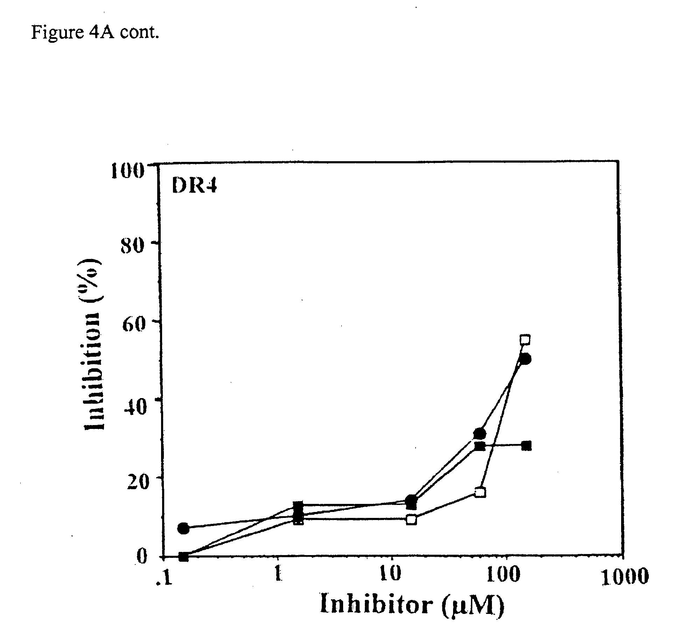

Figure 4A illustrates the competitive inhibition of TAL binding to Class II major histocompatibility molecules by bacterial superantigens SEA, SEB and TSST-1. Purified HLA-DR1 (top), HLA-DR2 (middle) and HLA-DR4 (bottom) molecules were incubated with a constant amount of biotinylated TAL, in the presence of increasing amounts of unlabeled bacterial superantigen SEA (□); SEB (■); or TSST-1 (●). Binding was at pH 5.0 for 40 hr at 37°C. The amount of superantigen was varied between 0.1 -1000 $\mu$M, as indicated on the y-axis. Specific binding is expressed as the percentage of inhibition using equation 1.

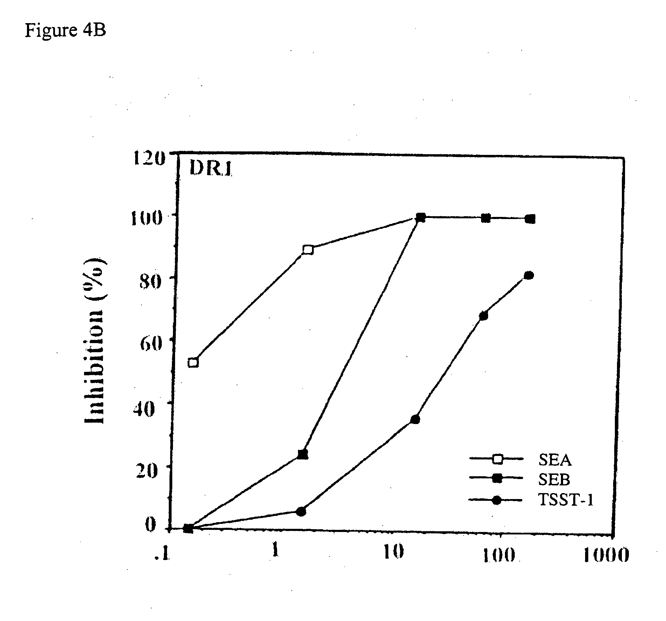

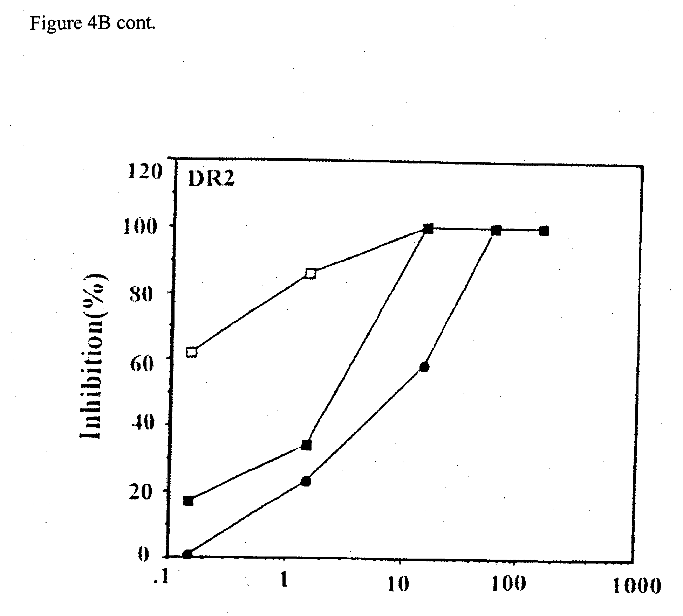

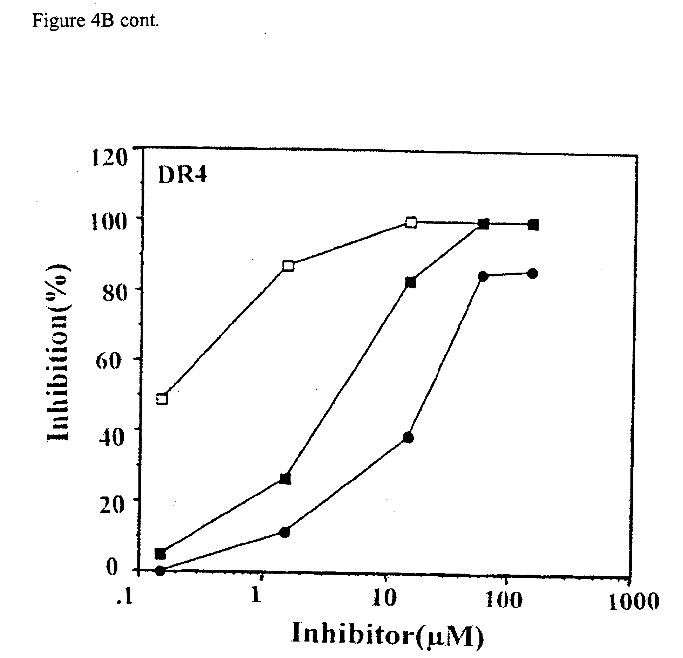

Figure 4B illustrates the competitive inhibition of TGA binding to Class II major histocompatibility molecules by bacterial superantigens SEA, SEB and TSST-1. Purified HLA-DR1 (top), HLA-DR2 (middle) and HLA-DR4 (bottom) molecules were incubated with a constant amount of biotinylated polypeptide TGA, in the presence of increasing amounts of unlabeled bacterial superantigen SEA (□); SEB (■); or TSST-1 (●). Binding was at pH 5.0 for 40 hr at 37°C. The amount of superantigen was varied between 0.1 -1000 $\mu$M, as indicated on the y-axis. Specific binding is expressed as the percentage of inhibition, calculated according to equation above 1.

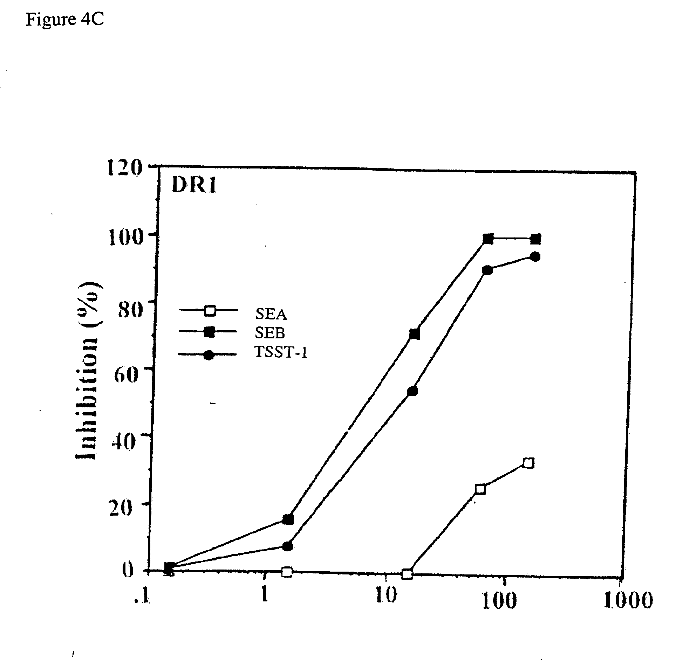

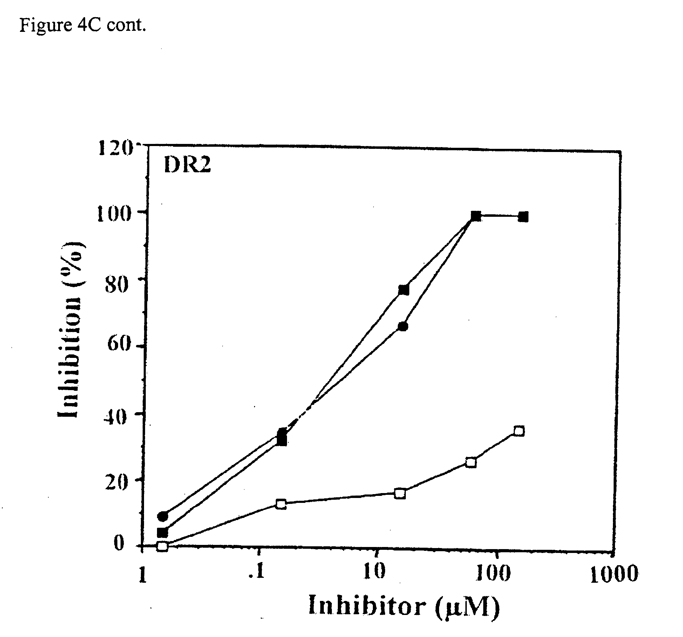

Figure 4C illustrates the competitive inhibition of GAL binding to Class II major histocompatibility molecules by bacterial superantigens SEA, SEB and TSST-1. Purified HLA-DR1 (top), and HLA-DR2 (bottom) molecules were incubated with a constant amount of biotinylated GAL, in the presence of increasing amounts of unlabeled bacterial superantigen SEA (□); SEB (■); or TSST-1 (●). Binding was at pH 5.0 for 40 hr at 37°C. The amount of superantigen was varied between 0.1 -1000 $\mu$M, as indicated on the y-axis. Specific binding is expressed as the percentage of inhibition using equation 1 above.

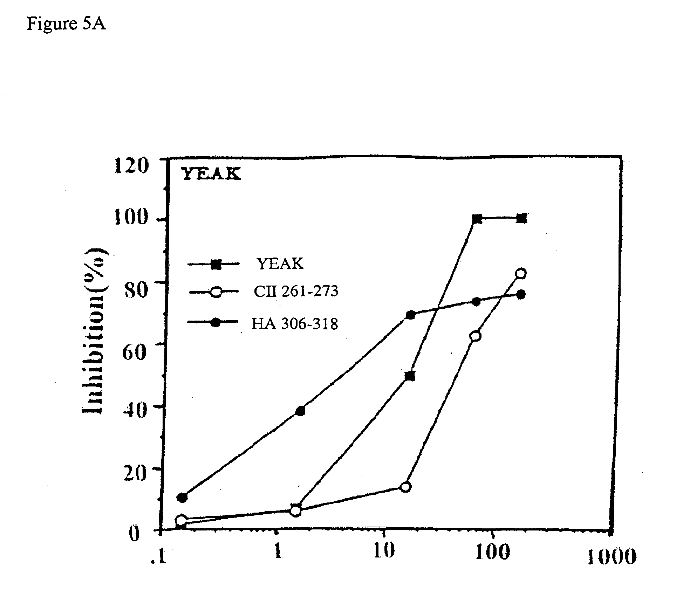

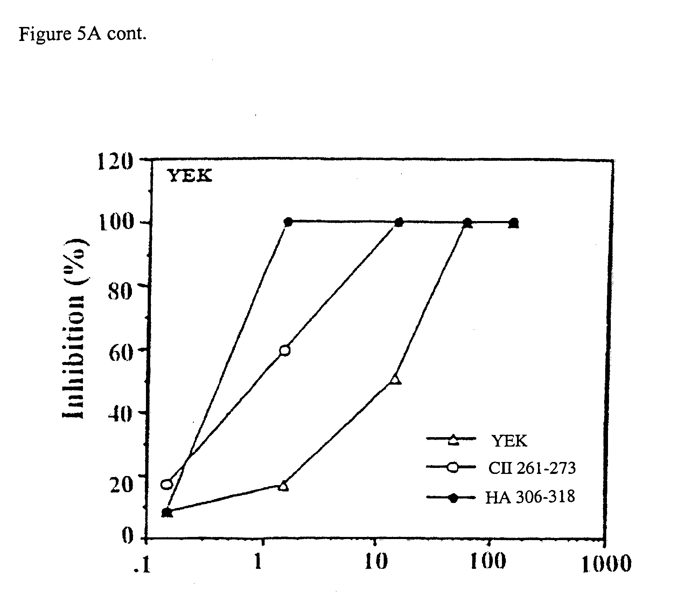

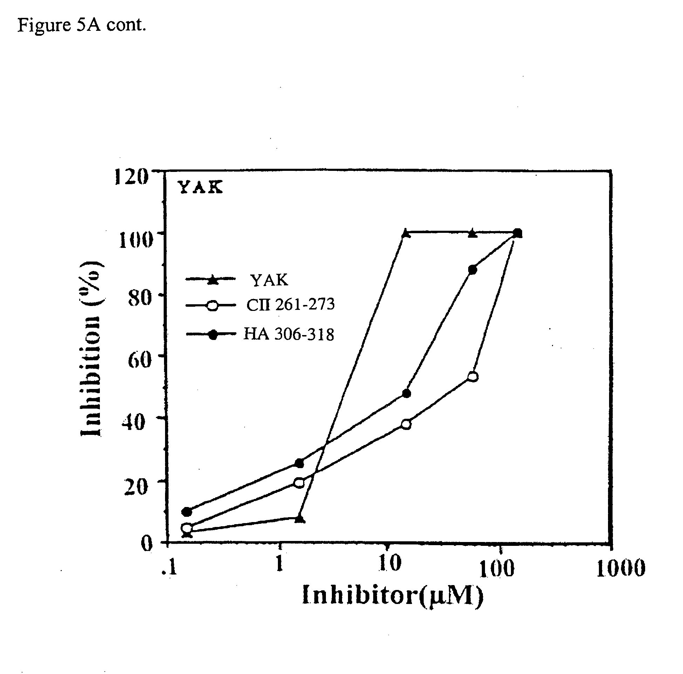

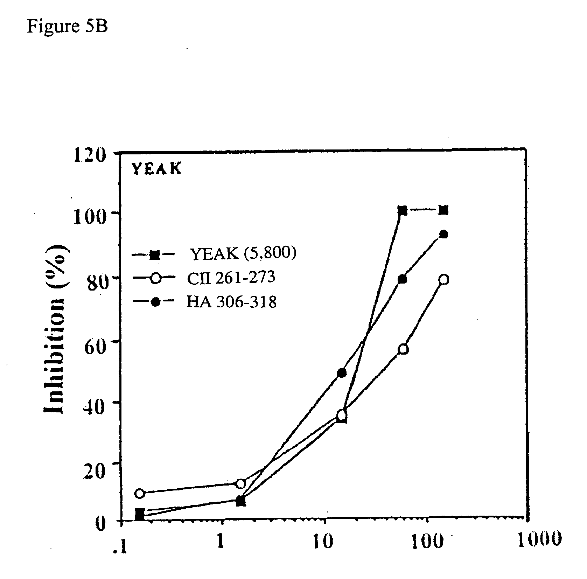

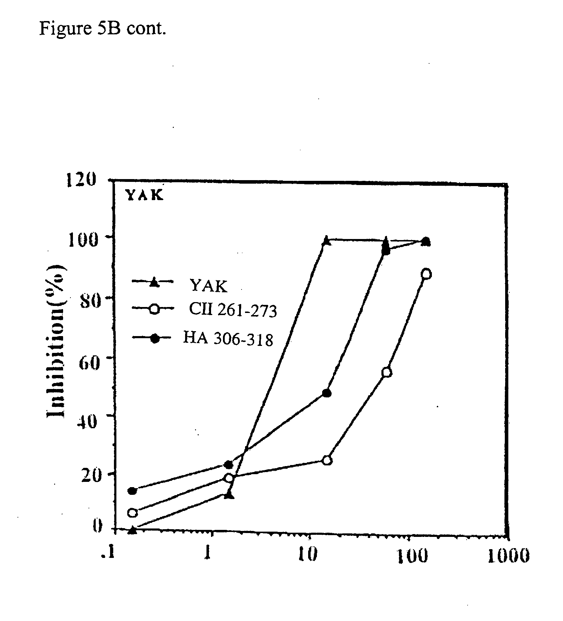

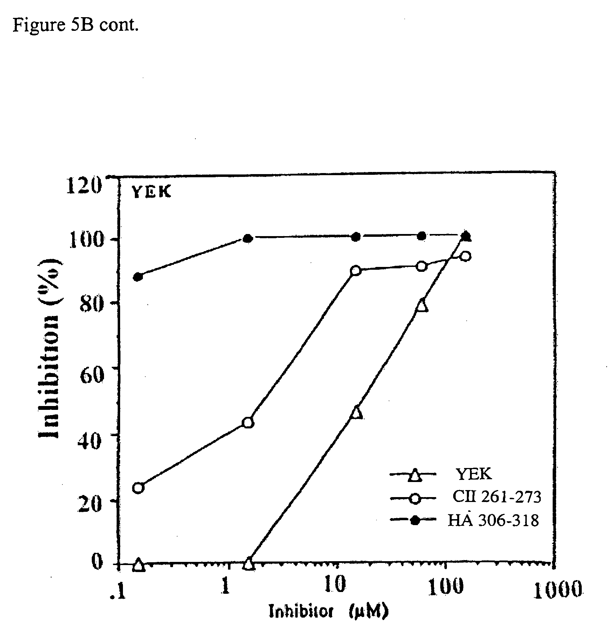

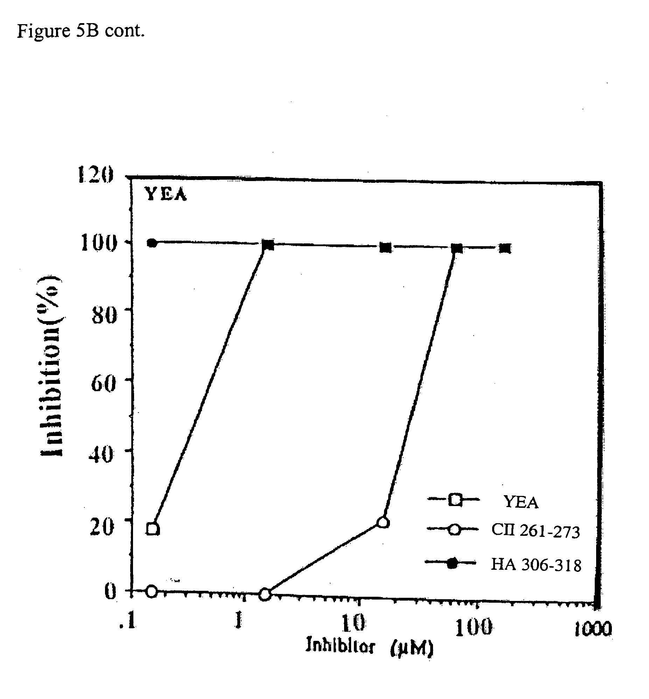

Figure 5 shows inhibition of binding of labeled molecules to MHC class II purified proteins by Terpolymers of the present invention. Fig. 5A shows inhibition of binding to the MHC HLA-DR1 protein. Fig. 5B shows inhibition of binding to the MHC HLA-DR4 protein. Unlabeled competitors include Terpolymers of the present invention, influenza virus hemagglutinin (HA) peptide 306-318 (SEQ ID NO:2), and type II collagen (CII) peptide 261-273 (SEQ ID NO:3). The concentration of unlabeled competitor is indicated on the abscissa. In each panel, inhibition by CII 261-273 (SEQ ID NO:3) is shown as open circles (○), inhibition by HA 306-318 (SEQ ID NO: 2) is shown by solid circles (●), inhibition by one of the present terpolymers is indicated as shown by open or solid triangles or squares. The extent of inhibition by GTL is shown using open triangles (△), by TAL is shown as solid triangles (▲), by TGA as open squares (□), and by Copolymer 1 as solid squares (■). Specific binding observed and shown on the ordinate was calculated as percentage of inhibition using equation 1 above.

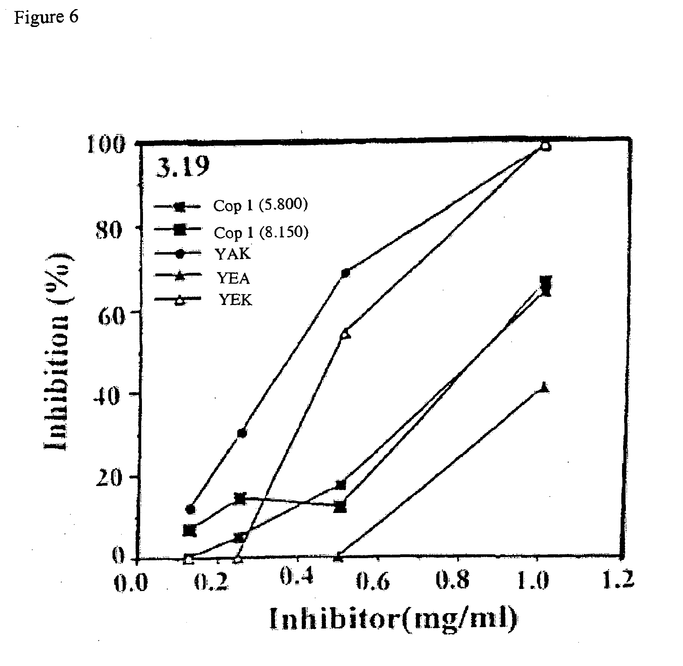

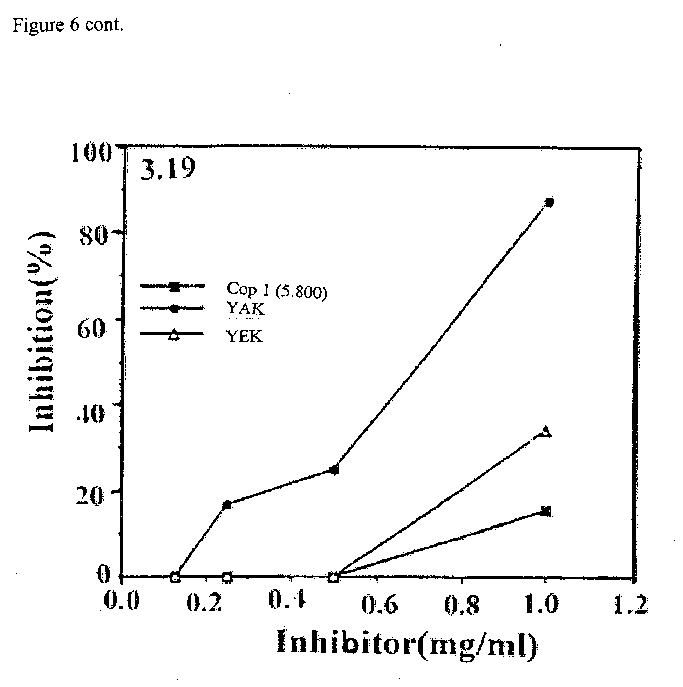

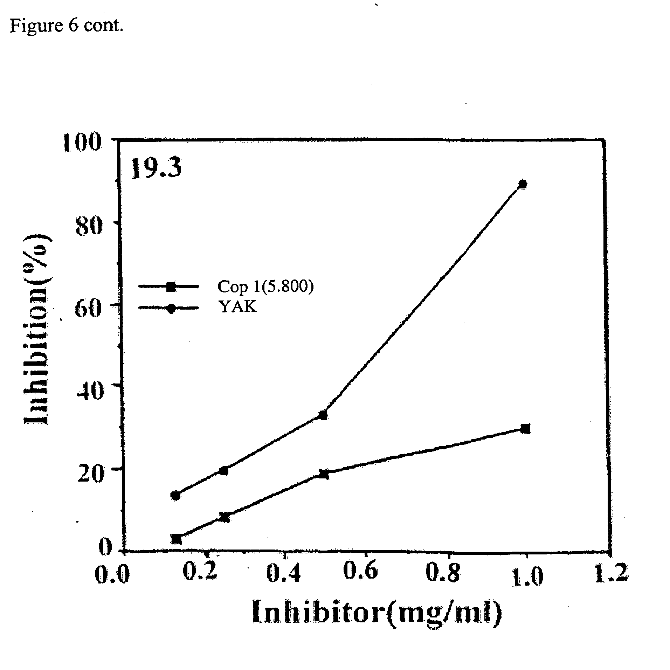

Figure 6 shows inhibition of IL-2 production by DR1-restricted-CII-specifiic T cell hybridomas in the presence of different polypeptides of the present invention. Irradiated L57.23 cells (fibroblasts transfected with a gene encoding HLA-DR1) were coincubated in duplicate with collagen peptide CII 261-273 (40 $\mu$g/ml) and varying concentrations, shown on the abscissa, of one of the present polypeptides for 2 hr at 37°C, then T cells (clone 3.19 or 19.3 as indicated) were added, and the mixtures were further incubated for 24 hr at 37°C. Supernatants (30 $\mu$l) were then removed, and were assayed for activation by IL-2-induced proliferation of IL-2-dependent cytotoxic T lymphocytes (CTL-L). The extent of inhibition by TAL is shown as solid circles (●), by TGA as solid triangles (▲), by GTL as open triangles (△), and by Copolymer 1 as solid squares (■). Percent inhibition of CTL-L proliferation shown on the ordinate was calculated according to equation 1.

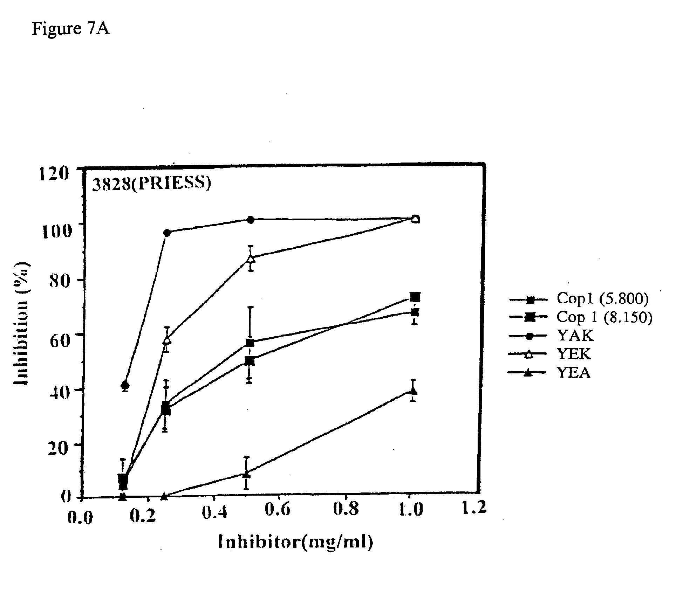

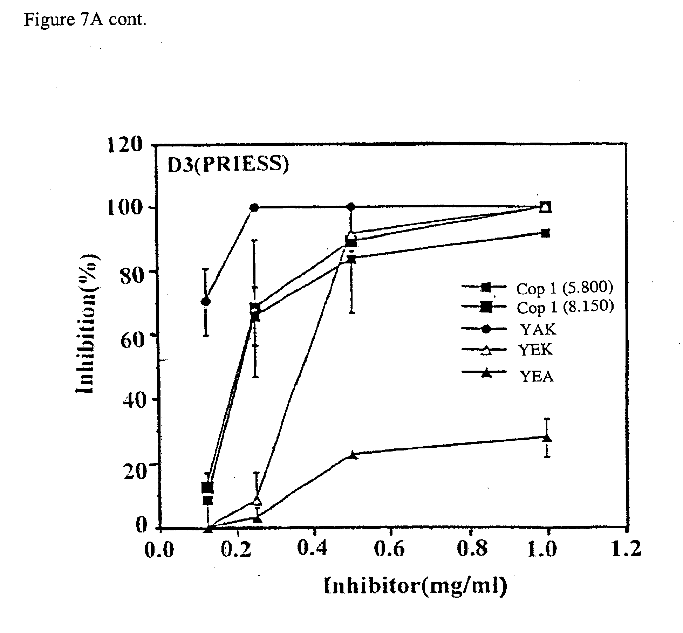

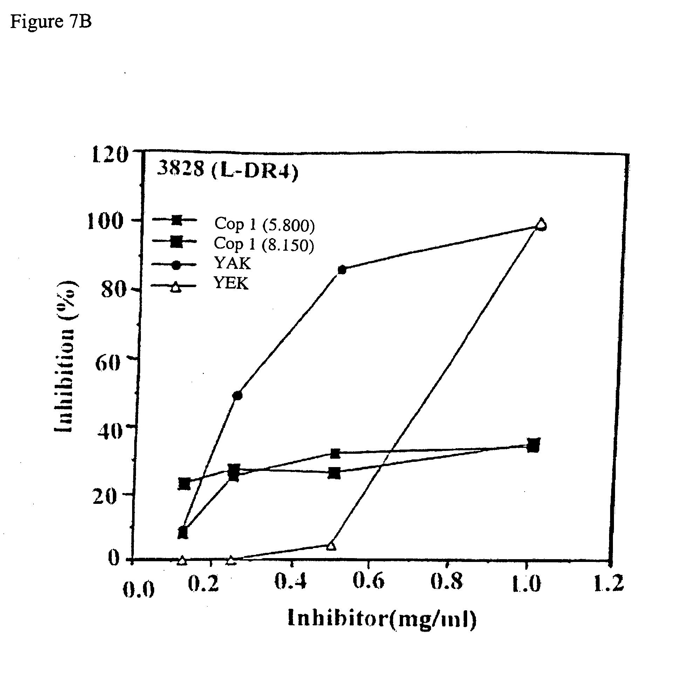

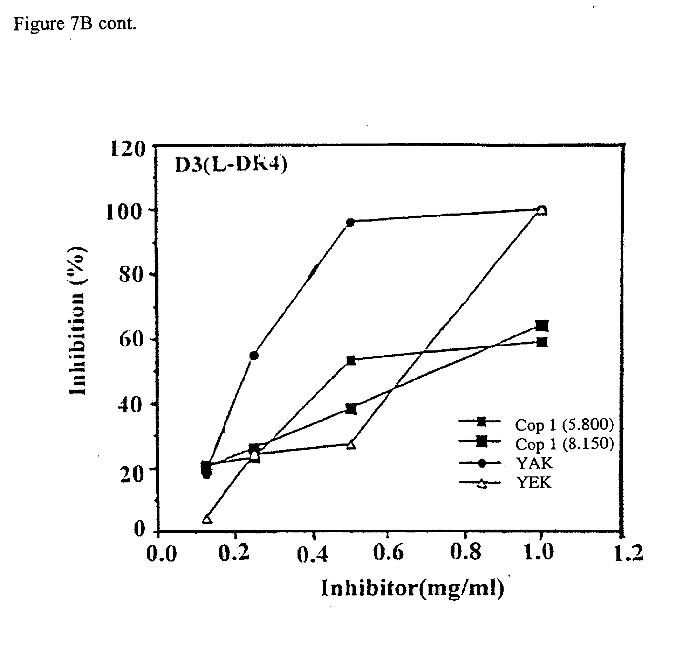

Figure 7 shows inhibition of IL-2 production by DR4-restricted CII-specific T cell hybridomas (3838 and D3) in the presence of different polypeptides of the present invention. Fig. 7A shows the effects of coincubating irradiated 3838 or D3 Priess cells with collagen peptide CII 261-273 (SEQ ID NO: 3) at the fixed concentration of 40 μg/ml, and with varying concentrations of each of the present polypeptides, for 2 hr at 37°C. Fig. 7B shows the effects of incubating L cells transfected with a gene encoding HIA-DR4 with collagen peptide CII 261-273 (SEQ ID NO:3) at the fixed concentration of 40 μg/ml, and with varying concentrations of each of the present polypeptides, for 2 hr at 37°C. T cells were then added (clones 3838 or D3 as indicated), samples were further incubated for 24 hr at 37°C, and supernatants were assayed as described in Figure 6. Each polypeptide was tested in duplicate. The concentration of the present polypeptides is indicated on the abscissa. The same symbols as used in Figure 6 are used for this figure.

## DETAILED DESCRIPTION OF THE INVENTION

The invention in its broadest form is defined in independent claims 1 and 8:

1. Use of a mixture of polypeptides having an average molecular weight of 2,000 to 40,000 daltons, wherein each polypeptide consists of glutamic acid, tyrosine, alanine and lysine, in an amount effective to treat a human subject afflicted with Crohn's disease for the preparation of a pharmaceutical composition to be administered to a subject afflicted with Crohn's disease for the treatment of Crohn's disease.

8. Copolymer 1 for use in treating Crohn's disease.

Preferred embodiments are defined in dependent claims 2-7. The disclosure of treatment of diseases other than Crohn's disease in the present specification is for reference only.

According to the present invention, polypeptides and peptides having at least three different amino acids randomly polymerized in a linear configuration are useful for treating autoimmune diseases. Autoimmune diseases occur when the immune system inappropriately attacks certain tissues or cells. The polypeptides and peptides of the present invention can prevent the immune system from attacking, for example, by suppressing the proliferation or function of T or B cells responsible for the attack, or by shielding the tissue from attack by binding to a MHC protein on the surface of the cells that make up the tissue.

Amino acids of the present invention include, but are not limited to the 20 commonly occurring amino acids. Also included are naturally occurring and synthetic derivatives, for example, selenocysteine. Amino acids further include amino acid analogs. Aa amino acid "analog" is chemically related forms of the amino acid having a different configuration, for example, an isomer, or a D-configuration rather than an L-configuration, or an organic molecule with the approximate size and shape of the amino acid, or an amino acid with modification to the atoms that are involved in the peptide bond, so as to be protease resistant when polymerized in a peptide or polypepide.

The phrases "amino acid" and "amino acid sequence" as defined here and in the claims can include one or more components which are amino acid derivatives and/or amino acid analogs comprising part or the entirety of the residues for any one or more of the 20 naturally occurring amino acids indicated by that sequence. For example, in an amino acid sequence having one or more tyrosine residues, a portion of one or more of those residues can be substituted with homotyrosine. Further, an amino acid sequence having one or more non-peptide or peptidomimetic bonds between two adjacent residues, is included within this definition.

The one letter and three letter amino acid codes (and the amino acid that each represents) are as follows: A means ala (alanine); C means cys (cysteine); D means asp (aspartic acid); E means glu (glutamic acid); F means phe (phenylalanine); G means gly (glycine); H means his (histidine); I means ile (isoleucine); K means lys (lysine); L means leu (leucine); M means met (methionine); N means asn (asparagine); P means pro (proline); Q means gin (glutamine); R means arg (arginine); S means ser (serine); T means thr (threonine); V means val (valine); W means trp (tryptophan); and Y means tyr (tyrosine).

The term "hydrophobic" amino acid is defined here and in the claims as including aliphatic amino acids alanine (A, or ala), glycine (G, or gly), isoleucine (I, or ile), leucine (L, or leu), proline (P, or pro), and valine (V, or val), the terms in parentheses being the one letter and three letter standard code abbreviation s for each amino acid, and aromatic amino acids tryptophan (W, or trp), phenylalanine (F or phe), and tyrosine (Y, or tyr). The amino acids confer hydrophobicity as a function of the length of aliphatic and size of aromatic side chains, when found as residues within a protein.

The term "charged" amino acid is defined here and in the claims as including an amino acids aspartic acid (D, or asp), glutamic acid (E, or glu), histidine (H, or his), arginine (R, or arg) and lysine (K, or lys), which confer a positive (his, lys and arg) or negative (asp and gly) charge at physiological values of pH in aqueous solutions on proteins containing these residues.

Polypeptide Compositions Contemplated by the Invention

The polypeptides and peptides of the present invention comprise Copolymer 1 and Terpolymers consisting essentially of three of the four amino acids of Copolymer 1, namely tyrosine, glutamic acid, alanine and lysine. Thus, the present invention further contemplates conservative amino acid substitutions for tyrosine, glutamic acid, alanine and lysine in the present polypeptides. Such conservative substitutions are structurally-related amino acid substitutions, including those amino acids which have about the same charge, hydrophobicity and size as tyrosine, glutamic acid, alanine or lysine. For example, lysine is structurally-related to arginine and histidine; glutamic acid is structurally-related to aspartic acid; tyrosine is structurally-related to serine, threonine, phenylalanine and tryptophan; and alanine is structurally-related to valine, leucine and isoleucine. These and other conservative substitutions, such as structurally-related synthetic amino acids, are contemplated by the present invention.

Moreover, the Terpolymers and Copeptides can be composed of *l*- or *d*-amino acids. As is known by one of skill in the art, *l*-amino acids occur in most natural proteins. However, *d*-amino acids are commercially available and can be substituted for some or all of the amino acids used to make the Terpolymers and Copeptides. The present invention contemplates Terpolymers and Copeptides formed from mixtures of *d*- and *l*-amino acids, as well as Terpolymers and Copeptides consistently essentially of either *l*- or *d*-amino acids.

The average molecular weight and the average molar fraction of the amino acids in the Terpolymers can vary. However, an average molecular weight range of about 2,000 to about 40,000 daltons is contemplated. A preferred average molecular weight range is from about 4,000 to about 12,000 daltons. The Copeptides can be from about 7 to about 100 amino acids in length, or from about 7 to about 50 amino acids in length, or from about 7 to about 25 amino acids in length, or from about 7 to about 15 amino acids in length. Preferred average molecular weight ranges and processes of making the Terpolymers are described in U.S. Patent No. 5,800,808.

In one embodiment, the present invention provides Terpolymers containing tyrosine, alanine and lysine. The average molar fraction of the amino acids in these Terpolymers can vary, for example, tyrosine can be present in a mole fraction of about 0.005 to about 0.250; alanine can be present in a mole fraction of about 0.3 to about 0.6; and lysine can be present in a mole fraction of about 0.1 to about 0.5. The average molecular weight is between 2,000 to about 40,000 daltons and preferably between about 3,000 to about 35,000 daltons. In a more preferred embodiment, the average molecular weight is about 5,000 to about 25,000 daltons.

In another embodiment, the present invention provides Terpolymers containing tyrosine, glutamic acid and lysine. The average molar fraction of the amino acids in these polypeptides can also vary, for example, glutamic acid can be present in a mole fraction of about 0.005 to about 0.300, tyrosine can be present in a mole fraction of about 0.005 to about 0.250; lysine can be present in a mole fraction of about 0.3 to about 0.7. The average molecular weight is between 2,000 to about 40,000 daltons and preferably between about 3,000 to about 35,000 daltons. In a more preferred embodiment, the average molecular weight is about 5,000 to about 25,000 daltons.

In another embodiment, the present invention provides Terpolymers containing glutamic acid, alanine and lysine. The average molar fraction of the amino acids in these polypeptides can also vary, for example, glutamic acid can be present in a mole fraction of about 0.005 to about 0.300, alanine can be present in a mole fraction of about 0.005 to about 0.600; lysine can be present in a mole fraction of about 0.2 to about 0.7. The average molecular weight is between 2,000 to about 40,000 daltons and preferably between about 3,000 to about 35,000 daltons. In a more preferred embodiment, the average molecular weight is about 5,000 to about 25,000 daltons.

In another embodiment, the present invention provides Terpolymers containing tyrosine, glutamic acid and alanine. The average molar fraction of the amino acids in these polypeptides can also vary, for example, tyrosine can be present in a mole fraction of about 0.005 to about 0.250; glutamic acid can be present in a mole fraction of about 0.005 to about 0.300; and alanine can be present in a mole fraction of about 0.005 to about 0.800. The average molecular weight is between 2,000 to about 40,000 daltons and preferably between about 3,000 to about 35,000 daltons. In a more preferred embodiment, the average molecular weight is about 5,000 to about 25,000 daltons.

In a more preferred embodiment, the mole fraction of amino acids of the Terpolymers is about what is preferred for Copolymer 1. The mole fraction of amino acids in Copolymer 1 is glutamic acid (about 0.14), alanine (about 0.43), tyrosine (about 0.10) and lysine (about 0.34). The most preferred average molecular weight for Copolymer 1 is between about 5,000 and about 9,000 daltons.

The molar ratios of the monomers of the more preferred terpolymer of glutamic acid, alanine and tyrosine is about 0.21 to about 0.65 to about 0.14.

The molar ratios of the monomers of the more preferred terpolymer of glutamic acid, alanine and lysine is about 0.15 to about 0.48 to about 0.36.

The molar ratios of the monomers of the more preferred terpolymer of glutamic acid, tyrosine, and lysine is about 0.26 to about 0.16 to about 0.58.

The molar ratios of the monomers of the more preferred terpolymer of tyrosine, alanine and lysine is about 0.10 to about 0.54 to about 0.35.

In one embodiment, the Terpolymers and Copeptides of the present invention are capable of binding to an MHC class II protein which, preferably, is associated with an autoimmune disease. Any available method can be used to ascertain whether the Terpolymer or Copeptide binds to one or more MHC class II proteins. For example, the polypeptide can be labeled with a reporter molecule (such as a radionuclide or biotin), mixed with a crude or pure preparation of MHC class II protein and binding is detected if the reporter molecule adheres to the MHC class II protein after removal of the unbound polypeptide.

In another embodiment, the Terpolymers and Copeptides of the invention are capable of binding to an MHC class II protein associated with multiple sclerosis. A polypeptide of this embodiment can have similar or greater affinity for the antigen binding groove of an MHC class II protein associated with multiple sclerosis than does Copolymer 1. Hence, the contemplated polypeptide can inhibit binding of or displace the binding of myelin autoantigens from the MHC class II protein. One MHC class II protein associated with multiple sclerosis is HLA-DR4 (DRB1*1501).

In another embodiment, Copolymer 1, the Terpolymers and Copeptides of the invention are capable of binding to an MHC class II protein associated with an arthritic condition, for example, rheumatoid arthritis or osteoarthritis. Copolymer 1 a Terpolymer or Copeptide of this embodiment can have a greater affinity for the antigen binding groove of an MHC class II protein associated with the autoimmune disease than does a type II collagen 261-273 (SEQ ID NO:3) peptide. Hence, the contemplated Copolymer 1, Terpolymers or Copeptides can inhibit binding of or displace the type II collagen 261-273 peptide from the antigen binding groove of an MHC class II protein. The Class II MHC protein consists of approximately equalsized $\alpha$ and $\beta$ subunits, both of which are transmembrane proteins. A peptide-binding cleft is formed by parts of the amino termini of both $\alpha$ and $\beta$ subunits. This peptide-binding cleft is the site of presentation of the antigen to T cells. There are at least three types of Class II MHC molecules: HLA-DR, HLA-DQ, and HLA-DP molecules. There are also numerous alleles encoding each type of these HLA molecules. The Class II MHC molecules are expressed predominantly on the surfaces of B lymphocytes and antigen presenting cells such as macrophages.

The Copeptides of this invention are synthetic peptides at least seven amino acid residues in length and capable of binding to an MHC class II protein associated with an autoimmune disease. The Copeptide can include an amino-terminal alanine, and the sequence is selected from the group of amino acid sequences consisting of alanine - glutamic acid-lysine - tyrosine - alanine (AEKYA) (SEQ ID NO: 4); alanine -glutamic acid - lysine - valine - alanine (AEKVA)(SEQ ID NO: 5); alanine - glutamic acid - lysine - phenylalanine - alanine (AEKFA)(SEQ ID NO: 6); lysine - glutamic acid - tyrosine - alanine (KEYA)(SEQ ID NO: 7); lysine - tyrosine - alanine - glutamic acid (KYAE)(SEQ ID NO: 8); lysine - glutamic acid - valine - alanine (KEVA)(SEQ ID NO: 9); lysine - valine - alanine - glutamic acid (KVAE)(SEQ ID NO: 10); lysine - glutamic acid - phenylalanine - alanine (KEFA)(SEQ ID NO: 11); lysine - phenylalanine - alanine - glutamic acid (KFAE)(SEQ ID NO: 12); lysine - tyrosine - alanine - alanine (KYAA) (SEQ ID NO: 13); lysine - lysine - tyrosine - alanine (KKYA)(SEQ ID NO: 14); lysine - valine - alanine - alanine (KVAA)(SEQ ID NO: 15); lysine - lysine - valine - alanine (KKVA)(SEQ ID NO: 16); lysine - phenylalanine - alanine - alanine (KFAA)(SEQ ID NO: 17); and lysine - lysine - phenylalanine - alanine (KKFA)(SEQ ID NO: 18). In this embodiment, the peptide can further comprise two alanine residues, and the sequence can be selected from the group or sequences consisting of alanine - lysine - tyrosine - alanine - glutamic acid (AKYAE)(SEQ ID NO: 19); glutamic acid - alanine - lysine - tyrosine - alanine (EAKYA)(SEQ ID NO: 20); alanine - lysine. - valine - alanine - glutamic acid (AKVAE)(SEQ ID NO: 21); glutamic acid - alanine - lysine - valine - alanine (EAKVA)(SEQ ID NO: 22); alanine - lysine - phenylalanine - alanine - glutamic acid (AKFAE)(SEQ ID NO: 23); and glutamic acid - alanine - lysine - phenylalanine - alanine (EAKFA)(SEQ ID NO: 24).

In another embodiment, the polypeptide is a Copeptide which has a sequence selected from the group consisting of:

AKEYAAAAAAKAAAA (SEQ ID NO: 25)
AAEYAAAAAAKAAAA (SEQ ID NO: 26)
AAKYAEAAAAKAAAA (SEQ ID NO: 27)
EAKYAAAAAAKAAAA (SEQ ID NO: 28)
AEKYAAAAAAKAAAA (SEQ ID NO: 29)
KEAYAAAAAAKAAAA (SEQ ID NO: 30)
AEEYAAAAAAKAAAA (SEQ ID NO: 31)
EKAYAAAAAAKAAAA (SEQ ID NO: 32)
AAKYEAAAAAKAAAA (SEQ ID NO: 33)
EAAYAAAAAAKAAAA (SEQ ID NO: 34)
EKKYAAAAAAKAAAA (SEQ ID NO: 35)
AKKYEAAAAAAAAAA (SEQ ID NO: 36)
AAEYKAAAAAAAAAA (SEQ ID NO: 37)
AAKYE (SEQ ID NO: 38)
AAKYAE (SEQ ID NO: 39)
AEYAKAAAAAAAAAA (SEQ ID NO: 40)

AEKAYAAAAAAAAAA (SEQ ID NO: 41)
AYKAEAAAAAAAAAA (SEQ ID NO: 42) and
AKYAEAAAAAAAAAA (SEQ ID NO: 43),

wherein the peptide has high affinity for an MHC class II protein.

Yet another embodiment of the invention is a Copeptide having any of the preceding sequences in which the tyrosine (Y) has been substituted by valine (F) or phenylalanine (F).

Another embodiment of the invention provides a Copeptide with an amino acid sequence capable of inhibiting the immune response to an autoantigen in a mammal, wherein the identity and position of at least one amino acid in the polypeptide sequence corresponds to the identity and position of at least one amino acid in the peptide binding groove of an MHC class II protein. (In this embodiment, the Copeptide can contain other amino acids as indicated below). For treating multiple sclerosis or rheumatoid arthritis, the MHC class II protein can be selected from the group consisting of an HLA-DR1 protein, an HLA-DR4 protein or an HLA-DR2 protein. In a preferred embodiment, the identity and position of at least one amino acid in the polypeptide corresponds to the identity and position of an amino acid in the P1 pocket of the MHC class II peptide binding groove. More preferably, the Copeptide has a tyrosine, a valine, or a phenylalanine corresponding to the position of the P1 pocket of the MHC class II peptide binding groove. This embodiment further provides an Copeptide composition wherein the amino acid residue corresponding to the first amino acid of the P1 pocket in the MHC class II peptide binding groove is alanine. The embodiment further provides a peptide, wherein the amino acid which corresponds to the eighth amino acid of the PI pocket in the MHC class II peptide binding groove is lysine or alanine, and the amino acid residue that corresponds to the PI pocket is tyrosine, valine, or phenylalanine.

Another example of this invention provides a composition containing a first Copeptide and a second Copeptide wherein the first Copeptide has a lysine and the second Copeptide has an alanine at the amino acid position corresponding to the eighth amino acid beyond the P1 pocket in the MHC class II peptide binding groove.

The present Terpolymers and Copeptides can be formulated into pharmaceutical compositions containing a pharmaceutically acceptable carrier. As used herein, "pharmaceutically acceptable carrier" includes any and all solvents, dispersion media, coatings, antibacterial and antifungal agents, isotonic and absorption delaying agents, sweeteners and the like. The pharmaceutically acceptable carriers may be prepared from a wide range of materials including, but not limited to, flavoring agents, sweetening agents and miscellaneous materials such as buffers and absorbents that may be needed in order to prepare a particular therapeutic composition. The use of such media and agents with pharmaceutically active substances is well known in the art. Except insofar as any conventional media or agent is incompatible with the active ingredient, its use in the therapeutic compositions is contemplated. Supplementary active ingredients can also be incorporated into the compositions.

The present compositions may be formulated as an injectable solution or suspension, a spray solution or a suspension.

Therapeutic Methods Contemplated by the Invention

The present invention further provides methods for treating and preventing autoimmune diseases in a mammal which include administering a therapeutically effective amount of a composition having a polypeptide containing at least three different amino acids selected from the group consisting of the amino acids which comprise Copolymer 1, namely glutamic acid, tyrosine, lysine, and alanine, wherein the selected amino acids are randomly polymerized in a linear configuration. In one embodiment the polypeptide is Copolymer 1 or a Terpolymer.

Autoimmune diseases contemplated by the present invention include either cell-mediated disease (e.g. T-cell) or antibody-mediated (e.g. B cell) disorders. Such disorders can be *inter alia* arthritic conditions, demyelinating diseases and inflammatory diseases. For example, autoimmune diseases which can be treated by the present polypeptides include multiple sclerosis, autoimmune hemolytic anemia, autoimmune oophoritis, autoimmune thyroiditis, autoimmune uveoretinitis, Crohn's disease, chronic immune thrombocytopenic purpura, colitis, contact sensitivity disease, diabetes mellitus, Graves disease, Guillain-Barre's syndrome, Hashimoto's disease, idiopathic myxedema, myasthenia gravis, psoriasis, pemphigus vulgaris, rheumatoid arthritis, or systemic lupus erythematosus, The present compositions can be used to treat one or more of these diseases.

The term "arthritic condition" as used herein is a condition wherein at least one symptom of rheumatoid arthritis is observed in at least one joint of a mammal, for example in a shoulder, knee, hip, backbone or a digit of the mammal. Examples of arthritic conditions include "Polyarthritis", which is an arthritic condition that affects more than a single joint; "juvenile arthritis", an arthritic condition of humans under the age of 21; and Felty's syndrome, which can include the symptoms of neutropenia, splenomegaly, weight loss, anemia, lymphadenopathy, and pigment spots on the skin.

In one embodiment, any autoimmune disease can be treated by the present polypeptides so long as the contemplated polypeptide binds to an MHC class II protein that has been associated with the autoimmune disease. One aspect of this embodiment provides a method which includes selecting a polypeptide that inhibits binding of an antigenic peptide to an MHC class II protein, for example, a method wherein step (a) further comprises selecting the heteropolymer that inhibits class II-specific T cell responses to an MHC class II protein-peptide complex, and a method wherein the antigenic peptide is associated with an autoimmune disease; in another embodiment of the invention, a method is provided wherein the MHC class II protein is associated with an autoimmune disease.

In another embodiment, the method for treating an autoimmune disease in a mammal further involves inhibiting the proliferation or function of T cells which are responsive to an autoantigen. RA is a T cell-mediated autoimmune disease which can be treated with the present polypeptides. The pathological process of autoimmune diseases and immune rejection is mediated by T cells. Upon binding to and recognition of an antigen, T cells proliferate, secrete cytokines and recruit additional inflammatory and cytotoxic cells to the site. The present polypeptides prevent T cell proliferation and T cell functions such as cytokine secretion and recruitment of inflammatory and cytotoxic cells to the site. When the autoimmune disease is an arthritic condition the autoantigen can be collagen, and the present polypeptides can inhibit the proliferation and function of collagen-responsive T cells.

In another embodiment, the method for treating an autoimmune disease in a mammal involves binding the polypeptide to an antigen presenting cell such as a macrophage, a dendritic cell of the lymphoid tissue or an epidermal cell. The proliferation and functions of a T cell are activated when an appropriate antigen is presented to it. By binding to antigen presenting cells, the present polypeptides may block or otherwise interfere with T cell activation.

In yet another embodiment, the method for treating an autoimmune disease in a mammal involves binding the polypeptide to a major histocompatibility complex class II protein which is associated with an autoimmune disease. The Class II MHC proteins are expressed predominantly on the surfaces of B lymphocytes and antigen presenting cells such as macrophages. These Class II MHC proteins have a peptide-binding cleft which is the site at which antigenic peptides are presented to T cells. When the present polypeptides bind to a major histocompatibility complex class II protein, those polypeptides can block or otherwise interfere with antigen presentation and/or T cell activation.

In another embodiment, the method for treating an autoimmune disease in a mammal involves binding the polypeptide to Copolymer 1-reactive B cell antibodies, and/or Copolymer 1-reactive T cells. Copolymer 1-reactive $T_H2/3$ T cells facilitate the therapeutic effects of Copolymer 1. When binding to Copolymer 1-reactive T cells, the present polypeptides stimulate those T cells proliferate, secrete antiinflammatory cytokines and enhance the therapeutic benefits of treatment by the present methods. According to the present invention, the present polypeptides also bind to autoantigen-reactive antibodies which may block the antibody from attacking the target tissue, thereby helping to prevent the autoimmune disease from progressing. For example, when the present polypeptides are bound to MBP-specific antibodies, those antibodies may not bind to MBP and thereby lead to the destruction of MBP in the myelin sheath.

The present polypeptides may be administered by any convenient route. In one embodiment the present polypeptides and peptides can be administered by injection to facilitate delivery to the tissues affected by the autoimmune disease. Thus, the present peptides and polypeptides may, for example, be injected, ingested, inhaled, or topically applied. The subject polypeptides may be incorporated into a cream, solution or suspension for topical administration. The present polypeptides are preferably administered orally, topically or by injection without addition of an adjuvant.

Useful Kits of the Invention

Another embodiment of the invention, provides a kit for assaying the binding of an analyte to an MHC protein, which includes a water-soluble MHC protein which has been recombinantly produced in a non-mammalian cell, and a means for detection of the bound analyte on the MHC protein, and instructions for use. The MHC protein used in the kit is an MHC class II protein selected from the group consisting of an MHC class II HLA-DR1 protein, an MHC class II HLA-DR2 protein and an MHC class II HLA-DR4 protein. The kit can further comprise an autoantigenic peptide.

In a preferred embodiment, the MHC class II protein is produced in an invertebrate or a microbial cell, such as an insect cell or a yeast cell and is therefore devoid of bound peptide in the antigen cleft. The means for detecting binding of the analyte to the MHC protein can be any radioactive, fluorimetric, chemiluminescent, enzymatic or colorimetric means known to one of ordinary skill in the art. In a preferred embodiment, the MHC protein is a class II HLA-DR1 or HLA-DR4 protein. Further, the kit can include also an autoantigenic peptide, such as a collagen II peptide, or a peptide derived from myelin basic protein, myelin oligodendrite protein, or a peptide from some other protein implicated in an autoimmune disease.

Synthesis of the Terpolymers of the invention

The Terpolymers can be made by any procedure available to one of skill in the art. For example, the Terpolymers can be made under condensation conditions using the desired molar fraction of amino acids in solution or by solid phase synthetic procedures. Condensation conditions include the proper temperature, pH and solvent conditions for condensing the carboxyl group of one amino acid with the amino group of another amino acid to form a peptide bond. Condensing agents, for example, dicyclohexyl-carbodiimide, can be used to facilitate the formation of the peptide bond. Blocking groups can be used to protect functional groups, such as the side chain moieties and some of the amino or carboxyl groups, against undesired side reactions.

For example, the process disclosed in U.S. Patent 3,849,550 can be used where the N-carboxyanhydrides of tyrosine, alanine, γ-benzyl glutamate and N-trifluoroacetyl-lysine are polymerized at ambient temperatures in anhydrous dioxane with diethylamine as an initiator. The γ-carboxyl group of the glutamic acid can be deblocked by hydrogen bromide in glacial acetic acid. The trifluoroacetyl groups are removed from lysine by 1 molar piperidine. One of skill in the art readily understands that the process can be adjusted to make peptides and polypeptides containing the desired amino acids, for example, only three of the four amino acids in Copolymer 1 by selectively eliminating the reactions that relate to any one of glutamic acid, alanine, tyrosine or lysine. For purposes of the application, the terms "ambient temperature" and "room temperature" mean a temperature ranging from about 20 to about 26 degrees Centigrade (°C).

The average molecular weight of the Terpolymers can be adjusted during polypeptide synthesis or after the Terpolymers have been made. To adjust the average molecular weight during polypeptide synthesis, the synthetic conditions or the amounts of amino acids are adjusted so that synthesis stops when the polypeptide reaches the approximate length which is desired. After synthesis, polypeptides with the desired average molecular weight can be obtained by any available size selection procedure, for example, chromatography of the polypeptides on a molecular weight sizing column or gel, and collection of the average molecular weight ranges desired. The present polypeptides can also be partially hydrolyzed to remove high molecular weight species, for example, by acid or enzymatic hydrolysis, and then purified to remove the acid or enzymes.

In one embodiment, the present invention provides a process for preparing Terpolymers with a desired average molecular weight which includes reacting a protected polypeptide with hydrobromic acid to form polypeptide having the desired average molecular weight profile. The reaction is performed for a time and at a temperature which is predetermined by one or more test reactions. During the test reactions, the time and temperature are varied and the average molecular weight range of a given batch of test polypeptides is determined. The test conditions which provide the optimal average molecular weight range for that batch of polypeptides are used for the batch. Thus, polypeptides having the desired average molecular weight profile, can be produced by a process which includes reacting the protected polypeptide with hydrobromic acid for a time and at a temperature predetermined by test reaction.

In a preferred embodiment, a test sample of protected polypeptide from a given batch is reacted with hydrobromic acid for about 10-50 hours at a temperature of about 20-28°C. The best conditions for that batch are determined by running several test reactions. For example, in one embodiment, the protected polypeptide is reacted with hydrobromic acid for about 17 hours at a temperature of about 26°C.

The examples which follow describe the invention in detail with respect to showing how certain specific representative embodiments thereof can be made, the materials, apparatus and process steps being understood as examples that are intended to be illustrative only. In particular, the invention is not intended to be limited to the methods, materials, conditions, process parameters, apparatus and the like specifically recited herein.

EXAMPLE I

REPARATION OF POLYPEPTIDES

**Preparation of Protected Polypeptides**

Protected polypeptides are prepared as described by Teitelbaum et al.,using the N-carboxyanhydride (NCA) blocked amino acids tyrosine, alanine, glutamic acid and trifluoroacetyllysine dissolved in dioxane. 1 EUR. J. IMMUN. 242 (1971). The carboxylate group on glutamic acid is blocked with a benzyl (BZ) group.

The polymerization process is initiated by the addition of 0.01 - 0.02% diethylamine. The reaction mixture is stirred at room temperature for 20 hours and then poured into water. The protected polypeptide product is filtered, washed with water and dried. The removal of the γ-benzyl blocking groups from the glutamate residue is carried out by treating the protected polypeptide with 33% hydrobromic acid in glacial acetic acid at room temperature for 6-12 hours with stirring. The product is poured into excess water, filtered, washed and dried, yielding the protected polypeptide.

**Preparing Polypeptides with Molecular Weight 7,000 ± 2,000 Da**

Treatment of γ-benzyl protected polypeptides 33% HBr in acetic acid not only insures removal of the benzyl protecting group from the γ-carboxylate of the glutamate residue but also cleaves the polypeptides into smaller polypeptides.

The time needed for obtaining a polypeptide of molecular weight 7,000 ± 2,000 daltons depends on the reaction temperature and the size of protected polypeptide. At temperatures of between 20°C to 28°C a test reaction is performed on every batch for different time periods, for example, from 10-50 hours. A curve of average molecular weight obtained over time is drawn. The time needed for obtaining molecular weight 7,000 ± 2,000 Da is calculated from the curve and the reaction is performed on a larger scale. On average, at 26°C, the time period for obtaining a mixture of polypeptides with a molecular weight of 7,000 ± 2,000 Da is 17 hours. The product is poured into excess water, filtered, washed and dried, yielding a polypeptide with a desired range of average molecular weights.

**Preparation of low-toxicity lysine-containing polypeptides**

Protected trifluoroacetyl-polypeptide (20 g) is dispersed in 1 liter of water and 100 g piperidine is added. The mixture is stirred for 24 hours at room temperature and filtered. The solution of crude polypeptide is distributed into dialysis bags and dialyzed at 10-20°C in water until pH 8 is attained. The polypeptide solution is dialyzed in 0.3% acetic acid and then again in water until pH 5.5 to 6.0 is obtained. This solution is then concentrated and lyophilized to dryness.

**Synthesis of: Molecular weight 7,600 TGA, poly[L-Tyr$^{0.136}$, L-Glu$^{0.21}$, L-Ala$^{0.648}$]**

1) Raw Materials:

| | |
|---|---|
| L-Ala-NCA | 18.74 g |
| L-Tyr-NCA | 6.5 g |
| 5-BZ-L-Glu-NCA | 13 g |
| Diethylamine | 0.165 g |
| Dioxane | 790 ml |
| Hbr/AcOH (33%) | 480 ml |
| Phenol | 4.8 g |
| Piperidine | 13.2 ml |
| Deionized water | |
| Acetic acid | |

2) Procedure:
L-Tyr-NCA (6.5 g) is placed in dioxane (211 ml) and heated to 60°C for 10 min, then filtered. 5-BZ-L-Glu-NCA (13 g) is placed in dioxane (226 ml) and stirred at 20-25 °C for 10 min, then filtered. L-Ala-NCA (18.74 g) is placed in dioxane (350 ml) and stirred at 20-25°C for 10 min, then filtered.
A 2L Erlenmeyer equipped with a magnetic stirrer, is charged with the solution of L-Tyr-NCA, the solution of 5-BZ-L-Glu-NCA and the solution of L-Ala-NCA. Diethylamine (0.165 g) in dioxane (2.5 ml) is introduced to the reaction mixture and stirred at 20-25 °C for 20 hours. The reaction mixture is added to deionized water (1L), filtered and dried at 60°C under vacuum. Yield - 25.8g.
A solution of phenol (4.8 g) in Hbr/AcOH (33%;480 ml) is stirred for 20 hours. A 2L Erlenmeyer equipped with a magnetic stirrer, is charged with a solution of Hbr/AcOH, poly[5-BZ-L-Glu,L-Ala,L-LTyr] and stirred at 26±1 °C for 16 hours. The reaction mixture is added to deionized water (2L) and stirred for 1 hour. The precipitate is filtered and washed with deionized water until the pH is 6. The product is dried in a circulating air oven at 40°C, for about 40 hours. Yield - 24 g.
A 2L Erienmeyer equipped with a magnetic stirrer, is charged with a piperidine (13.2 ml), deionized water (1200 ml), and poly[L-Glu,L-Ala,L-LTyr], then stirred at 20-25°C for 24 hours. The resulting solution is ultrafiltered through polyethersulphone membranes with a cutoff of 5000 daltons until two thirds of the original volume is removed. The original volume is restored by addition of fresh deionized water. The process is repeated 5 times, until the impurity content is less than 1% by HPLC. The resulting solution (at full volume) is acidified to pH 4.4 with acetic acid. The solution is ultrafiltered to pH 5.5-6 and the volume is reduced to one third. The resulting solution is lyophilized to dryness.

**Synthesis of Molecular Weight 8850 GAL, poly[L-Glu$^{0.153}$,L-Ala$^{0479}$,L-Lys$^{0.365}$]**

1) Raw Materials

| | |
|---|---|
| L-Ala-NCA | 14 g |
| N6-TFA-L-Lys-NCA | 22.9 g |
| 5-BZ-L-Glu-NCA | 9.8 g |
| Diethylamine | 0.12 g |

(continued)

| | |
|---|---|
| Dioxane | 850 ml |
| Hbr/AcOH (33%) | 480 ml |
| Phenol | 4.8 g |
| Piperidine | 13.2 ml |
| Deionized water | |
| Acetic acid | |

2) Procedure

N6-TFA-L-Lys-NCA (22.9 g) in dioxane (420 mi) is stirred at 20-25°C for 10 min. and filtered. 5-BZ-L-Glu-NCA (9.8 g) in dioxane (170 ml) is stirred at 20-25°C for 10 min. and filtered. L-Ala-NCA (14 g) in dioxane (260 ml) is stirred at 20-25°C for 10 min. and filtered. A 2L Erlenmeyer equipped with a magnetic stirrer, is charged with the solution of N6-TFA-L-Lys-NCA, the solution of 5-BZ-L-Glu-NCA and the solution of L-Ala-NCA. Diethylamine (0.12 g) in dioxane (2.5 ml) is added, and the reaction mixture is stirred at 20-25°C for 20 hours. The reaction mixture is added to deionized water (1L), filtered and dried at 60°C under vacuum.

A solution of phenol (4.8 g) in Hbr/AcOH (33%;480 ml) is stirred for 20 hours. A 2L Erlenmeyer equipped with a magnetic stirrer, is charged with a solution of Hbr/AcOH, poly[5-BZ-L-Glu,L-Ala,N6-TFA-L-Lys] (24 g) and is stirred at 26±1 °C for 16 hours. The reaction mixture is added to deionized water (2L) and stirred for 1 hour. The precipitate is filtered and washed with deionized water until pH=6. The product is dried in a circulating air oven at 40°C, about 40 hours.

A 2L Erlenmeyer equipped with a magnetic stirrer, is charged with piperidine (13.2 ml), deionized water (1200 ml) and poly[L-Glu,L-Ala,N6-TFA-L-Lys] (24 g) is stirred at 20-25°C for 24 hours. The solution is ultrafiltered through polyethersulphone membranes with a cut off of 5000 daltons until two thirds of the original volume are removed. The original volume is restored by addition of fresh deionized water. The process is repeated 5 times, until the impurity content is less than 1% (by HPLC). The resulting solution (at full volume) is acidified to pH 4.4 with acetic acid. The solution is ultrafiltered until the pH is 5.5-6 and the volume is reduced to one third. The resulting solution is lyophilized to dryness.

**Synthesis of Molecular Weight 11,050 TGL, poly[L-Tyr$^{0.162}$, L-Glu$^{0.259}$,L-Lys$^{0.579}$]**

1) Raw Materials

| | |
|---|---|
| 5-BZ-L-Glu-NCA | 10.34 g |
| N6-TFA-L-Lys-NCA | 24.16 g |
| L-Tyr-NCA | 5.2 g |
| Diethylamine | 0.095 g |
| Dioxane | 810 ml |
| Hbr/AcOH (33%) | 460 ml |
| Phenol | 4.6 g |
| Piperidine | 150 ml |
| Deionized water | |
| Acetic acid | |

2) Procedure

L-Tyr-NCA (5.2 g) in dioxane (180 ml) is heated to 60°C for 10 min, and filtered. N6-TFA-L-Lys-NCA (24.16 g) in dioxane (450 ml) is stirred at 20-25°C for 10 min. and filtered. 5-BZ-L-Glu-NCA (10.35 g) in dioxane (180 ml) is stirred at 20-25°C for 10 min. and filtered.

A 2L Erlenmeyer equipped with a magnetic stirrer, is charged with the solution of N6-TFA-L-Lys-NCA, the solution of L-Tyr-NCA and the solution of 5-BZ-L-Glu-NCA. Diethylamine (0.095 g) in dioxane (2.5 ml) is introduced, and the reaction mixture is stirred at 20-25°C for 20 hours. The reaction mixture is added to deionized water (0.7L), filtered and dried at 60°C under vacuum.

A solution of phenol (4.6 g) in Hbr/AcOH (33%;460 ml) is stirred for 20 hours. A 2L Erlenmeyer equipped with a magnetic stirrer, is charged with the solution of HB/r/AcOH and the poly[5-BZ-L-Glu, L-Tyr, N6-TFA-L-Lys]. This mixture is stirred at 26±1 °C for 16 hours and then the mixture is added to deionized water (1.5 L) and stirred for ½ hour. The precipitate is filtered and washed with deionized water until the pH is 5.5.

A 2L Erlenmeyer equipped with a magnetic stirrer, is charged with a piperidine (150 ml), deionized water (1400 ml), and poly[L-Glu,L-Tyr,N6-TFA-L-Lys] (50 g) and is stirred at 20-25°C for 24 hours. The resulting solution of poly[L-Glu, L-Tyr, L-Lys] is ultrafiltered through polyethersulphone membranes with a cut off of 5000 daltons until two thirds of the original volume were removed. The original volume is restored by addition of fresh deionized water. The process is repeated 5 times, until the impurity content is less than 1% (by HPLC). The resulting solution (at full volume) is acidified to pH 4.2 with acetic acid. The solution is ultrafiltered to a pH of 5.5-6 and the volume is reduced to one third. The resulting solution is lyophilized to dryness.

**Synthesis of Molecular Weight 20,000 TAL, poly[L-Tyr$^{0.102}$, L-Ala$^{0.542}$, L-Lys$^{0.353}$]**

1) Raw Materials

| | |
|---|---|
| L-Ala-NCA | 14 g |
| N6-TFA-L-Lys-NCA | 22.9 g |
| L-Tyr-NCA | 4.9 g |
| Diethylamine | 0.1 g |
| Dioxane | 850 ml |
| Hbr/AcOH (33%) | 500 ml |
| Phenol | 5 g |
| Piperidine | 162 ml |
| Deionized water | |
| Acetic acid | |

2) Procedure

L-Tyr-NCA (4.9 g) in dioxane (170 ml) is heated to 60°C for 10 min, and filtered. N6-TFA-L-Lys-NCA (22.9 g) in dioxane (417 ml) is stirred at 20-25°C for 10 min. and filtered. L-Ala-NCA (14 g) in dioxane (260 ml) is stirred at 20-25°C for 10 min. and filtered.

A 2L Erlenmeyer equipped with a magnetic stirrer, is charged with the solution of N6-TFA-L-Lys-NCA, the solution of Tyr-NCA in dioxane and the solution of L-Ala-NCA. Diethylamine (0.1 g) in dioxane (2 ml) is introduced and the mixture is stirred at 20-25°C for 20 hours. The reaction mixture is then added to deionized water (1L), filtered and dried at 60°C under vacuum.

A solution of phenol (5 g) in HBr/AcOH (33%;500 ml) is stirred for 20 hours. A 2L Erlenmeyer equipped with a magnetic stirrer, is charged with the solution of HB/r/AcOH and the paly[L-Ala,L-Tyr,N6-TFA-L-Lys] and is stirred at 26±1 °C for 16 hours. The reaction mixture is then added to deionized water (2L) and stirred for ½ hour. The precipitate is filtered and washed with deionized water until the pH is 5.5.

A 2L Erlenmeyer equipped with a magnetic stirrer, is charged with a piperidine (162 ml), water (1500 ml) and the poly[L-Ala,L-Tyr,N6-TFA-L-Lys] (30 g), and is stirred at 20-25°C for 24 hours. The resulting solution of poly[L-Ala,L-Tyr,L-Lys] is ultrafiltered through polyethersulphone membranes with a cut off of 5000 daltons until two thirds of the original volume were removed. The original volume is restored by addition of fresh deionized water. The process is repeated 5 times, until the impurity content is less than 1% (by HPLC). The resulting solution (at full volume) is acidified to pH 4.4 with acetic acid. The solution is ultrafiltered to pH 5.5-6 and the volume is reduced to one third. The resulting solution is lyophilized to dryness.

**EXAMPLE 2**

**LOW MOLECULAR WEIGHT POLYPEPTIDES**

**In Vivo Tests**

Three batches of copolymer-1 having an average molecular weight of 7.3 and 8.4 KDa (less than 2.5% copolymer-1 species over 40KDa) and 22KDa (more than 5% copolymer-1 species over 40KDa) were subjected to the toxicity test described below. Five mice were used in each experimental group.

**Method**

Copolymer-1 is dissolved in distilled water to yield a solution of 2 mg/ml of the active ingredient. Each mouse is injected with 0.5 ml of the test solution into the lateral tail vein. Mice were observed for mortality and relevant clinical signs over a 48 hour period. Observations were recorded 10 minutes, 24 hours and 48 hours post-injection. If, at the end of 48 hours, all the animals were alive and no adverse signs had been observed, then the batch is designated "non-toxic". If, however, one or more of the mice had died or had shown adverse signs, then the batch is designated "toxic".

**Results**

Three of five mice died after 48 hours when treated with the 22 kDa average molecular weight Copolymer 1 polypeptides. Accordingly, this high average molecular weight batch is designated "toxic". The Copolymer 1 batches having average molecular weights of 7.3 and 8.4 KDa were both designated "non-toxic."

**In Vitro Rat Basophilic Leukemia Cell Degranulation Tests**

Histamine (or serotonin) release from basophile is an in vitro model for immediate hypersensitivity. The Rat Basophilic Leukemia cell line, RBL-2H$_o$, is uniform and easy to maintain in culture but is a highly sensitive and reproducible system for testing for degranulation. E.L. Basumian, et al., 11 EuR. J. IMMUNOL. 317 (1981). The physiological stimulus for histamine release involves binding of the antigen to membrane-bound IgE molecules, which triggers an intricate biochemical cascade. Degranulation is induced by non-IgE-mediated stimuli, including various peptides and synthetic polymers, e.g. polylysine. R.P. Siraganian, TRENDS IN PHARMACOLOGICAL SCIENCES 432 (Oct. 1983). The RBL degranulation test is, therefore, used to screen out those batches of COP-1 which evoke substantial degranulation and thus might elicit undesirable local and/or systemic side effects.

**Method**

Rat Basophilic Leukemia cells (RBL-2H$_o$), are loaded with [H$^3$]-serotonin, followed by incubation with 100 $\mu$g of the COP-1 to be tested. Batches of COP-1 which induce non-specific degranulation, release [H$^3$]-serotonin into the medium. The radioactivity in the medium is counted by a scintillation counter and the total radiolabeled serotonin incorporated into the cells is determined in the pelleted cells. Percent degranulation is calculated as the percentage of serotonin released out of the total incorporated.

**Results**

Four batches of Copolymer 1, with average molecular weight between 8,250-14,500 were analyzed for both percentage of the species with molecular weight over 40 kDa and for degranulation of RBL's. Results are summarized in Table 1.

**Table 1**

| Average M.W. | % of species with M.W. over 40 KDa | % Serotonin Release |
|---|---|---|
| 6,250 | < 2.5 | 12.4 |
| 7,300 | < 2.5 | 21.0 |
| 13,000 | > 5 | 66.9 |
| 14,500 | > 5 | 67.8 |

As can be seen, when the percentage of high average molecular weight species is low (less than 2.5%), the percent release of serotonin is also low, and vice versa. These data indicate that lower average molecular weight Copolymer 1 polypeptides are preferable to higher average molecular weight Copolymer 1 polypeptides.

**EXAMPLE 3**

**SUPPRESSION OF EAE BY THE POLYPEPTIDES**

Injection of Copolymer 1 in incomplete Freund's adjuvant before disease induction can suppress experimental allergic encephalomyelitis (EAE). This suppression appears to be mediated by Copolymer 1-specific suppressor T cells of the $T_H2$ type which cross react with myelin basic protein. Lando et al., 123 J. IMMUNOL. 2156 (1979); Aharoni et al., 17 EUR. J. IMMUNOL. 23 (1993); Aharoni et al., 94 PROC. NATL. ACAD. SCI. USA 10821 (1997). Other researchers have observed that the therapeutic effect of Copolymer 1 in multiple sclerosis patients is also associated with the induction of $T_H2$ cells. Lahat et al., 244 J. Neurol. 129 (1997). In this example, EAE is suppressed to by different polypeptides of the present invention.

## METHODS

### Copolymer 1

Copolymer 1 batches # 02095 and 55495, with average molecular weights of 6000 Da and 5800 Da, respectively, were obtained from Teva Pharmaceutical Industries (Petach Tikva, Israel).

### Terpolymers

Four terpolymers were obtained from Teva Pharmaceutical Industries (Petach Tikva, Israel). The properties of these terpolymers are provided below:

1) The GAL terpolymer (SD-1689) is a mixture of polypeptides containing the following mole fraction of amino acids glutamic acid (0.153), alanine (0.479) and lysine (0.365). The range of GAL average molecular weights is about 4650 daltons to about 20,050 daltons. The average molecular weight of this GAL preparation is 8850 daltons.
2) The TGA terpolymer (SD-1690) is a mixture of polypeptides containing the following mole fraction of amino acids tyrosine (0.136), glutamic acid (0.210) and alanine (0.648). The range of TGA average molecular weights is about 1000 daltons to about 40,000 daltons. The average molecular weight of this TGA preparation is 7600 daltons.
3) The TAL terpolymer (SD-1691) is a mixture of polypeptides containing the following mole fraction of amino acids tyrosine (0.102), alanine (0.542) and lysine (0.353). The range of TAL average molecular weights is about 5700 daltons to about 34,400 daltons. The average molecular weight of this TAL preparation is about 20,000 daltons.
4) The GTL terpolymer (SD-1697) is a mixture of polypeptides containing the following mole fraction of amino acids glutamic acid (0.259), tyrosine (0.162) and lysine (0.579). The range of GTL average molecular weights is about 4,000 daltons to about 23,500 daltons. The average molecular weight of this GTL preparation is about 11050 daltons.

A control polypeptide is used, consisting of a mixture of polypeptides containing a 1:1:1 mixture of amino acids alanine, glutamic acid and tyrosine, with an average molecular weight 26,700 Da. This polypeptide is obtained from Sigma Chemical Company (St. Louis, MO).

### Induction of EAE

Two to three month old female (SJL/JxBALB/c)FI mice are injected in all four footpads with mouse spinal cord homogenate (3.5mg/mouse) emulsified in a 1:1 ratio in complete Freund's adjuvant (CFA) supplemented with 4 mg/ml H37Ra. Pertussis toxin (0.25ml, 250ng, Sigma) is injected intravenously, immediately after and 48 hr later. Mice are examined daily from day 10 post induction for clinical signs of EAE which were scored on a 0-5 scale as described in Lando et al., 123 J. IMMUNOL. 2156 (1979).

### EAE suppression by injection with incomplete adjuvant

The tested polypeptides (10mg/mouse) are injected in incomplete Freund's adjuvant (ICFA) subcutaneously in one nuchal area in 2-3 spots. EAE is induced 3 weeks later as described above.

### EAE suppression by oral administration

In a second test, female Lewis rats are fed 5 mg/kg guinea pig BP or Copolymer 1 dissolved in phosphate buffered saline (PBS) at 2-3 day intervals before EAE induction. EAE is induced two days after the last feeding by injection of 25 $\mu$g guinea pig MBP emulsified in 1:1 CFA containing 4 mg/ml mycobacterium tuberculosis (H37Ra) (Difco Lab, Detroit, Mich.). A total volume of 0.1 ml is injected into each of two hindfoot pads. Control rats are mock fed with phosphate buffered saline.

The efficacy of orally administered Copolymer 1 for preventing EAE in rats is compared to that of rats fed guinea pig MBP by the method of Higgins et al., 140 J. IMMUNOL. 440 (1988).

Animals were examined daily from day 10 post induction for signs of disease. EAE is scored as follows: 0 = no disease; 1 = limp tail; 2 = hind limb paralysis; 3 = paralysis of all four limbs; 4 = moribund condition; and 5 = death.

## RESULTS

### EAE suppression by injection with incomplete adjuvant

Table 2 illustrates the effects of administering the present polypeptides by injection in ICFA prior to induction of EAE. Of the four polypeptides tested, Copolymer 1 and TAL caused the greatest EAE suppression. GTL also exhibited good suppressive activity. GAL and TGA, were somewhat less effective.

**Table 2**

| Suppression of EAE in mice by Injection of the Present Polypeptides | | | | |
|---|---|---|---|---|
| Polypeptide | Incidence | MMS* | Suppression % | |
| | | | Incidence | MMS* |
| None (Negative Control) | 11/11 | 4.0 | | |
| ICFA (Negative Control) | 11/11 | 3.4 | 0.0 | 15.0 |
| Copolymer 1 (Positive Control) | 2/11 | 0.45 | 82.0 | 89.0 |
| SD-1689 - GAL | 7/8 | 3.5 | 12.5 | 12.5 |
| SD-1690 - TGA | 6/8 | 2.5 | 25.0 | 37.5 |
| SD-1691 - TAL | 3/8 | 1.1 | 62.5 | 72.5 |
| SD-1697 - GTL | 5/8 | 1.5 | 37.5 | 62.5 |
| D-Copolymer 1 | 11/11 | 4.1 | 0.0 | 0.0 |
| *MMS = Mean Maximal Score<br>D-Copolymer 1 is a copolymer of d-lysine, d-tyrosine, d-glutamic acid and d-alanine in the molar ratios of Copolymer 1. | | | | |

Table 3 illustrates the efficacy of orally administered Copolymer 1 in preventing the clinical manifestations of EAE in Lewis rats compared to rats receiving only phosphate buffered saline (PBS) or guinea pig basic protein (GPBP).

**Table 3**

| Suppression of EAE in Rats by Oral Administration of the Present Polypeptides | | | |
|---|---|---|---|
| **Fed Antigen** | **Incidence** | **MMS $\pm$ SD** | **Mean Onset (days)** |
| PBS (Control) | 27/28 (96%) | 1.8 $\pm$ 0.5 | 11.9 |
| GPBP | 10/17 (59%) p=0.0026 | 0.9 $\pm$ 0.5 | 11.4 |
| Copolymer 1 | 13/28 (46%) p=0.00005 | 0.78 $\pm$ 0.45 | 12.6 |
| Each numerical value represents the cumulative results of 3-5 independent experiments. The p values represent the statistical significance of the difference from the control (PBS) group. Mean maximal score is calculated for the entire group. | | | |

These data indicate that the present polypeptides are therapeutically effective for preventing the onset and severity of EAE when administered either orally or by injection.

## EXAMPLE 4

### BINDING TO ANTIGEN PRESENTING CELLS

Several of the present polypeptides bind efficiently to living antigen presenting cells.

## METHODS

### Copolymer 1

Copolymer 1 batches # 02095 and 55495, with average molecular weights of 6000 Da and 5800 Da, respectively, were obtained from Teva Pharmaceutical Industries (Petach Tikva, Israel).

### Terpolymers

GAL, TGA, TAL, GTL and control polypeptides are as described above under Example 3.

### Biotinylation of antigens

Biotinylation of Copolymer 1 and Terpolymers is performed at 0°C with biotin-*N*-hydroxysuccinimide (Sigma) according to Fridkis-Hareli et al. 91 PROC. NATL. ACAD. SCI. USA 4872 (1994).

### Binding of biotinylated antigens to antigen presenting cells

The biotinylated polypeptides were examined for binding to living antigen presenting cells of mouse and human origin, using fluorescently labeled streptavidin and FACS analysis. Adherent spleen cells from (SJL/JxBALB/c)FI mice, or EBV transformed human B cells of DR7,w I I haplotype (1 x $10^6$/100 $\mu$l), were incubated at 37°C for 20 hr. with 50 $\mu$g biotinylated Copolymer 1 or Terpolymers dissolved in 100 $\mu$l PBS containing 0.1% BSA. The cells were then incubated at 4°C for 30 min. with phycoerythrin-conjugated streptavidin (Jackson Immuno Research, West Grove, PA) at a concentration of 0.5 $\mu$g/100 $\mu$l cell suspension. After each incubation the cells were washed three times with PBS containing 0.1% BSA. Thereafter, cells were analyzed by flow cytometry using FACScan (Becton-Dickinson, Mountain View, CA). For each analysis, 5000 cells were examined. Dead cells were excluded on the basis of forward and side-angle light scatter.

### Epstein-Barr virus (EBV) transformed B-cell lines

EBV-transformed B-cell lines were initiated according to Teitelbaum et al., 89 PROC. NATL. ACAD. Sci. USA 137 (1992). Approximately 20 x $10^6$ peripheral blood mononuclear cells were cultured with B95.8 cell line supernatant, for 1 hr at 37 °C. The cells were then washed and cultured in RPMI medium with 10% FCS and cyclosporin A (10 $\mu$g/ml) to deplete T cells.

## RESULTS

Table 4 illustrates the binding of terpolymers and Copolymer 1 polypeptides to living antigen presenting cells, including mouse spleen macrophages and human EBV transformed B-cell lines. Data illustrating both the percent of binding and the intensity of cell staining are provided (Table 4 I+II). TAL bound the most efficiently - - better even than Copolymer 1. GAL and GTL also bound very well - the same or even somewhat better than Copolymer 1. TGA bound well but somewhat less than Copolymer 1.

TAL bound most efficiently both to spleen macrophages of (SJUJ x BALB/c) $F_1$ mice and to EBV-transformed B cells from a normal DR7.w11 donor, as expressed by the intensity of the binding (Table 4).

**Table 4**

| Binding of Terpolymers to antigen presenting cells<br>I. Antigen presenting cells from mouse spleen macrophages | | |
|---|---|---|
| Polypeptide | % Binding | MFI* |
| Copolymer 1 (positive control) | 85 | 493 |
| SD-1689 - GAL | 88 | 600 |
| SD-1690 - TGA | 74 | 39 |
| SD-1691 - TAL | 100 | 1929 |
| SD-1697 - GTL | 90 | 973 |
| *MFI = Mean Fluorescence Intensity | | |

(continued)

| Binding of Terpolymers to antigen presenting cells I. Antigen presenting cells from mouse spleen macrophages | | |
|---|---|---|
| Polypeptide | % Binding | MFI* |
| | | |
| II. Antigen presenting cells from human EBV transformed B-cell line | | |
| Polypeptide | % Binding | MFI* |
| Copolymer 1 (positive control) | 96 | 727 |
| SD-1689 - GAL | 95 | 661 |
| SD-1690 - TGA | 72 | 49 |
| SD-1691 - TAL | 100 | 1438 |
| SD-1697 - GTL | 97 | 1057 |
| *MFI = Mean Fluorescence Intensity | | |

## EXAMPLE 5

### THE PRESENT POLYPEPTIDES BIND TO PURIFIED HUMAN LEUKOCYTE ANTIGENS (HLA)

This example illustrates that polypeptides of the present invention bind to human B cells and to purified human lymphocyte antigens with high affinity, including the HLA-DR1, HLA-DR2 and HLA-DR4 molecules.

### METHODS

### Cell lines and antibodies

Homozygous EBV-transformed human B lymphocyte lines used for immunoaffinity purification of HLA-DR1, -DR2 and -DR4 molecules were LG-2 (DRB1÷), MGAR (DRB1±1501) and Preiss (DRB÷0401/DRB4-0101), respectively.

Cells were grown in RPMI 1640 supplemented with 10% FCS, 2mM glutamine, 50 U/ml penicillin G and 50 μg/ml streptomycin in roller bottles and stored as pellets at -80°C. The anti-DR hybridoma LB3.1 (IgG2b) is grown in serum-free medium (Macrophage-SFM, Gibco BRL). See Gorga et al., 103 CELL. IMMUNOL. 160 (1986).

### Protein purification

Immunoaffinity purification of HLA-DR1, -DR2 and -DR4 molecules is performed, with minor modifications, as previously reported by Gorga et al., 262 J. BIOL. CHEM. 16087 (1987). Briefly, detergent soluble membrane preparations from LG-2, MGAR and Priess cells were passed at a flow rate of approximately 11 ml/hr through a series of columns in the following sequence: Sepharose CL-6B (30 ml), normal mouse serum-Aff-gel 10 (10 ml), protein A-Sepharose CL-4B (5 ml) and LB3.1-protein A-Sepharose CL-4B (5 ml). DR2a (DRB5*0101) and Drw53) (DRB4*0101), the products of DR genes linked to the DRB1 alleles were not removed from the MGAR and Priess lysates before passage through the LB3.1 immunoaffinity column, and contaminate the DR2 and DR4 preparations in the amount of 5-10%. All the subsequent steps were as previously described by Gorga et al., 262 J. BIOL. CHEM. 16087 (1987). The eluate is dialyzed against 0.1 % deoxycholate, 10 mM Tris-HCl, pH 8.0 and concentrated on a Centriprep 30 membrane (amicon). Protein concentrations were determined by bicinchonitric acid assay (Pierce Chemical Co.).

### Peptides and polypeptides

### Copolymer 1

Copolymer 1 batches # 55495 and 52596, with average molecular weight of 5800 daltons and 8,150 daltons, respectively, were obtained from Teva Pharmaceutical Industries (Petach Tikva, Israel). Copolymer 1 batch 52596 had a molar ratio of 1 tyrosine: 1.5 glutamic acid: 4.3 alanine: 3.1 lysine.

**Myelin Basic Protein Peptides**

MBP peptides were synthesized on an Applied Biosystems Peptide Synthesizer using solid phase techniques. Barany et al., THE PEPTIDES 1 (1979). Peptides purified by reversed-phase HPLC. The peptides used were HA 306-318, having the sequence PKYVKQNTLKLAT (MW 1718) (SEQ ID NO: 2), and MBP 84 102, having the sequence DENPVVHFFKNIVTPRTPP (MW 2529) (SEQ ID NO: 1).

**Terpolymers**

GAL, TGA, TAL, GTL and control polypeptides are as described above under Example 3.

**Polypeptide labeling**

Biotinylation of the various polypeptides is performed as in Example 4. Unreacted biotin is removed by dialysis (Spectra/Por® membrane MWCO 500, Spectrum Medical Industries).

**Assays for polypeptide binding to class II MAC proteins**

**Solutions:** The solutions used in this assay were the following. Binding buffer is 20 mM 2-[N-morpholino]ethanesulfonic acid (MES), 1% n-octyl β-D-glycopyranoside, 140 mM NaCl, 0.05% $NaN_3$, pH 5.0, unless otherwise specified. PBS is 150 mM sodium chloride, 7.5 mM sodium phosphate, dibasic, 2.5 mM sodium phosphate, monobasic, pH 7.2. TBS is 137 mM sodium chloride, 25 mM Tris pH 8.0, 2.7 mM potassium chloride. TTBS is TBS plus 0.05% Tween-20.

**Microtiter Assay Plate Preparation:** Ninety-six well microtiter immunoassay plates (PRO-BIND™, Falcon) were coated with 1 μg/well affinity-purified LB3.1 monoclonal antibodies in PBS (100 μl total) for 18 hrs at 4°C. The wells were then blocked with TBS containing 3% BSA for 1 hr at 37°C and washed three times with TTBS. Before sample addition, 50 μl of TBS/1% BSA is added to each well.

**Binding reactions:** Detergent-solubilized HLA-DR1, -DR2 and -DR4 molecules (0.5 μg/sample) were incubated with biotinylated peptides at various concentrations for 40 hours at 37°C in 50 μl of the binding buffer and transferred to prepared microtiter assay plates and incubated for 1 hr at 37°C for capture of polypeptide-class II complexes.

**Inhibition reactions:** Biotinylated polypeptides at a final concentration of 1.5 μM in 50 μl of binding buffer were coincubated with unlabeled polypeptides as well as the peptides HA 306-318 (SEQ ID NO: 2) or MBP 84-102 (SEQ ID NO: 1), used as inhibitors, and HLA-DR molecules for 40 hr at 37°C.

**Detection of class II/polypeptide complexes:** Bound polypeptide-biotin is detected using streptavidin-conjugated alkaline phosphatase as follows. Plates were washed three times with TTBS and incubated with 100 μl of streptavidin-conjugated alkaline phosphatase (1:3000, BioRad) for 1 hr at 37°C, followed by addition of p-nitrophenyl phosphate in triethanolamine buffer (BioRad). The absorbency at 410 nm is monitored by a microplate reader (Dynatech MR4000).

**RESULTS**

**Terpolymers Binding to Class II MHC Proteins**

Detergent-soluble HLA-DR1, HLA-DR2 and HLA-DR4 proteins were purified from homozygous EBV-transformed B cell lines LG-2 (DRB1*0101), MGAR (DRB1*1501) and Priess (DRB1*0401), respectively, as described previously by Fridkis-Hareli et al., 160 J. IMMUNOL. 4386 (1998). Three different preparations of Copolymer 1 had bound to these molecules with high affinity. *Id*. To determine the affinity of the terpolymers for HLA-DR proteins, binding assays were carried out with biotinylated Terpolymers, and compared to Copolymer 1. The polypeptides were incubated at a range of concentrations with purified HLA-DR1, HLA-DR2 and HLA-DR4 molecules at pH 5.0 followed by capture with class II-specific mAb and detection with alkaline phosphatase-streptavidin.

Binding by TAL and Copolymer 1 to detergent-soluble HLA-DR1 and HLA-DR4 molecules is better than that of GAL, TGA or GTL. However, GTL and Copolymer 1 bound better than the other polypeptides to HLA-DR2, based on the saturation binding curves (Fig. 2A), and on $K_d$ values, calculated from the double-reciprocal plots of the binding data (Fig. 2B, Table 5).

Competitive binding assays were carried out with biotinylated Copolymer 1 and the following unlabeled inhibitors: Copolymer 1, TAL, TGA, GAL, TGL, MBP 84-102 and HA 306-318 polypeptides (Fig. 3). The MBP 84-102 (SEQ ID NO: 1) peptide is a poor inhibitor of the binding of Copolymer 1 to HLA-DR2. The binding of biotinylated Copolymer 1 to detergent-soluble HLA-DR1 and -DR4 molecules is efficiently inhibited by unlabeled TGA, TAL, HA 306-318 (SEQ ID NO: 2) and Copolymer 1. However, binding of biotinylated Copolymer 1 to detergent-soluble HLA-DR1 and -DR4 molecules is inhibited more than 10-fold less by TGL, as indicated by 50% inhibitory dosages (IC$_{50}$) (Fig. 3, Table 5). Similarly, GAL is also a poor inhibitor of Copolymer 1 binding to HLA-DR1 and -DR4 molecules. In general, the binding pattern to HLA-DR2 is similar to that observed for HLA-DR1 (Table 5). These results show that the polypeptides of three amino acids, in particular TAL and TGA bind to class II MHC molecules with an affinity range similar to that of antigenic peptides and of Copolymer 1. Hence, TAL and TGA are effective competitors for the class II MHC molecules to which Copolymer 1 binds., Based on their binding capacity, the polypeptides could be arranged in the following order:

(A) binding to HLA-DR1: Copolymer 1 > TAL > GTL > TGA >> GAL;

(B) binding to HLA-DR2: Copolymer 1 > GTL > TAL > GAL > TGA;

(C) binding to HLA-DR4: TAL > Copolymer 1 >> TGA > GTL > GAL.

**Table 5**
**Affinity of the present polypeptides for purified human HLA-DR1, -DR2 and -DR4 molecules**

| Polypeptide[1] | DR1[2] | | DR2 | | DR4 | |
|---|---|---|---|---|---|---|
| | $K_d$[3] | IC$_{50}$[4] | $K_d$ | IC$_{50}$ | $K_d$ | IC$_{50}$ |
| Copolymer 1 | 7.4 | 8.8 | 8.2 | 10.1 | 1.5 | 10.8 |
| TAL | 2.0 | 3.3 | 1.7 | 2.7 | 2.0 | 6.5 |
| GAL | 0.5 | [5] | 1.7 | [5] | 0.3 | [5] |
| TGA | 1.0 | 1.3 | 0.8 | 9.5 | 0.8 | 1.0 |
| TGL | 0.4 | 43.0 | 5.0 | 25.4 | 0.8 | 43.0 |

[1]Copolymer 1 polypeptides with average MW of 5,800; TAL, MW 20,000; GAL, MW 8,850; TGA, MW 7,600; and TGL, MW 11,050, were incubated at a range of concentrations with purified HLA-DR1, -DR2 and -DR4 molecules at pH 5.0 followed by capture with class II-specific mAb and peptide detection with alkaline phosphatase-streptavidin.

[2]Detergent-soluble HLA-DR1, -DR2 and -DR4 molecules were purified as described in Materials and Methods.

[3]$K_d$, the dissociation constant at equilibrium, is calculated from the slope of the double reciprocal plot (Fig. 2B), and is expressed as x 10$^{-8}$ M.

[4]IC$_{50}$, inhibitory concentration giving 50% inhibition, is calculated based on the competitive binding assays (Fig. 3), and is expressed as x 10$^{-6}$ M.

[5]IC$_{50}$ values for GAL could not be determined exactly, but were less than 1000 $\mu$M (see Fig. 3).

**Effect of superantigens on the binding of polypeptides to HLA-DR molecules**

Bacterial superantigens SEA, SEB and TSST-1 have been shown to inhibit Copolymer 1 binding to purified HLA-DR molecules only at very high concentrations. Fridkis-Hareli et al., 160 J. IMMUNOL. 4386 (1998). To examine the effect of these superantigens on the binding of Terpolymers to purified HLA-DR1, HLA-DR2 and HLA-DR4 molecules, competitive binding assays were carried out with unlabeled SEA, SEB and TSST-1.

Binding of TAL to HLA-DR1, HLA-DR2 and HLA-DR4 is only inhibited by the superantigens at high molar ratios of superantigen: for example, fifty times the amount of superantigen is needed to inhibit TAL binding (Fig. 4A). However, binding of TGA and GAL is inhibited more significantly by the superantigens (Fig. 4B and C), indicating that these polypeptides bound the HLA antigens with lower affinity.

**EXAMPLE 6**

**INHIBITION OF MBP-INDUCED T CELL PROLIFERATION**

This example illustrates that the proliferation of T cells which are normally activated by myelin basic protein (MBP) can be inhibited by simultaneous exposure to the present polypeptides.

**METHODS**

**Copolymer 1**

Copolymer 1 batches # 02095 and 55495, with average molecular weights of 6000 Da and 5800 Da, respectively, were obtained from Teva Pharmaceutical Industries (Petach Tikva, Israel).

**Myelin Basic Protein Peptides** which were synthesized on an Applied Biosystems Peptides Synthesizer using solid phase techniques. Barany et al., THE PEPTIDES 1 (1979). Peptides purified by reversed-phase HPLC. The peptides used were HA 306-318, having the sequence PKYVKQNTLKLAT (MW 1718)((SEQ ID NO: 2), and MBP 84 102, having the sequence DENPVVHFFKNIVTPRTPP (MW 2529) (SEQ ID NO: 1).

### Terpolymers

GAL, TGA, TAL, GTL and control polypeptides are as described above under Example 3.

### T cell lines and clones

**Myelin Basic Protein (MBP) specifc T-cell lines** originated from spleens of mice which, ten days earlier, were immunized with 20 μg of the 84-102 peptide of MBP emulsified in complete Freund's adjuvant supplemented with 4 mg/ml of Mycobacterium tuberculosis H37Ra. Cells were cultured and selected *in vitro* using the immunizing antigen (0.2-1 mg/plate), in culture medium (RPMI, 2 mM glutamine, 1 mM sodium pyruvate, non essential amino acids, 5 x$10^{-5}$ M 2-mercaptoethanol, 100 /ml penicillin, 100 μg/ml streptomycin), supplemented with 1% autologous serum. After 4 days, cells were transferred to culture medium containing 10% FCS and supplemented with 10% supernatant of Con A activated normal spleen cells as T cell growth factor (TCGF). Every fourteen to twenty one days, cells were stimulated by exposure to the immunizing antigen presented on syngeneic irradiated (3000 rad) spleen cells (50 x $10^6$/plate) for 3 days, followed by propagation in TCGF medium. Cloning of T cell lines is performed by limiting dilution at 0.3 cells/well in microliter plate in the presence of the antigen (2-10 μg/well) and irradiated syngeneic spleen cells (5 x$10^6$/well).

**Human T cell lines** were derived from peripheral blood mononuclear cells according to Teitelbaum et al., 89 PROC. NATL.ACAD. SCI. USA 137 (1992). Approximately 5 x $10^6$ cells were incubated in each well of a 24-well culture plate with Copolymer 1 or MBP (50 μg/ml) in culture medium supplemented with 10% heat-inactivated autologous serum. After 7 days of culture, the cells were transferred to culture medium containing 10% fetal calf serum and recombinant human interleukin 2 (20 units/ml). The cells were grown continuously in this medium with periodic exposure to antigen presented on irradiated (3000 rad), autologous mononuclear cells, every 14-18 days.

### Proliferation assay

Cells of T lines or clones were tested for their specific proliferative response 10-21 days after antigenic stimulation. T Cells (1.5 x $10^4$) were cultured in triplicates with 5 x $10^5$ irradiated spleen cells and with the indicated antigens in a final volume of 0.2 ml in 10% FCS culture medium. At the end of 48 hr incubation, cultures were pulsed with 1 μCi[$^3$H]-thymidine (standard deviation <20% of the mean cpm), and harvested 6-12h later.

### Inhibition studies

Inhibition of the T-cell proliferative response is studied by adding various concentrations of Copolymer 1 and Terpolymers plus the stimulating MBP antigen to the proliferation assay system. Inhibition is calculated as percent inhibition using equation 2:

$$\% \text{ Inhibition} = [1 - (\text{cpm with inhibitor} / \text{ cpm without inhibitor})] \times 100.$$

### RESULTS

Figure 1 illustrates how copolymer 1 and terpolymers effect the proliferation of T cells which are specific for certain myelin basic protein (MBP) peptides. In general, T cells proliferate when exposed to the antigen to which they were sensitized. Thus, MBP and, in particular, certain antigenic peptides from MBP stimulate the proliferation of MBP-specific T cells.

Copolymer 1 and Terpolymers did not stimulate proliferation of T cells which were specific for the 84-102 peptide of MBP. Instead, they significantly inhibited the proliferation of T cells exposed to this MBP antigen.

TAL exhibited the most efficient inhibition of proliferation in T cells specific for the MBP 84-102 antigen. Inhibition by TAL is even greater than that provided by Copolymer 1. TGA induced lower inhibition of T cell proliferation. GAL and GTL inhibited T cell proliferation in a dose responsive manner similar to Copolymer 1.

## EXAMPLE 7

## TERPOLYMERS ARE RECOGNIZED BY SOME COPOLYMER 1-SPECIFIC T CELLS

Terpolymers can stimulate some Copolymer 1-specific T cell lines to proliferate and secrete the cytokine, IL-4.

## METHODS

### Copolymer 1

Copolymer 1 batches # 02095 and 55495, with average molecular weight of 6000 Da and 5800 Da, respectively, were obtained from Teva Pharmaceutical Industries (Petach Tikva, Israel).

**Myelin Basic Protein Peptides** which were synthesized on an Applied Biosystems Peptide Synthesizer using solid phase techniques. Barany et al., THE PEPTIDES 1 (1979). Peptides purified by reversed-phase HPLC. The peptides used were HA 306-318, having the sequence PKYVKQNTLKLAT (MW 1718) (SEQ ID NO: 2), and MBP 84 102, having the sequence DENPVVHFFKNIVTPRTPP (MW 2529) (SEQ ID NO: 1).

### Terpolymers

GAL, TGA, TAL, GTL and control polypeptides are as described above under Example 3.

### T cell lines and clones

**Mouse T cell lines** and clones were established according to Aharoni et al., 23 Eur. J. Immunol. 17 (1993); Aharoni et al., 94 PROC. NATL. ACAD. SCI. USA 10821 (1997). Copolymer 1 specific lines were originated from spleens of mice which, 15 to 35 days earlier, had been rendered unresponsive to EAE by subcutaneously injecting each mouse with 5-10 mg Copolymer 1, emulsified in incomplete Freund's adjuvant (Difco). Alternatively, Copolymer 1 specific lines were obtained from the lymph nodes of mice which had been immunized ten days earlier with 200 $\mu$g Copolymer 1 emulsified in complete Freund's adjuvant (Difco) supplemented with 4 mg/ml of Mycobacterium tuberculosis H37Ra (Difco).

Copolymer 1-specific T cell lines were used and T cell clones LN-1, LN-2, S-3, 5-22-5, C-14, and C-52. The LN-2 antibodies were derived from lymph nodes of (SJL/J x BALB/c) $F_1$ mice injected with Copolymer 1 in CFA. The 5-22-5 antibodies were obtained from spleens of (SJL/J x BALB/C) $F_1$ mice injected with Copolymer 1 in CFA, according to Aharoni et al., 23 EUR. J. IMMUNOL. 17 (1993); Aharoni et al., PROC. NATL. ACAD. SCI. USA 10821 (1997).

**Human T cell clones** were derived from peripheral blood mononuclear cells according Teitelbaum et al., 89 PROC. NATL. ACAD. SCI. USA 137 (1992). Approximately $5 \times 10^6$ cells were incubated in each well of a 24-well culture plate with Copolymer 1 or MBP (50 $\mu$g/ml) in culture medium supplemented with 10% heat-inactivated autologous serum. After 7 days of culture, the cells were transferred to culture medium containing 10% fetal calf serum and recombinant human interleukin 2 (20 units/ml). The cells were grown continuously in this medium with periodic exposure to antigen presented on irradiated (3000 rad), autologous mononuclear cells, every 14-18 days. The C-52 T-cell clone was derived from a DR7,w11 donor, also according to Teitelbaum et al., 89 PROC. NATL. ACAD. SCI. USA 137 (1992).

### Proliferation assay

Cells of T lines or clones were tested for their specific proliferative response 10-21 days after antigenic stimulation. T Cells ($1.5 \times 10^4$) were cultured in triplicates with $5 \times 10^5$ irradiated spleen cells or with human EBV-transformed B cells ($5 \times 10^4$), and with the indicated antigens in a final volume of 0.2 ml in 10% FCS culture medium. At the end of 48 hr incubation, cultures were pulsed with 1 $\mu$Ci[³H]-thymidine and then harvested 6-12 hr later. The variations of triplicates from their mean were under 20%.

cell line cross-reacted only with TAL. No cell lines cross reacted with GTL or with AGT (1:1:1) which has the same amino acids as TGA but in a different mole fraction than TGA or Copolymer 1.

**Table 6**

| Cross reactivity of Terpolymers with Copolymer 1 for Copolymer 1 specific T-cell lines and clones | | | | | | | | |
|---|---|---|---|---|---|---|---|---|
| **I. Murine T-cell lines and clones** | | | | | | | | |
| T-cells Polypeptide | Cross reactivity with Copolymer 1 (%) | | | | | | | |
| | LN -3 | | S-3 | | LN-1 | | S-22-5 | |
| | prol* | IL4‡ | prol* | IL-4‡ | Prol* | IL-4‡ | prol* | IL-4‡ |
| SD-1689 - GAL | 130 | **139** | **49** | 11 | 0 | 1 | 0 | 0 |
| SD-1690 - TGA | 3 | 6 | **102** | **107** | 0 | 1 | 0 | 0 |
| SD-1691 - TAL | 3 | 7 | 2 | 3 | **64** | **120** | 0 | 0 |
| SD-1697 - GTL | 2 | 4 | 12 | 6 | 0 | 1 | 0 | 0 |
| 1:1-1 AGT | 2 | 0 | 2 | 2 | 1 | 0 | 2 | 0T |
| *Prol = as measured by proliferation. ‡IL-4 = as measured by Interleukin-4. | | | | | | | | |
| **II. Human T-cell Clones** | | | | | | | | |

| T-cells Polypeptide | Cross reactivity with Copolymer 1 (%) | |
|---|---|---|
| | C-14 proliferation | C-52 proliferation |
| SD-1689 - GAL | 4 | **75** |
| SD-1690 - TGA | 1 | **58** |
| SD-1691 - TAL | 0 | 0 |
| SD-1697 - GTL | 0 | 5 |

## EXAMPLE 8

### TERPOLYMERS CROSS-REACT WITH ANTI-COPOLYMER 1 ANTIBODIES

Antibodies directed against Copolymer 1 cross-react with Terpolymers which lack either tyrosine, glutamic acid or alanine. However, Terpolymers lacking lysine are not recognized efficiently by anti-Copolymer 1 antibodies.

### Antibodies

Mouse anti-MBP and anti-Copolymer 1 monoclonal antibodies were obtained by fusion of MBP- or Copolymer 1-immunized spleen cells from SJUJ mice, with the NSO/1 murine plasmacytoma cell line. Teitelbaum et al., Proc. Natl. Acad. Sci. USA 9528 (1991).

### Radioimmunoassay

Flexible plastic microtiter plates were coated with Copolymer 1 or Terpolymers (2 $\mu$g/ml). After 16 hr incubation at room temperature, plates were washed three times and saturated for 2 hr with PBS containing 2% bovine serum albumin, 0.05% Tween 20, 0.1 % sodium azide, 10 mM EDTA, and heparin at 5 units/ml ("PBS buffer"). Monoclonal antibody supernatants (50 $\mu$l), were added to the wells for a 2 hr incubation, and the wells were washed again with PBS buffer. $^{125}$I-labeled goat anti-mouse Fab antibodies (1 x 10$^5$ cpm/well) were added for overnight incubation at 4°C. After extensive washing, radioactivity is measured in a gamma counter.

### Methods

### ELISA Assay for Antibody Cross-Reactivity

A standard ELISA assay is employed using anti-Copolymer 1 polyclonal antibodies and microtiter plates coated with 2 $\mu$g/ml of terpolymer preparation.

**Copolymer 1**

Copolymer 1 with the following amino acid composition is obtained from Teva Pharmaceutical Industries (Petach Tikva, Israel).

| Amino Acid | Molar Fraction |
|---|---|
| L-glutamic acid | 0.141 |
| L-alanine | 0.427 |
| L-tyrosine | 0.095 |
| L-lysine | 0.338 |

**Terpolymers**

The four Terpolymers of Example 1 were used.

**Results**

Table 7 indicated that anti-Copolymer 1 polyclonal antibodies cross react with Terpolymers which lack either tyrosine, glutamic acid or alanine. The relatively high percentage binding of the terpolymers lacking glutamic acid (TAL) might be explained by its high average molecular weight. Terpolymers which lack lysine are not efficiently recognized by anti-Copolymer 1 antibodies. These data suggest that charged amino acids like lysine may play a role in the recognition and binding of Copolymer 1 and Terpolymers.

**Table 7**

| Terpolymers | MW* | Percent Binding in separate experiments | | | | Mean | S.D. |
|---|---|---|---|---|---|---|---|
| | | 1 | 2 | 3 | 3 | | |
| TAL | 20,000 | 111.1 | 130.1 | 115.8 | 114.0 | 117.8 | 8.48 |
| GAL | 8,850 | 7.5 | 10.8 | 9.3 | 9.2 | 9.2 | 1.35 |
| GTL | 11,050 | 98.7 | 87.0 | n.d.‡ | n.d.‡ | 92.9 | 8.30 |
| TGA | 7,600 | 87.9 | 79.3 | n.d.‡ | n.d.‡ | 83.6 | 6.12 |
| * daltons. ‡n.d. - not determined. | | | | | | | |

**Cross reactivity of Terpolymers with Copolymer 1-reactive monoclonal antibodies**

The cross reactivity of the Terpolymers with Copolymer 1 at the level of B cell response is tested using monoclonal antibodies (mAbs), that are either reactive with both Copolymer 1 and MBP (mAbs 2-2-18 and 3-1-45), or are reactive with only Copolymer I (mAbs 3-3-9 and 5-7-2). See Teitelbaum et al., 88 Proc. Natl. Acad. Sci. USA 9528 (1991).
Table 8 illustrates that the Terpolymers differed in their ability to bind these mAbs. TGA and GTL were not recognized by any of the Copolymer 1 specific mAbs. On the other hand, TAL and GAL bound to Copolymer 1 and MBP specific mAbs with an affinity which is similar to that of Copolymer 1. GAL and TAL differed only in the binding to one mAb i.e. 5-7-2 which bound to TAL and not to GAL.

**Table 8**

| Cross reactivity of Terpolymers with MBP- and Copolymer 1-reactive B cell antibodies | | | | |
|---|---|---|---|---|
| Antibody Polypeptide | Cross reactivity with Copolymer 1 (%) | | | |
| | 2-2-18 | 3-1-45 | 3-3-9 | 5-7-2 |
| SD-1689 - GAL | **96** | **98** | **107** | 10 |
| SD-1690 - TGA | 3 | 2 | 1 | 10 |
| SD-1691 - TAL | **96** | **98** | **103** | **106** |

(continued)

| Cross reactivity of Terpolymers with MBP- and Copolymer 1-reactive B cell antibodies | | | | |
|---|---|---|---|---|
| | Cross reactivity with Copolymer 1 (%) | | | |
| Antibody Polypeptide | 2-2-18 | 3-1-45 | 3-3-9 | 5-7-2 |
| SD-1697 - GTL | 1 | 2 | 1 | 1 |
| 2-2-18 is anti mouse MBP monoclonal antibody **cross reactive** with Copolymer 1<br>3-1-45 is anti Copolymer 1 monoclonal antibody **cross reactive with** MBP<br>3-3-9 is anti Copolymer 1 monoclonal antibody **non cross reactive** with MBP<br>5-7-2 is anti Copolymer 1 monoclonal antibody **non cross reactive** with MBP | | | | |

## EXAMPLE 9

## COPOLYMER 1 AND TERPOLYMERS COMPETE WITH COLLAGEN FOR BINDING TO HUMAN LEUKOCYTE ANTIGENS AND INHIBIT COLLAGEN-SPECIFIC T-CELL RESPONSE

This example illustrates that Copolymer 1, TAL, GAL, GTL, and TGA compete for binding to MHC proteins with the rheumatoid arthritis-associated immunodominant collagen antigen, Cil 261-273 (SEQ ID NO: 3).

## Methods

## Protein Expression and Purification

Recombinant HLA-DR1 and HLA-DR4 molecules (encoded by DRA/DRB 1 *0101 and *0401, respectively) were expressed in *Drosophila* S2 cells as described in Stem, L. et al. 68 CELL 465 (1992) and Dessen, A. et al. 7 IMPUNITY 473 (1997). Cells were grown in roller bottles at 26°C in Excell 401 medium (Sigma, St. Louis, MO) supplemented with 0-5% fetal bovine serum (Sigma). Cells were induced by addition of $CuSO_4$ to I mM final concentration, then incubated an additional 4-5 days. Immunoaffinity purification of recombinant HLA-DR1 and HLA-DR4 is performed as previously reported by Stern, L. et al. 68 CELL 465 (1992) and Dessen, A. et al. 7 IMMUNITY 473 (1997). Supernatant from harvested cells is sequentially passed through Protein A, Protein G and Protein A-LB3.1 columns, followed by elution of the bound HLA-DR with 50 mM 3cyclohexylamino-1-propane sulfonic acid (CAPS), pH 11.5, and neutralized with 200 mm phosphate (pH 6.0). The eluate is concentrated on a Centriprep 10 membrane (Amicon). Protein concentrations were determined by bicinchoninic acid assay (Pierce Chemical Co.).

## Polypeptide labeling

The present polypeptides and the HA 306-318 peptide were biotinylated as in Examples 4 and 7.

## Class II MHC protein binding assay

In this assay, water-soluble recombinantly-produced proteins were incubated with biotinylated polypeptides of the present invention and varying quantities of unlabeled competitor polypeptides, collagen CII peptides or influenza virus HA peptides. Assays were performed in 96-well microliter immunoassay plates (PRO-BOND™, Falcon) which were coated with affinity-purified LB3.1 monoclonal antibodies. Antibody coating is performed by placing 100 $\mu$l of 10.0 $\mu$g/ml LB3.1 monoclonal antibodies in each well and incubating at 4°C for 18 hrs. Microtiter wells were then blocked with Tris buffered saline (TBS) containing 3% bovine serum albumin (BSA) for 1 hr at 37°C and washed three times with TTBS. Before sample addition, 50 $\mu$l of TBS containing 1% BSA is added to each well. Phosphate buffered saline (PBS) is 150 mM sodium chloride, 7.5 mM sodium phosphate dibasic, 2.5 mM sodium phosphate monobasic, pH 7.2. Tris buffered saline (TBS) is 137 mM sodium chloride, 25 mM TRIS pH 8.0, 2.7 mM potassium chloride. TTBS is TBS with 0.05% Tween-2G. Other solutions used in this assay are described in Fridkis-Hareli, M. et al., 160 J. IMMUNOL. 4386 (1998).

Binding analysis is performed by incubating the water soluble DR molecules with biotinylated polypeptides of the present invention and varying concentrations of unlabeled inhibitors (Copolymer 1, TAL, GAL, GTL, TGA, Collagen CII 281-273 peptide or HA 306-318 peptide). The collagen type CII peptide 261-273, has SEQ ID NO: 3 (AGFKGEQGPKGEP) and a molecular weight of 1516. The concentration of DR employed is 0.15 $\mu$M. The final concentration of biotinylated Copolymer 1 or terpolymers is 1.5 $\mu$M. Incubation is for 40 hr at 37°C in 50 $\mu$l binding buffer at pH 5.0.

Bound label is detected using streptavidin-conjugated alkaline phosphatase as follows. Plates were washed three times with TTBS and incubated with 100 μl of streptavidin-conjugated alkaline phosphatase (1:3000, BioRad, Richmond, VA) for 1 hr at 37°C, followed by addition of p-nitrophenyl phosphate in triethanolamine buffer (BioRad). The absorbency at 410 nm is monitored by a microplate reader (model MR4000, Dynatech, Chantilly, VA).

**T cell hybridoma and antigen presenting cell (APC) binding assays**

Copolymer 1 and Terpolymers were tested to ascertain if they could inhibit activation of T cells responsive to the collagen CII peptide. Mouse DR1-restricted 3.19 and 19.3 T cell hybridomas and mouse DR4-restricted 3838 and D3 T cell hybridomas were used. Rosioniec, E. F., et al., 185 J..EXP. MED. 1113-1122 (1997); Andersson, E. C., et al., PROC. NATL. ACAD. Scl. USA (1998). Antigen presenting cells (APCs) were L cells transfected with DR1 (the L57.23 cell line provided by Rosloniec, E. F., et al., 1997, 185 J. Exp. Med. 1113-1122 (1997)), L cells transfected with DR4, and Priess cells (DRB1*0401/DRB4*0101). T cell stimulation experiments were performed in 96-well microliter plates in a total volume of 0.2 ml. Irradiated (3000-rad) APC ($2.5 \times 10^4$/well) were coincubated with CII 26-273 (40 μg/ml) and varying concentrations of the present polypeptides for 2 hr at 37°C, then T cells ($5 \times 10^4$/well) were added and the incubation is continued for 24 hr at 37°C. Supernatants (30 μl) were removed and incubated with IL-2 dependent CTL-L ($5 \times 10^4$/well) for 12 hr, followed by labeling with $^3$H-thymidine (1 μCi/well) for 12 hr. Plates were harvested and the radioactivity is monitored using a 1450 microbeta Plus liquid scintillation counter (Wallac, Gaithersburg, MD).

**RESULTS**

**Class II MHC protein binding assay**

The recombinant water-soluble HLA-DR1 and -DR4 proteins produced in insect cells were largely free of bound autoantigens or other peptides. Hence, data obtained from insect cell produced proteins can more accurately indicate the actual binding affinities for polypeptides. Fridkis-Hareli et al., 160 J. IMMUNOL. 4386 (1998). Competitive binding of each of the Copolymer 1 and Terpolymers to HLA-DR1, HLA-DR2 and HLA-DR4 molecules is depicted in Figure 5. Binding of each of the Copolymer 1 (YEAK, top panel Fig. 5A) and Terpolymers is substantially greater than that of the CII 261-273 (SEQ ID NO: 3) peptide, as judged by quantity of CII 261-273 (SEQ ID NO: 3) peptide required for 50% inhibition. As also observed above, TAL bound HLA-DR1 and HLA-DR4 with greater affinity than did Copolymer 1. The kinetics of inhibition by unlabeled TAL (YAK, bottom panel Fig. 5A) polypeptides were also somewhat superior to that of the influenza virus peptide HA 306-318 (SEQ ID NO: 2). However, the influenza virus peptide HA 306-318 inhibited the binding of Copolymer 1 and of Terpolymers more efficiently than the CII 261-273 peptide.

**T cell Hybridoma and antigen presenting cell binding assay results**

Copolymer 1 and Terpolymers also inhibited DR1-restricted T cell activation by CII collagen peptide (Fig. 6). T cell activation was detected by observing IL-2 production by DR1-restricted-CII-specific T cell hybridomas. Collagen peptide CII 261-273 (SEQ ID NO: 3) at varying concentrations was coincubated with one of the present polypeptides and then T cells (clone 3.19 or 19.3 as indicated) were added, and the mixtures were further incubated. The supernatants from these incubated cells are removed, and were assayed for IL-2 by observing whether the supernatant induced proliferation of IL-2-dependent cytotoxic T lymphocytes (CTL-L). The extent of inhibition by TAL is shown as solid circles (●), by TGA as solid triangles (▲), by GTL as open triangles (∆), and by Copolymer 1 as solid squares (■). Percent inhibition of CTL-L proliferation shown on the ordinate was calculated using equation 1:

Again, TAL is the most potent inhibitor. However, GTL and Copolymer 1 were also potent inhibitors of T cell activation by the CII collagen peptide. TGA inhibited activation less efficiently. Similar results were obtained with other batches of Copolymer 1 and Terpolymers.

A similar inhibition of activation of DR4-restricted T cells was observed, as shown in Figure 7. IL-2 production was used to assess activation of DR4-restricted CII-specific T cell hybridomas (3838 and D3). The presence of different polypeptides inhibited IL-2 production, indicating that they inhibited DR4-restricted T cell activation. Fig. 7A shows the effects of coincubating irradiated 3838 or D3 Priess cells with collagen peptide CII 261-273 (SEQ ID NO: 3) at the fixed concentration of 40 μg/ml, and with varying concentrations of polypeptides, for 2 hr at 37°C. Fig. 7B shows the effects of incubating L cells transfected with a gene encoding HLA-DR4 with collagen peptide CII 261-273 (SEQ ID NO: 3) at the fixed concentration of 40 μg/ml, and with varying concentrations of GAL, TAL,GTL, TGA, and Copolymer 1, for 2 hr at 37°C T cells were then added (clones 3838 or D3 as indicated), samples were further incubated for 24 hr at 37°C. Supernatants (30 μl) were then removed, and were assayed for activation by IL-2-induced proliferation of IL-2-dependent cytotoxic T lymphocytes (CTL-L). Each polypeptide mixture was tested in duplicate. The concentration of the present polypeptides is indicated on the abscissa. The extent of inhibition by TAL is shown as solid circles (●), by TGA as solid triangles (▲), by GTL as open triangles(△), and by Copolymer 1 as solid squares (■). Percent inhibition of CTL-L proliferation shown on the ordinate was calculated using equation 1.

## EXAMPLE 10

## COPOLYMER 1 SEQUENCE MOTIFS WHICH BIND TO HLA-DR1, HLA-DR2 AND HLA-DR4 MOLECULES

### Methods

### Protein Expression and Purification

Recombinant HLA-DR1 and HLA-DR4 molecules (encoded by DRA/DRB I *0101 and *0401, respectively) were expressed in *Drosophila* S2 cells as described in Stem, L. et al. 68 CELL 465 (1992) and Dessen, A. et al. 7 IMMUNITY 473 (1997). Cells were grown in roller bottles at 26°C in Excell 401 medium (Sigma, St. Louis, MO) supplemented with 0-5% fetal bovine serum (Sigma). Cells were induced by addition of $CuSO_4$ to 1 mM final concentration, then incubated an additional 4-5 days. Immunoaffinity purification of recombinant HLA-DR1 and HLA-DR4 is performed as previously reported by Stern, L. et al. 68 CELL 465 (1992) and Dessen, A. et al. 7 IMMUNITY 473 (1997). Supernatant from harvested cells is sequentially passed through Protein A, Protein G and Protein A-LB3.1 columns, followed by elution of the bound HLA-DR with 50 mM 3cyclohexylamino-1-propane sulfonic acid (CAPS), pH 11.5, and neutralized with 200 mm phosphate (pH 6.0). The eluate is concentrated on a Centriprep 10 membrane (Amicon). Protein concentrations were determined by bicinchoninic acid assay (Pierce Chemical Co.).

### Binding and Quantifying Bound Polypeptide

Copolymer 1 is incubated with water-soluble HLA-DR1, -DR2 or -DR4 molecules at a molar ratio of 1:1 for 40 hr at 37°C. Unbound Copolymer 1 is separated from bound Copolymer 1 by Centricon ultrafiltration. Bound Copolymer 1 is then extracted from the HI-A-DR complex by acid treatment (Chicz, R. et al. 178 J. EXP. MED. 27 (1993)) and subjected to amino acid analysis.

For HPLC separation and microsequencing after elution, approximately 5-10% of the Copolymer 1 mixtures are fractionated by microbore, HPLC using a Zorbax $C_{18}$ 1.0 mm reverse-phase column on a Hewlett-Packard 1090 HPLC with 1040 diode array detector. At a flow rate of 54 μ/min, Copolymer 1 is eluted with a gradient of 0.055% trifluoroacetic acid (TFA) in acetonitrile (0% at 0-10 min, 33% at 73 min and 60% at 105 min). Strategies for peak elections, reverse phase separation and Edman microsequencing were performed as in Chicz et al. 178 J. EXP. MED. 27 (1993), and Lane, W. et al. 10 J. PROT. CHEM. 151(1991).

### Structural Characterization of Bound Copolymer 1

Fractionation of Copolymer 1 bound to human HLA-DR1, HLA-DR2 or HLA-DR4 molecules from homozygous EBV-transformed B cell lines, indicated that nearly all of the bound Copolymer 1 was found in the high average molecular weight fractions and less than 10% of bound Copolymer 1 was in the lower average molecular weight fractions.

To characterize the hydrophobicity and size of Copolymer 1 bound to isolated recombinant HLA molecules, bound and unbound Copolymer 1 samples are separated on reverse phase HPLC using an acetonitrile gradient. Control, untreated Copolymer 1 elutes as a very broad peak with several smaller peaks, which spread between approximately 40 and 75 min elution time. This elution profile is characteristic of a mixture of random polypeptides and resembles HPLC separations of other batches of Copolymer 1. Similar profiles were obtained when Copolymer 1 was eluted from HLA-DR1, HLA-DR2 or HLA-DR4 molecules, indicating that the bound fraction is similar to the original Copolymer 1 preparation in its chemical properties.

At least 95 % of the added Copolymer 1 molecules are observed in the fraction bound to isolated HLA-DR1 proteins; similarly, at least 95 % of the added Copolymer 1 molecules are observed in the fraction bound to isolated HLA-DR4 proteins. However, 80% of the added Copolymer 1 molecules are bound to HLA-DR2 proteins. Copolymer 1 eluted from the HLA complexes with had a ratio of amino acids tyrosine: glutamic acid: alanine: lysine which was similar to that of control untreated Copolymer 1. These results indicate that the bound fraction of Copolymer 1 had an amino acid composition like that of the bound/unbound mixture and that the population of Copolymer 1 molecules exhibited little or no preferential binding to different HLA-DR proteins.

To analyze the sequence of Copolymer 1 that bound to each of HLA-DR1, -DR2 and -DR4 molecules, HPLC fractions obtained above were pooled submitted to automated Edman degradation on a Hewlett-Packard G1005A (Palo Alto, CA) protein sequencer using the manufacturer's Routine 3.5.

For each HLA-DR protein, the bound copolymer had a random distribution of the four amino acid which corresponded to the input molar ratios of amino acids used for making Copolymer 1. Alanine was found at significantly higher levels compared to glutamic acid, tyrosine and lysine, as is expected from the initially higher molar ratio of alanine in Copolymer 1. No sequence specificity or preferential positioning of any of the amino acids of Copolymer 1 was observed, indicating that the bound fraction was also random and similar to the unfractionated Copolymer 1 preparation.

**Characterization of Bound and Unbound Copolymer 1 Epitopes**

Anti-Copolymer 1 polyclonal antibodies were used to determine whether the Copolymer 1 fractions eluted from each of the HLA-DR molecules contained the epitopes found in control untreated Copolymer 1. Cross reactivity between Copolymer 1 and various eluted fractions was detected by direct ELISA assay using biotinylated anti-Copolymer 1 polygonal antibodies. Copolymer 1 or Copolymer 1 fractions were diluted to 0.4 $\mu$g/ml and 2.0 $\mu$g/ml and 100 $\mu$g/well was plated in duplicate on a 96-well microtiter immunoassay plate (PRO-BIND™, Falcon, Lincoln Park, N.J), incubated for 1 hr at 37°C and washed three times with TBS containing 0.05% Tween-20. The wells were then blocked with TBS containing 3% BSA, followed by addition of biotinylated anti-Copolymer 1 antibodies (at a dilution of 1:5000, 100 $\mu$l/well). Antibody-ligand complexes were detected using streptavidin-conjugated alkaline phosphatase (at a dilution of 1:3000, BioRad ) and p-nitrophenyl phosphate in triethanolamine buffer (BioRad; Hercules, CA). The absorption at 410 nm was monitored by a microplate reader (Dynatech MR4000).

The antibody binding assays showed that all the fractions were similarly recognized by anti-copolymer antibodies, suggesting that these bound heteropolymer fractions shared similar or identical epitopes with each other and with control Copolymer 1.

**Characterization of Copolymer 1-HLA binding motifs**

When bound, the MHC class II protein protects the Copolymer 1 binding site from peptidase digestion. To obtain the amino acid sequence(s) of the binding site(s) within Copolymer 1 for HLA-DR molecules, 1 mM Copolymer 1 was separately incubated with 100 $\mu$m of each type of HLA-DR molecule in a volume of 10 $\mu$l PBS for 40 hours at 37°C, so that the molar ratio of Copolymer 1 to HLA-DR was 10:1. Two units of aminopeptidase I was added to the reaction for the last 18 hr of incubation. Aminopeptidase I is a metalloprotein isolated from *Streptomyces griseus* (Spungin et al. 283 J. BIOCHEM 471 (1989)) and is available from Sigma Chemicals, St. Louis, MO. Aminopeptidase 1 removes the amino-terminal ends of Copolymer 1 which protrude from the HLA-DR molecules, and digests the remaining unbound Copolymer 1 (Mouritsen et al. 148 J. IMMUNOL. 1987 (1992); Larsen et al. 184 J. EXP, MED. 183 (1996)). Subsequent digestions of Copolymer 1 with aminopeptidase are performed in volumes scaled up by a factor of twenty-forty fold, for example, 300 $\mu$l of heteropolymer digested with 60 $\mu$l of aminopeptidase. Samples are spin-concentrated to a final volume of approximately 100 $\mu$l using Centricon 10 ultrafiltration devices.

The Copolymer 1-HLA-DR complexes and the unbound Copolymer 1 were analyzed by SDS-PAGE, using a NOVEX mini-cell electrophoresis system, a 10% acrylamide separating gel and a 5% acrylamide stacking gel. The Copolymer 1-HLA-DR1 complexes were run under non-reducing conditions for 1 hr at 200 V, stained with Coomassie Brilliant Blue, fixed for 3 hr in 10% methanol/10% acetic acid and dried on Cellophane paper (BioRad) at 25°C. The Copolymer 1-HLA-DR complexes were found to be resistant to SDS-mediated dissociation, forming higher molecular weight complexes with HLA-DR1 $\alpha\beta$ heterodimers, and were observed as numerous bands on the polyacrylamide gel with molecular weights greater than 50 kD, indicating that the Copolymer 1-HLA-DR complexes were protected. Aminopeptidase I treatment resulted in unbound Copolymer 1 appearing as a smear in the lower part of the gel, indicating that it was completely digested by the enzyme.

Fractions containing the peaks of Copolymer 1-HLA-DR complexes were selected in the region between approximately 40 and 75 min elution time for each class of HLA-DR complex to obtain the sequence of the binding motifs. Bound, digested Copolymer 1 polypeptides, which lack the protruding N-termini, are eluted from HLA-DR by addition of acetic acid (10%) and incubation at 70°C for 15 min, followed by ultrafiltration and vacuum concentration in a SpeedVac (Savant Instruments, Farmingdale, NY). Fridkis-Hareli et al. 163 CELL. IMMUNOL. 229 (1995).

The N-terminal 20 to 25 amino acids of Copolymer 1 protrude beyond the HLA-DR proteins, and are not part of the binding motifs of Copolymer 1 for these proteins. The sequence data (Table 9) show that Copolymer 1 peptides bound to HLA-DR1 have significantly higher levels of glutamine at the first and second positions of the Copolymer 1 binding domain, higher levels of lysine at the second and third positions, and higher levels of tyrosine at the third to fifth positions. The third position of the bound Copolymer 1 peptide (lysine or tyrosine) may correspond to the P1 position of the peptide binding site within the MHC class II groove because the P1 site is the third amino acid in the binding sites of other peptides which bind HLA-DR1. For example, tyrosine is found in the third (P1) position of the HA 306-318 peptide, which corresponds to the Y308 position of the HA protein. Stern et al., 368 NATURE (Lond.) 215. These data are in contrast to the random patterns of the sequences found in untreated Copolymer 1, which shows no sequence specificity or preferential positioning within the MHC class II groove for any of the four amino acids within Copolymer 1.

For HLA-DR2, both tyrosine and alanine are detected after the third cycle of Edman degradation (Table 9). No sequence specificity or preferential positioning is observed for positions corresponding to anchor positions following P1 (at positions corresponding to the P4, P6 or P9 positions of HLA-DR1 and HLA-DR4; and at positions corresponding to the P4 or P7 positions of HLA-DR2b molecules). In all the samples the levels of alanine are higher than those of glutamine, tyrosine and lysine, which was expected because a higher molar ratio of alanine is present in Copolymer 1. For each of the HLA-DR1 and HLA-DR4 molecules, tyrosine was found at the position corresponding to the first anchor position (the third residue in the sequence analysis), followed by alanine in the positions corresponding to the subsequent pockets. In the Copolymer 1 bound to HLA-DR-2, tyrosine was also enriched at the position corresponding to P1. Glutamic acid was enriched at the first cycle position corresponding to the P-2 position and at the next adjacent position corresponding to P-1, lysine was enriched. These residues can contribute to the stable interactions of Copolymer 1 with the HLA-DR molecules and the interaction of this complex with the T cell receptor (TCR).

These results indicate that Copolymer 1 contains class II MHC binding motifs. It may be that Copolymer 1 bound to the antigen groove of HLA-DR molecules can act either as a blocking peptide or as an antagonist or partial agonist, resulting in suppression of autoimmune T cell responses or anergy, or both. Applicants' invention does not depend upon the theory underlying the action of Copolymer 1 or terpolymers.

**Table 9**

**Binding sequences of Copolymer 1 Peptides Bound to HLA-DR1, HLA-DR2 and HLA-DR4 molecules**

| HLA-DR | relative amino acid positions | | | | | | |
| --- | --- | --- | --- | --- | --- | --- | --- |
| | -2 | -1 | 1 | 4 | 6 | 7 | 9 |
| DRIBI*0101 DR-1 | E | K | Y | A | A | A | A |
| DRBI*0401 DR-4 | E | K | Y | A | A | A | A |
| DRBI*1501 DR-2 | E | K | Y,A | A | A | A | A |

## EXAMPLE 11

### DEVELOPMENT OF PEPTIDES FOR BINDING HLA-DR1 AND HLA-DR4 MOLECULES

The previous examples indicate that Copolymer 1 and Terpolymers bind to purified human HLA-DR molecules within the peptide binding groove and inhibit binding of HA 306-318, a peptide from influenza virus which binds with high affinity to both HLA-DR1 (DRB 1*0101) and HLA-DR4 (DRB 1*0401) molecules. Further, terpolymers composed of only three amino acids (GAL, TGA, TAL and GTL) bound to purified HLA-DR1, HLA-DR2 and HLA-DR4 molecules and competed with the collagen peptide, CII 261-273, for binding to RA-associated HLA-DR1 (DR-B I *0101) and -DR4 (DRB 1*0401) protein molecules. The terpolymers also inhibited CII-reactive T cell clones.

In this example, peptides of defined sequence and length (15 residues) were synthesized using the sequences of the binding motifs summarized in Table 9. These peptides were analyzed for affinity and specificity of binding to MHC class II HLA-DR protein molecules, for an ability to inhibit binding of competitor molecules and for an ability to inhibit T cell responses. According to the present invention, these properties are useful in therapeutic compositions for treating immune diseases.

Peptides shown in Table 10 were synthesized using solid phase techniques (Barany, G. et al., 1979. The Peptides, E. Gross et al., eds. (New York, NY: Academic Press) on an Applied Biosystems Peptide Synthesizer, and were purified by reversed-phase HPLC. Peptide sequences included HA 306-318, having the sequence PKYVKQNTLKLAT (SEQ ID NO: 2)(MW 1718); CII 261-273, having the sequence AGFKGEQGPKGEP (SEQ ID NO: 3)(MW 1516); and HA 306-318 bracketed by alanines at the N- and C-terminals, APKYVKQNTLKLATA (SEQ ID NO: 44). Peptides were also synthesized on a 1 μmole scale using the Multipin Peptide Synthesis System (Chiron Technologies, Raleigh, NC). Peptides were synthesized as 15-mers with free amino groups at the N-terminus and free carboxyl groups at the C-terminus, and with biotin linked to the N-terminus by the spacer SGSG and having a free carboxyl group at the C-terminus. Peptide synthesis was monitored by including two standard peptide sequences as controls, which were subjected to HPLC and mass spectroscopy analysis. The HA 306-318 peptide was also used as a positive control for binding experiments. Pin peptides were lyophilized and resuspended at a concentration of 2 mg/ml in dimethyl sulfoxide (DMSO). Under these conditions, the majority of peptides were completely solubilized. Biotinylation was performed with excess N-hydroxysuccinimide biotin (Sigma, St. Louis, MO) in DMSO as described in Fridkis-Hareli et al., 91 PROC. NAD. ACAD. Scl., USA 4872-4876 (1994). Unreacted biotin was removed by dialysis (SpectraPor® membrane MWCO 500, Spectrum Medical Industries, Houston, TX).

Peptides were made to have test sequence motifs for binding to the groove of HLA-DR1 and -DR4 molecules (see Table 10). The peptides generally contained various combinations of glutamic acid, lysine and alanine at the N-terminus, followed by tyrosine at the position corresponding to P1 (shown in bold in Table 10), and then alanine in the subsequent binding pockets. The test sequence motifs fall into three different groups according to these positions in the consensus (Table 10). Peptides in group I had lysine (K) at the position corresponding to P8 and tyrosine (Y) at the position corresponding to PI (in bold in Table 10). A reference peptide in this set with lysine (K) at the position corresponding to P8 to increase solubility and alanine at all other residues (SEQ ID NO: 45) has previously been synthesized. Jardetzky et al., 9 EMBO J. 1797-1803 (1990). Peptides in group II had tyrosine (Y) at the position corresponding to PI, but had alanine (A) at the position corresponding to P8. Peptides in group III had amino acid tyrosine (Y) shifted one or two residues with respect to the P1 position in HA 306-318 peptide. Peptides in all groups contained one or more glutamic acid (E) and/or lysine (K) residues, in variable positions as was observed in the binding motifs supra, and to enhance solubility. Both N-terminal biotinylated and unlabeled sets of peptides were synthesized for these studies.

**Table 10**
**Groups of synthetic peptides and consensus positions**

| Group | SEQ ID NO | peptide sequence | amino acid consensus positions |
|---|---|---|---|
| Control | 44 | APKYVKQNTLKLATA | A(HA 306-318)A |
| I | 45 | AAAYAAAAAAKAAAA | P1Y, P8K |
| | 46 | AKKYAAAAAAKAAAA | |
| | 47 | AEAYAAAAAAKAAAA | |
| | 25 | AKEYAAAAAAKAAAA | |
| | 26 | AAEYAAAAAAKAAAA | |
| | 27 | AAKYAEAAAAKAAAA | |
| | 28 | EAKYAAAAAAKAAAA | |
| | 29 | AEKYAAAAAAKAAAA | |
| | 30 | KEAYAAAAAAKAAAA | |
| | 31 | AEEYAAAAAAKAAAA | |
| | 32 | EKAYAAAAAAKAAAA | |
| | 33 | AAKYEAAAAAKAAAA | |
| | 34 | EAAYAAAAAAKAAAA | |
| | 35 | EKAYAAAAAAKAAAA | |
| II | 48 | AEKYAAAAAAAAAAA | P1Y, P8A |
| | 49 | AKEYAAAAAAAAAAA | |
| | 50 | AKKYAEAAAAAAAAA | |
| | 51 | AEAYKAAAAAAAAAA | |
| | 52 | KEAYAAAAAAAAAAA | |
| | 53 | AEEYKAAAAAAAAAA | |
| | 54 | EKAYAAAAAAAAAAA | |
| | 55 | EKKYAAAAAAAAAAA | |
| | 56 | EAKYAAAAAAAAAAA | |
| | 36 | AKKYEAAAAAAAAAA | |
| | 37 | AAEYKAAAAAAAAAA | |
| | 38 | AAKYEAAAAAAAAAA | |
| | 39 | AAKYAEAAAAAAAAA | |
| III | 40 | AEYAKAAAAAAAAAA | P1A, P8A |
| | 41 | AEKAYAAAAAAAAAA | |
| | 57 | EKYAAAAAAAAAAAA | |
| | 42 | AYKAEAAAAAAAAAA | |
| | 43 | AKYAEAAAAAAAAAA | |

Competitive binding assays are used to examine whether the synthetic peptides can compete successfully with Copolymer 1 or with the high affinity HA 306-318 peptide for binding to HLA-DR1 and HLA-DR4 proteins. In the competitive binding assays, either Copolymer 1 or the HA 306-318 peptide bracketed by alanines (APKYVKQNTLKLATA, SEQ ID NO: 44) is biotinylated and unlabeled Copolymer 1 or peptide inhibitors are added. Kinetic studies indicated that biotinylated Copolymer 1 inhibited binding of unlabeled Copolymer 1 and of HA 306-318 (SEQ ID NO: 2) to recombinant HLA-DR1 better than peptides of groups I-III. However, several peptides containing lysine at the P8 position (group I) are better inhibitors than peptides of similar sequence but with an alanine at the P8 position (groups II and III of Table 10). In contrast, the binding of biotinylated Copolymer 1 to HLA-DR4 molecules was efficiently inhibited by many of the peptides in groups I-III. Conversely, the binding of biotinylated HA 306-318 to HLA-DR4 was inhibited more by Copolymer 1 than by HA 306-318 or by the peptides of groups I-III.

To further characterize the relative affinity of the Group I-III peptides for binding to HLA-DR1, -DR2 and -DR4 molecules, competitive binding assays are performed with biotinylated Multipin peptides using unlabeled Copolymer 1, HA 306-318 or CII 261-273 as inhibitors. The binding of the majority of Group I-III peptides to HLA-DR1 and HLA-DR4 was inhibited by unlabeled Copolymer 1, HA 306-318 (SEQ ID NO: 2) and CII 261-273 (SEQ ID NO: 3), however, less efficiently than the binding of HA 306-318 (SEQ ID NO: 2). Some of the peptides showed higher affinity for the HLA-DR1 (Table 11A) or HLA-DR4 (Table 11 B) proteins than did Copolymer 1, HA 306-318, or CII 261-273.

**Table 11A**

| | **Affinity of Group I-III peptides for HLA-DR1 molecules** | | |
|---|---|---|---|
| SEQ ID NO | peptide sequence | HA 306-318 | Inhibitors Copolymer 1 |
| 44 | APKYVKQNTLKLATA | 13.0 | 3.3 |
| 25 | AKEYAAAAAAKAAAA | 19.0 | |
| 26 | AAEYAAAAAAKAAAA | 47.0 | |
| 27 | AAKYAEAAAAKAAAA | 42.0 | 16.0 |
| 28 | EAKYAAAAAAKAAAA | 33.0 | |
| | Copolymer 1 | 10.0 | 8.0 |

**Table 11B**

| | | **Affinity of Selected Copeptides for HLA-DR4 (DRB1*0401)** | | |
|---|---|---|---|---|
| Group | SEQ ID NO | peptide sequence | HA 306-318 ($\mu$M) | Copolymer 1 ($\mu$M) |
| Control | 44 | APKYVKQNTLKLATA | 26.0 | 8.2 |
| | 45 | AAAYAAAAAAKAAAA | 7.0 | |
| | 25 | AKEYAAAAAAKAAAA | 4.5 | |
| | 26 | AAEYAAAAAAKAAAA | 3.2 | 1.6 |
| | 27 | AAKYAEAAAAKAAAA | 1.8 | <1.0 |
| | 28 | EAKYAAAAAAKAAAA | 4.4 | 3.0 |
| | 29 | AEKYAAAAAAKAAAA | 6.5 | |
| | 30 | KEAYAAAAAAKAAAA | 4.5 | |
| | 31 | AEEYAAAAAAKAAAA | 2.0 | |
| | 32 | EKAYAAAAAAKAAAA | 3.3 | |
| | 33 | AAKYEAAAAAKAAAA | 4.0 | |
| | 34 | EAAYAAAAAAKAAAA | 5.0 | |
| | 35 | EKKYAAAAAAKAAAA | 1.8 | |
| II | 36 | AKKYEAAAAAAAAAA | 2.2 | |
| | 37 | AAEYKAAAAAAAAAA | 1.8 | |
| | 38 | AAKYEAAAAAAAAAA | 1.2 | |
| | 39 | AAKYAEAAAAAAAAA | 1.2 | |
| III | 40 | AEYAKAAAAAAAAAA | 3.0 | |
| | 41 | AEKAYAAAAAAAAAA | <1.0 | |
| | 42 | AYKAEAAAAAAAAAA | 1.3 | |
| | 43 | AKYAEAAAAAAAAAA | 3.0 | |

(continued)

| Affinity of Selected Copeptides for HLA-DR4 (DRB1*0401) | | | | |
|---|---|---|---|---|
| Group | SEQ ID NO | peptide sequence | HA 306-318 ($\mu$M) | Copolymer 1 ($\mu$M) |
| | | Copolymer 1 | 2.5 | 20.0 |

Affinities were determined by competition with biotinylated competitors HA306-318 and Copolymer 1.

All peptides were further tested for inhibition of CII-spscific T cell responses using methods described in previous examples. HA 306-318 (peptide SEQ ID NO: 2) inhibited both DR1 3.19 and 19.3 T cell clones very efficiently (over 95% and 98% for 19.3 and 3.19 cells, respectively). However, peptides with SEQ ID NO: 27, 48, 36 and 53 were as good or better inhibitors of T cell response than the reference influenza virus hemagglutinin peptide HA 306-318.

For HLA-DR4-restricted T cells, using L fibroblasts transfected with HLA-DR4 as the antigen presenting cell, the following pattern of activity was obtained: peptides SEQ ID NOS: 29, 31, 34, 35, 50, 51, 54, 38 and 41 were good inhibitors of the DR4 3838 T cell clone, whereas the D3 clone was inhibited best by peptides SEQ ID NOS: 46, 27, 34, 28 and 54. These peptides produced levels of inhibition of over 80% for the D3 and 3838 cells. Copolymer 1 had only a minimal effect on the CII-specific T cell response, consistently giving less than 20% inhibition. HA 306-318 (SEQ ID NO: 2) inhibited both DR4 3838 and D3 T cell clones less efficiently (less than 60% inhibition) than it inhibited the DR1, 3.19 and 19.3 clones.

These data show that peptides of SEQ ID NO: 46, 27, 34, 28 and 41 are significantly better inhibitors of T cell response than the reference influenza virus hemagglutinin peptide HA 306-318. Peptides of SEQ ID NO: 27 and 41 are high level inhibitors both of HLA-DR-1- and -DR-4-restricted CII-specific T cells.

The data in these examples, performed with each peptide at least in duplicate, show that of 33 unique synthetic peptides, several inhibited binding of HA 306-318 and Copolymer 1 to recombinant HLA-DR1 and -DR4 molecules. Peptides which inhibited binding of HA 306-318 or copolymer1 to HLA-DR1 or -DR4 molecules contained tyrosine at the PI position. The presence of glutamic acid, alanine and lysine in various combinations on the N-terminal side of PI did not seem to influence the affinity of the binding. Of the subsequent residues, lysine at P8 was important for inhibition of HA 306-318 but not of Copolymer 1 binding to HLA-DR1. In contrast to HLA-DR1, a larger number of peptides inhibited binding of both HA 306-318 and Copolymer 1 to HLA-DR4 molecules. These peptides contained tyrosine at the position corresponding to P1 and either lysine or alanine at the position corresponding to P8, with no apparent preference for specific amino acids at other positions.

The affinity of the HA 306-318 for recombinant HLA-DR4 was lower, and that of Copolymer 1 higher, than for HLA-DR1 molecules, These affinities are similar to those observed with HLA-DR1 and -DR4 molecules purified from human blood. The binding of some of the biotinylated peptides to either HLA-DR1 or -DR4 was inhibited by CII 261-273, as well as by HA 306-318 and Copolymer 1, showing that these peptides may compete for presentation to CII-reactive T cells, in a similar fashion to that for the whole Copolymer 1 mixture. Peptides with an affinity close to or higher than that of CII 261-273, HA 306-318 and Copolymer 1 preparations are listed in Table 11A, for HLA-DR1 and Table 11B, for HLA-DR4.

Several of the 15-mer peptides inhibited type II collagen-specific T cell clones. These peptides all had tyrosine at the position corresponding to PI and either lysine or alanine at the position corresponding to P8, with no other specific sequence patterns. The examples provided here show the strong inhibition by several random heteropolymers composed of three amino acids selected from the group consisting of tyrosine, glutamic acid, alanine and lysine. These heteropolymers, especially TAL, competed with CII 261-273 for binding to RA-associated HLA-DR1 and -DR4 molecules, and inhibited CII-reactive T cell clones. Further, peptide SEQ ID NO: 46, which includes the direct sequence tyrosine-alanine-lysine inhibited type II collagen-reactive T cells better than Copolymer 1, indicating that a peptide of approximately 15 amino acids in length having single tyrosine-alanine-lysine sequence can substitute for the mixture of random polypeptides found in the TAL heteropolymer.

The results of the foregoing examples indicate that the embodiments of the invention, with individual amino acid sequences that include at least three amino acids selected from tyrosine, glutamic acid, alanine and lysine, have binding motifs for anchor positions fitting particular HLA-DR molecules and can act as effective therapeutic agents for autoimmune diseases. A pharmaceutical composition comprising a pure synthetic short polypeptide of single sequence may have fewer side effects and greater specificity for a particular binding site or autoimmune disease than a mixture of polypeptides of random sequence. On the other hand, the polypeptide mixtures contemplated by the present invention may have broader efficacy for many types of autoimmune diseases. Hence, both the short polypeptides of a single sequence, and the mixtures of polypeptides easily provided by the present invention, are valuable therapeutic agents.

## EXAMPLE 12

### COPOLYMER 1 INHIBITS ACTIVATION OF T CELLS RESPONSIVE TO A MYASTHENIA GRAVIS ANTIGENIC PEPTIDE

### METHODS

### Copolymer 1

Copolymer 1 was obtained from Teva Pharmaceutical Industries (Petach Tikva, Israel).

**Myasthenia Gravis-Related Peptides** were synthesized on an Applied Biosystems Peptide Synthesizer using solid phase techniques. Barany et al., THE PEPTIDES 1 (1979). Peptides were purified by reversed-phase HPLC. The peptides used were the p259 peptide, see Zisman et al., Hum Immuol 1995 Nov; 44(3):121-30; Brocke et al., Immunology 1990 Apr; 69(4):495-500

### IL-2 secretion

Secretion of IL-2 by the cell line WCB AB. in response to the myasthenia gravis peptides and/or Copolymer 1 were evaluated. Cells ($1.5 \times 10^4$) were incubated with the indicated antigen. Secretion of IL-2 by the cell line WCB AB was used as a measure of activation of that T-cell line.

### RESULTS

Table 12 indicates that the p259 peptide stimulates T cell secretion. However, when Copolymer 1 is incubated with the p259 peptide, T cell secretion of IL-2 is inhibited in a dose-related fashion. At 100 $\mu$M Copolymer 1 inhibits about 91% of IL-2 secretion (Table 13), indicating that Copolymer 1 is a potent inhibitor of T cell activation.

**Table 12**

| IL-2 secretion from WCB AB line in response to p259 | | | | |
|---|---|---|---|---|
| p 259 conc. ($\mu$M) | Avg OD (450 nm) | SD | %CV | IL-2 (pg/ml) |
| 0 | 0.077 | 0.01 | | 0 |
| 0.25 | 0.135 | 0.00 | | 24 |
| 0.5 | 0.227 | 0.01 | 3.9 | 72 |
| 1 | 0.387 | 0.01 | 2.9 | 159 |
| 2 | 0.725 | 0.02 | 2.4 | 347 |

**Table 13**

| IL-2 secretion from WCB AB line in response to Cop1 alone or 2$\mu$M p259 + Cop1 | | | | | |
|---|---|---|---|---|---|
| Cop1 ($\mu$M) | Avg OD (450 nm) | SD | %CV | IL-2 (pg/ml) | % Inhibition |
| 0 | 0.725 | 0.02 | 2.4 | 347 | |
| 1 | 0.086 | 0.01 | | 0 | |
| 2 | 0.079 | 0.00 | | 0 | |
| 10 | 0.086 | 0.00 | | 0 | |
| 20 | 0.266 | 0.01 | 8.0 | 93 | 73 |
| 60 | 0.166 | 0.01 | | 40 | 88 |
| 100 | 0.151 | 0.01 | 11.2 | 32 | 91 |
| Absorbance is the average of 3 samples. Confidence values were calculated relative to the absorbance of a blank (%CV = SD/(Avg-blank)*100). | | | | | |

**Claims**

1. Use of a mixture of polypeptides having an average molecular weight of 2,000 to 40,000 daltons, wherein each polypeptide consists of glutamic acid, tyrosine, alanine and lysine, in an amount effective to treat a human subject afflicted with Crohn's disease for the preparation of a pharmaceutical composition to be administered to a subject afflicted with Crohn's disease for the treatment of Crohn's disease.

2. The use of claim 1, wherein in the mixture of polypeptides, the glutamic acid is present in a mole fraction of 0.14; the tyrosine is present in a mole fraction of 0.10; said alanine is present in a mole fraction of 0.43; and lysine is present in a mole fraction of 0.34.

3. The use of claim 1 or 2, wherein the mixture of polypeptides has an average molecular weight of 4,000 to 12,000 daltons.

4. The use of claim 3, wherein the mixture of polypeptides has an average molecular weight of 5,000 to 9,000 daltons.

5. The use of claim 1, wherein in the mixture of polypeptides the glutamic acid is present in a mole fraction of 0.14; the tyrosine is present in a mole fraction of 0.10; said alanine is present in a mole fraction of 0.43; and lysine is present in a mole fraction of 0.34, and wherein the mixture of polypeptides has an average molecular weight of 5,000 to 9,000 daltons.

6. The use of any one of claims 1-5, wherein the pharmaceutical composition is administered orally, topically, by inhalation or by injection.

7. The use of any one of claims 1-6, wherein the mixture of polypeptides is Copolymer 1.

8. Copolymer 1 for use in treating Crohn's disease.

**Patentansprüche**

1. Verwendung eines Polypeptidgemischs, das ein Durchschnittsmolekulargewicht von 2000 bis 40000 Dalton hat, wobei jedes Polypeptid aus Glutaminsäure, Tyrosin, Alanin und Lysin besteht, in einer Menge, die wirksam ist, ein menschliches Individuum zu behandeln, das unter Morbus Crohn leidet, zur Herstellung einer pharmazeutischen Zusammensetzung, die einem Individuum, das unter Morbus Crohn leidet, für die Behandlung von Morbus Crohn zu verabreichen ist.

**2.** Verwendung nach Anspruch 1, wobei in dem Polypeptidgemisch die Glutaminsäure in einem Stoffmengenanteil von 0,14 vorliegt; das Tyrosin in einem Stoffmengenanteil von 0,10 vorliegt; das Alanin in einem Stoffmengenanteil von 0,43 vorliegt; und Lysin in einem Stoffmengenanteil von 0,34 präsent ist.

**3.** Verwendung nach Anspruch 1 oder 2, wobei das Polypeptidgemisch ein Durchschnittsmolekulargewicht von 4000 bis 12000 Dalton hat.

**4.** Verwendung nach Anspruch 3, wobei das Polypeptidgemisch ein Durchschnittsmolekulargewicht von 5000 bis 9000 Dalton hat.

**5.** Verwendung nach Anspruch 1, wobei in dem Polypeptidgemisch die Glutaminsäure in einem Stoffmengenanteil von 0,14 vorliegt; das Tyrosin in einem Stoffmengenanteil von 0,10 vorliegt; das Alanin in einem Stoffmengenanteil von 0,43 vorliegt; und Lysin in einem Stoffmengenanteil von 0,34 vorliegt und wobei das Polypeptidgemisch ein Durchschnittsmolekulargewicht von 5000 bis 9000 Dalton hat.

**6.** Verwendung nach einem der Ansprüche 1-5, wobei die pharmazeutische Zusammensetzung oral, topisch, durch Inhalation oder durch Injektion verabreicht wird.

**7.** Verwendung nach einem der Ansprüche 1-6, wobei das Polypeptidgemisch Copolymer 1 ist.

**8.** Copolymer 1 zur Verwendung in der Behandlung von Morbus Crohn.

## Revendications

**1.** Utilisation d'un mélange de polypeptides ayant un poids moléculaire moyen de 2000 à 40000 daltons, où chaque polypeptide consiste en de l'acide glutamique, de la tyrosine, de l'alanine et de la lysine, dans une quantité efficace pour traiter un sujet humain affecté de la maladie de Crohn pour la préparation d'une composition pharmaceutique destinée à être administrée à un sujet affecté de maladie de Crohn pour le traitement de la maladie de Crohn.

**2.** Utilisation selon la revendication 1, où dans le mélange de polypeptides, l'acide glutamique est présent dans une fraction molaire de 0,14; la tyrosine est présente dans une fraction molaire de 0,10; ladite alanine est présente dans une fraction molaire de 0,43; et la lysine est présente dans une fraction molaire de 0,34.

**3.** Utilisation selon la revendication 1 ou 2, où le mélange de polypeptides a un poids moléculaire moyen de 4000 à 12000 daltons.

**4.** Utilisation selon la revendication 3, où le mélange de polypeptides a un poids moléculaire moyen de 5000 à 9000 daltons.

**5.** Utilisation selon la revendication 1, où dans le mélange de polypeptides l'acide glutamique est présent dans une fraction molaire de 0,14; la tyrosine est présente dans une fraction molaire de 0,10; ladite alanine est présente dans une fraction molaire de 0,43; et la lysine est présente dans une fraction molaire de 0,34, et où le mélange de polypeptides a un poids moléculaire moyen de 5000 à 9000 daltons.

**6.** Utilisation selon l'une quelconque des revendications 1 à 5, où la composition pharmaceutique est administrée par voie orale, par voie topique, par inhalation ou par injection.

**7.** Utilisation selon l'une quelconque des revendications 1 à 6, où le mélange de polypeptides est le Copolymère 1.

**8.** Copolymère 1 pour une utilisation dans le traitement de la maladie de Crohn.

Figure 1

Figure 2A

Figure 2A cont.

Figure 2A cont.

Figure 2B

Figure 2B cont.

Figure 2B cont.

Figure 3

Figure 3 cont.

Figure 3 cont.

Figure 4A

Figure 4A cont.

Figure 4A cont.

Figure 4B

Figure 4B cont.

Figure 4B cont.

Figure 4C

Figure 4C cont.

Figure 5A

Figure 5A cont.

Figure 5A cont.

Figure 5B

Figure 5B cont.

Figure 5B cont.

Figure 5B cont.

Figure 6

Figure 6 cont.

Figure 6 cont.

Figure 7A

Figure 7A cont.

Figure 7B

Figure 7B cont.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 983022 A
- US 5679640 A
- US 5800808 A
- US 3849550 A
- US 60093859 B
- US 60101825 B
- US 60102960 B
- US 60106350 B
- US 60108184 B
- US 60123675 B

### Non-patent literature cited in the description

- **AHARONI et al.** *immunology Letters,* 1997, vol. 58, 79
- **ALLISON.** *IMMUNOSUPPRESSION AND ANTI-IN-FLAMMATORY DRUGS, ANNALS OF THE NEW YORK ACADEMY OF SCIENCE,* 1993, vol. 696, xi
- **BEN-NUN A et al.** *J NEUROL,* 1996, vol. 243 (1), S14-S22
- **DORLING et al.** *CUR. OPINIONS IMMUNOL.,* 1994, 765
- **FERRARA et al.** *NEW ENGLAND J. OF MEDICINE,* 1991, 667
- **FRIDKIS-HARELI et al.** *J. NEUROCHEM.,* 1994, vol. 63 (1), 61
- **FRIDKIS-HARELI et al.** *CELL. IMMUNOL.,* 1995, 229
- **FRIDKIS-HARELI et al.** *J. IMMUNOL.,* 1998, 4386
- **JOHNSON.** *1 NEUROLOGY,* 1995, 65-70
- **KAY et al.** *TRANSPLANTATION PROCEEDINGS,* 1990, vol. 22, 96
- **KELEMEN et al.** *INT ARCH ALLERGY IMMUNOL.,* 1993, vol. 102, 309
- The Major Histocompatibility Complex (MHC). **MEN-GLE-GAW.** ENCYCLOPEDIA OF MOLECULAR BI-OLOGY. Blackwell Science Ltd, 1994, 602-06
- **ROTHBARD, J. B. et al.** *ANNU. REV. IMMUNOL.,* 1991, vol. 9, 527
- **SCHLEGEL et al.** *BLOOD,* 1994, vol. 84, 2802
- **SELA M et al.** *BULL INST PASTEUR,* 1990, vol. 88, 303-14
- **STAZL.** *TRANSPLANTATION PROCEEDINGS,* 1990, vol. 22, 5
- **SYKES.** *THE FASEB JOURNAL,* 1996, vol. 10, 721
- **TEITELBAUM et al.** *1 EUR. J. IMMUNOL.,* 1971, 242-48
- **TEITELBAUM et al.** *EUR. J. IMMUNOL.,* 1973, vol. 3, 273-79
- **TEITELBAUM et al.** *J. NEUROIMMUNOL.,* 1996, vol. 64, 209-17
- **THOMSON.** *IMMUNOLOGY TODAY,* 1988, vol. 10, 6
- **VAN DEN BOGAERDE et al.** *TRANSPLANTATION,* 1991, vol. 52, 15
- **WEBB et al.** *IMMUNOCHEM.,* 1976, vol. 13, 333
- **FRIDKIS-HARELI, M. et al.** *Proc. Natl. Acad. Sci. USA,* 1994, vol. 91, 4872-4876
- *Journal of Immunology,* 1998, vol. 160, 4386-4397
- **TEITELBAUM et al.** using the N-carboxyanhydride (NCA) blocked amino acids tyrosine, alanine, glutamic acid and trifluoroacetyllysine dissolved in dioxane. *1 EUR. J. IMMUN.,* 1971, 242
- **E.L. BASUMIAN et al.** *EuR. J. IMMUNOL.,* 1981, vol. 11, 317
- **R.P. SIRAGANIAN.** *TRENDS IN PHARMACOLOG-ICAL SCIENCES,* October 1983, 432
- **LANDO et al.** *J. IMMUNOL.,* 1979, 2156
- **AHARONI et al.** *EUR. J. IMMUNOL.,* 1993, vol. 17, 23
- **AHARONI et al.** *PROC. NATL. ACAD. SCI. USA,* 1997, 10821
- **LAHAT et al.** *J. Neurol.,* 1997, vol. 244, 129
- **LANDO et al.** *J. IMMUNOL.,* 1979, vol. 123, 2156
- **HIGGINS et al.** *J. IMMUNOL.,* 1988, vol. 140, 440
- **FRIDKIS-HARELI et al.** *PROC. NATL. ACAD. SCI. USA,* 1994, vol. 91, 4872
- **TEITELBAUM et al.** *PROC. NATL. ACAD. Sci. USA,* 1992, vol. 89, 137
- **GORGA et al.** *CELL. IMMUNOL.,* 1986, vol. 103, 160
- **GORGA et al.** *J. BIOL. CHEM.,* 1987, vol. 262, 16087
- **BARANY et al.** *THE PEPTIDES,* 1979, 1

- **FRIDKIS-HARELI et al.** *J. IMMUNOL.,* 1998, vol. 160, 4386
- **TEITELBAUM et al.** *PROC. NATL.ACAD. SCI. USA,* 1992, vol. 89, 137
- **AHARONI et al.** *Eur. J. Immunol.,* 1993, vol. 23, 17
- **AHARONI et al.** *PROC. NATL. ACAD. SCI. USA,* 1997, vol. 94, 10821
- **AHARONI et al.** *EUR. J. IMMUNOL.,* 1993, vol. 23, 17
- **TEITELBAUM et al.** *PROC. NATL. ACAD. SCI. USA,* 1992, vol. 89, 137
- **TEITELBAUM et al.** *Proc. Natl. Acad. Sci. USA,* 1991, 9528
- **TEITELBAUM et al.** *Proc. Natl. Acad. Sci. USA,* 1991, vol. 88, 9528
- **STEM, L. et al.** *CELL,* 1992, vol. 68, 465
- **DESSEN, A. et al.** *IMPUNITY,* 1997, vol. 7, 473
- **STERN, L. et al.** *CELL,* 1992, vol. 68, 465
- **DESSEN, A. et al.** *IMMUNITY,* 1997, vol. 7, 473
- **FRIDKIS-HARELI, M. et al.** *J. IMMUNOL.,* 1998, vol. 160, 4386
- **ROSIONIEC, E. F. et al.** *J..EXP. MED.,* 1997, vol. 185, 1113-1122
- **ANDERSSON, E. C. et al.** *PROC. NATL. ACAD. Scl. USA,* 1998
- **ROSLONIEC, E. F. et al.** *J. Exp. Med.,* 1997, vol. 185, 1113-1122
- **CHICZ, R. et al.** *J. EXP. MED.,* 1993, vol. 178, 27
- **CHICZ et al.** *J. EXP. MED.,* 1993, vol. 178, 27
- **LANE, W. et al.** *J. PROT. CHEM.,* 1991, vol. 10, 151
- **SPUNGIN et al.** *J. BIOCHEM,* 1989, vol. 283, 471
- **MOURITSEN et al.** *J. IMMUNOL.,* 1992, vol. 148, 1987
- **LARSEN et al.** *J. EXP, MED.,* 1996, vol. 184, 183
- **FRIDKIS-HARELI et al.** *CELL. IMMUNOL.,* 1995, vol. 163, 229
- **STERN et al.** *NATURE,* vol. 368, 215
- **BARANY, G. et al.** The Peptides. Academic Press, 1979
- **FRIDKIS-HARELI et al.** *PROC. NAD. ACAD. Scl., USA,* 1994, vol. 91, 4872-4876
- **JARDETZKY et al.** *EMBO J.,* 1990, vol. 9, 1797-1803
- **ZISMAN et al.** *Hum Immuol,* November 1995, vol. 44 (3), 121-30
- **BROCKE et al.** *Immunology,* 1990, vol. 69 (4), 495-500

SEQUENCE LISTING

<110> Aharoni, Rina
Teitelbaum, Dvora
Arnon, Ruth
Sela, Michael
Fridkis-Harelli, Masha
Strominger, Jack
<120> Treatment of Autoimmune Conditions with Copolymer 1
and Related Copolymers and Peptides
<130> 1662/493762
<140> filed herewith
<141> 1999-07-23
<150> US 60/093,859
<151> 1998-07-23
<150> US 60/101,825
<151> 1998-09-25
<150> US 60/102,960
<151> 1998-10-02
<150> US 60/106,350
<151> 1998-10-30
<150> US 60/108,184
<151> 1998-11-12
<150> US 60/123,675
<151> 1999-03-09
<160> 57
<170> WordPerfect 8.0 for Windows
<210> 1
<211> 19
<212> PRT
<213> Artificial Sequence
<220>
<223> synthetic peptide (MBP residues 84-102)
<400> 19

```
Asp Glu Asn Pro Val Val His Phe Phe Lys Asn Ile Val Thr Pro Arg
 1               5                  10                  15

                        Thr Pro Pro
                                19
```

<210> 2
<211> 13
<212> PRT
<213> Artificial Sequence
<220>
<223> synthetic peptide (HA residues 306-318)
<400> 2

```
Pro Lys Tyr Val Lys Gln Asn Thr Leu Lys Leu Ala Thr
 1               5                  10            13
```

<210> 3
<211> 13
<212> PRT
<213> Artificial Sequence
<220>

<223> synthetic peptide (CII amino acids 261-273)
<400> 3

```
Ala Gly Phe Lys Gly Glu Gln Gly Pro Lys Gly Glu Pro
 1               5                   10          13
```

<210> 4
<211> 5
<212> PRT
<213> Artificial Sequence
<220>
<223> Synthetic peptide
<400> 4

```
Ala Glu Lys Tyr Ala
 1               5
```

<210> 5
<211> 5
<212> PRT
<213> Artificial Sequence
<220>
<223> synthetic peptide
<400> 5

```
Ala Glu Lys Val Ala
 1               5
```

<210> 6
<211> 5
<212> PRT
<213> Artificial Sequence
<220>
<223> synthetic peptide
<400> 6

```
Ala Glu Lys Phe Ala
 1               5
```

<210> 7
<211> 4
<212> PRT
<213> Artificial Sequence
<220>
<223> synthetic peptide
<400> 7

```
Lys Glu Tyr Ala
 1           4
```

<210> 8
<211> 4
<212> PRT

<213> Artificial Sequence
<220>
<223> synthetic peptide
<400> 8

```
Lys Tyr Ala Glu
 1           4
```

<210> 9
<211> 4
<212> PRT
<213> Artificial Sequence
<220>
<223> synthetic peptide
<400> 9

```
Lys Glu Val Ala
 1           4
```

<210> 10
<211> 4
<212> PRT
<213> Artificial Sequence
<220>
<223> synthetic peptide
<400> 10

```
Lys Val Ala Glu
 1           4
```

<210> 11
<211> 4
<212> PRT
<213> Artificial Sequence
<220>
<223> synthetic peptide
<400> 11

```
Lys Glu Phe Ala
 1           4
```

<210> 12
<211> 4
<212> PRT
<213> Artificial Sequence
<220>
<223> synthetic peptide
<400> 12

```
Lys Phe Ala Glu
 1           4
```

<210> 13

<211> 4
<212> PRT
<213> Artificial Sequence
<220>
<223> synthetic peptide
<400> 13

Lys Tyr Ala Ala
1           4

<210> 14
<211> 4
<212> PRT
<213> Artificial Sequence
<220>
<223> synthetic peptide
<400> 14

Lys Lys Tyr Ala
1           4

<210> 15
<211> 4
<212> PRT
<213> Artificial Sequence
<220>
<223> synthetic peptide
<400> 15

Lys Val Ala Ala
1           4

<210> 16
<211> 4
<212> PRT
<213> Artificial Sequence
<220>
<223> synthetic peptide
<400> 16

Lys Lys Val Ala
1           4

<210> 17
<211> 4
<212> PRT
<213> Artificial Sequence
<220>
<223> synthetic peptide
<400> 17

Lys Phe Ala Ala
1           4

<210> 18
<211> 4
<212> PRT
<213> Artificial Sequence
<220>
<223> synthetic peptide
<400> 18

Lys Lys Phe Ala
1               4

<210> 19
<211> 5
<212> PRT
<213> Artificial Sequence
<220>
<223> synthetic peptide
<400> 19

Ala Lys Tyr Ala Glu
1                   5

<210> 20
<211> 5
<212> PRT
<213> Artificial Sequence
<220>
<223> synthetic peptide
<400> 20

Glu Ala Lys Tyr Ala
1                   5

<210> 21
<211> 5
<212> PRT
<213> Artificial Sequence
<220>
<223> synthetic peptide
<400> 21

Ala Lys Val Ala Glu
1                   5

<210> 22
<211> 5
<212> PRT
<213> Artificial Sequence
<220>
<223> synthetic peptide
<400> 22

Glu Ala Lys Val Ala
1               5

<210> 23
<211> 5
<212> PRT
<213> Artificial Sequence
<220>
<223> synthetic peptide
<400> 23

Ala Lys Phe Ala Glu
1               5

<210> 24
<211> 5
<212> PRT
<213> Artificial Sequence
<220>
<223> synthetic peptide
<400> 24

Glu Ala Lys Phe Ala
1               5

<210> 25
<211> 15
<212> PRT
<213> Artificial Sequence
<220>
<223> synthetic peptide
<400> 25

Ala Lys Glu Tyr Ala Ala Ala Ala Ala Ala Lys Ala Ala Ala Ala
1               5                   10                  15

<210> 26
<211> 15
<212> PRT
<213> Artificial Sequence
<220>
<223> synthetic peptide
<400> 26

Ala Ala Glu Tyr Ala Ala Ala Ala Ala Ala Lys Ala Ala Ala Ala
1               5                   10                  15

<210> 27
<211> 15
<212> PRT
<213> Artificial Sequence
<220>
<223> synthetic peptide

<400> 27

```
Ala Ala Lys Tyr Ala Glu Ala Ala Ala Ala Lys Ala Ala Ala Ala
 1               5                  10                  15
```

<210> 28
<211> 15
<212> PRT
<213> Artificial Sequence
<220>
<223> synthetic peptide
<400> 28

```
Glu Ala Lys Tyr Ala Ala Ala Ala Ala Ala Lys Ala Ala Ala Ala
      1               5                  10                  15
```

<210> 29
<211> 15
<212> PRT
<213> Artificial Sequence
<220>
<223> synthetic peptide
<400> 29

```
Ala Glu Lys Tyr Ala Ala Ala Ala Ala Ala Lys Ala Ala Ala Ala
 1               5                  10                  15
```

<210> 30
<211> 15
<212> PRT
<213> Artificial Sequence
<220>
<223> synthetic peptide
<400> 30

```
Lys Glu Ala Tyr Ala Ala Ala Ala Ala Ala Lys Ala Ala Ala Ala
 1               5                  10                  15
```

<210> 31
<211> 15
<212> PRT
<213> Artificial Sequence
<220>
<223> synthetic peptide
<400> 31

```
Ala Glu Glu Tyr Ala Ala Ala Ala Ala Ala Lys Ala Ala Ala Ala
 1               5                  10                  15
```

<210> 32
<211> 15
<212> PRT
<213> Artificial Sequence

<220>
<223> synthetic peptide
<400> 32

```
Glu Lys Ala Tyr Ala Ala Ala Ala Ala Ala Lys Ala Ala Ala Ala
 1               5                  10                  15
```

<210> 33
<211> 15
<212> PRT
<213> Artificial Sequence
<220>
<223> synthetic peptide
<400> 33

```
Ala Ala Lys Tyr Glu Ala Ala Ala Ala Ala Lys Ala Ala Ala Ala
 1               5                  10                  15
```

<210> 34
<211> 15
<212> PRT
<213> Artificial Sequence
<220>
<223> synthetic peptide
<400> 34

```
Glu Ala Ala Tyr Ala Ala Ala Ala Ala Ala Lys Ala Ala Ala Ala
 1               5                  10                  15
```

<210> 35
<211> 15
<212> PRT
<213> Artificial Sequence
<220>
<223> synthetic peptide
<400> 35

```
Glu Lys Lys Tyr Ala Ala Ala Ala Ala Ala Lys Ala Ala Ala Ala
 1               5                  10                  15
```

<210> 36
<211> 15
<212> PRT
<213> Artificial Sequence
<220>
<223> synthetic peptide
<400> 36

```
Ala Lys Lys Tyr Glu Ala Ala Ala Ala Ala Ala Ala Ala Ala Ala
 1               5                  10                  15
```

<210> 37
<211> 15

<212> PRT
<213> Artificial Sequence
<220>
<223> synthetic peptide
<400> 37

```
Ala Ala Glu Tyr Lys Ala Ala Ala Ala Ala Ala Ala Ala Ala Ala
1               5                   10                  15
```

<210> 38
<211> 15
<212> PRT
<213> Artificial Sequence
<220>
<223> synthetic peptide
<400> 38

```
Ala Ala Lys Tyr Glu Ala Ala Ala Ala Ala Ala Ala Ala Ala Ala
1               5                   10                  15
```

<210> 39
<211> 15
<212> PRT
<213> Artificial Sequence
<220>
<223> synthetic peptide
<400> 39

```
Ala Ala Lys Tyr Ala Glu Ala Ala Ala Ala Ala Ala Ala Ala Ala
1               5                   10                  15
```

<210> 40
<211> 15
<212> PRT
<213> Artificial Sequence
<220>
<223> synthetic peptide
<400> 40

```
Ala Glu Tyr Ala Lys Ala Ala Ala Ala Ala Ala Ala Ala Ala Ala
1               5                   10                  15
```

<210> 41
<211> 15
<212> PRT
<213> Artificial Sequence
<220>
<223> synthetic peptide
<400> 41

```
Ala Glu Lys Ala Tyr Ala Ala Ala Ala Ala Ala Ala Ala Ala Ala
1               5                   10                  15
```

<210> 42
<211> 15
<212> PRT
<213> Artificial Sequence
<220>
<223> synthetic peptide
<400> 42

```
        Ala Tyr Lys Ala Glu Ala Ala Ala Ala Ala Ala Ala Ala Ala Ala
          1               5                  10                  15
```

<210> 43
<211> 15
<212> PRT
<213> Artificial Sequence
<220>
<223> synthetic peptide
<400> 43

```
        Ala Lys Tyr Ala Glu Ala Ala Ala Ala Ala Ala Ala Ala Ala Ala
          1               5                  10                  15
```

<210> 44
<211> 15
<212> PRT
<213> Artificial Sequence
<220>
<223> synthetic peptide
<400> 44

```
        Ala Pro Lys Tyr Val Lys Gln Asn Thr Leu Lys Leu Ala Thr Ala
          1               5                  10                  15
```

<210> 45
<211> 15
<212> PRT
<213> Artificial Sequence
<220>
<223> synthetic peptide
<400> 45

```
        Ala Ala Ala Tyr Ala Ala Ala Ala Ala Ala Lys Ala Ala Ala Ala
          1               5                  10                  15
```

<210> 46
<211> 15
<212> PRT
<213> Artificial Sequence
<220>
<223> synthetic peptide
<400> 46

```
Ala Lys Lys Tyr Ala Ala Ala Ala Ala Ala Lys Ala Ala Ala Ala
 1               5                  10                 15
```

<210> 47
<211> 15
<212> PRT
<213> Artificial Sequence
<220>
<223> synthetic peptide
<400> 47

```
Ala Glu Ala Tyr Ala Ala Ala Ala Ala Ala Lys Ala Ala Ala Ala
 1               5                  10                 15
```

<210> 48
<211> 15
<212> PRT
<213> Artificial Sequence
<220>
<223> synthetic peptide
<400> 48

```
Ala Glu Lys Tyr Ala Ala Ala Ala Ala Ala Ala Ala Ala Ala Ala
 1               5                  10                 15
```

<210> 49
<211> 15
<212> PRT
<213> Artificial Sequence
<220>
<223> synthetic peptide
<400> 49

```
Ala Lys Glu Tyr Ala Ala Ala Ala Ala Ala Ala Ala Ala Ala Ala
 1               5                  10                 15
```

<210> 50
<211> 15
<212> PRT
<213> Artificial Sequence
<220>
<223> synthetic peptide
<400> 50

```
Ala Lys Lys Tyr Ala Glu Ala Ala Ala Ala Ala Ala Ala Ala Ala
 1               5                  10                 15
```

<210> 51
<211> 15
<212> PRT
<213> Artificial Sequence
<220>
<223> synthetic peptide

<400> 51

```
        Ala Glu Ala Tyr Lys Ala Ala Ala Ala Ala Ala Ala Ala Ala Ala
        1               5                   10                  15
```

<210> 52
<211> 15
<212> PRT
<213> Artificial Sequence
<220>
<223> synthetic peptide
<400> 52

```
        Lys Glu Ala Tyr Ala Ala Ala Ala Ala Ala Ala Ala Ala Ala Ala
        1               5                   10                  15
```

<210> 53
<211> 15
<212> PRT
<213> Artificial Sequence
<220>
<223> synthetic peptide
<400> 53

```
        Ala Glu Glu Tyr Lys Ala Ala Ala Ala Ala Ala Ala Ala Ala Ala
        1               5                   10                  15
```

<210> 54
<211> 15
<212> PRT
<213> Artificial Sequence
<220>
<223> synthetic peptide
<400> 54

```
        Glu Lys Ala Tyr Ala Ala Ala Ala Ala Ala Ala Ala Ala Ala Ala
        1               5                   10                  15
```

<210> 55
<211> 15
<212> PRT
<213> Artificial Sequence
<220>
<223> synthetic peptide
<400> 55

```
        Glu Lys Lys Tyr Ala Ala Ala Ala Ala Ala Ala Ala Ala Ala Ala
        1               5                   10                  15
```

<210> 56
<211> 15
<212> PRT
<213> Artificial Sequence

```
<220>
<223> synthetic peptide
<400> 56
```

```
Glu Ala Lys Tyr Ala Ala Ala Ala Ala Ala Ala Ala Ala Ala Ala
 1               5                  10                  15
```

```
<210> 57
<211> 15
<212> PRT
<213> Artificial Sequence
<220>
<223> synthetic peptide
<400> 57
```

```
Glu Lys Tyr Ala Ala Ala Ala Ala Ala Ala Ala Ala Ala Ala Ala
 1               5                  10                  15
```